# EUROPEAN PATENT SPECIFICATION

(11) **EP 0 931 078 B1**
(45) Date of publication and mention of the grant of the patent: **15.11.2006**
(21) Application number: 97941477.8
(22) Date of filing: 11.09.1997
(51) Int. Cl.: C07D 401/14, C07F 9/6558, C07H 19/04, C07D 401/04, A61K 31/435

(54) **NOVEL TRICYCLIC PIPERIDINYL COMPOUNDS USEFUL AS INHIBITORS OF FARNESYL-PROTEIN TRANSFERASE**
TRICYCLISCHE PIPERDINYL DERIVATE VERWENDBAR ALS FARNESYL-PROTEIN TRANSFERASE INHIBITOREN
NOUVEAUX COMPOSES TRICYCLIQUES DE PIPERIDINYLE INHIBITEURS DE LA FARNESYL-PROTEINE TRANSFERASE

(30) Priority: 13.09.1996 US 713703
(43) Date of publication of application: 28.07.1999
(73) Proprietor: SCHERING CORPORATION, Kenilworth, New Jersey 07033 (US)
(72) Inventor: COOLER, Alan, B., West Caldwell, NJ 07006 (US); MALLANS, Alan, K., Hackettstown, NJ 07840 (US); GIRIJAVALLABHAN, Viyyoor, M., Parsippany, NJ 07054 (US); DOLL, Ronald, J., Maplewood, NJ 07040 (US); TAVERAS, Arthur, G., Rockaway, NJ 07866 (US); NJOROGE,, F., George, Union, NJ 07083 (US); BALDWIN, John, J., Gwynedd Valley, PA 19437 (US); READER, John, C., Princeton, NJ 08540 (US)
(74) Representative: Adams, Harvey Vaughan John
(86) International application number: PCT/US1997/015903
(87) International publication number: WO 1998/011098

(56) References cited:
- EP-A- 0 396 083
- WO-A-92/00293
- WO-A-95/10515
- WO-A-95/10516
- GB-A- 1 593 417

## Description

### BACKGROUND

Patent application WO 95/00497 published 5 January 1995 under the Patent Cooperation Treaty (PCT) describes compounds which inhibit the enzyme, famesyl-protein transferase (FTase) and the famesylation of the oncogene protein Ras. Oncogenes frequently encode protein components of signal transduction pathways which lead to stimulation of cell growth and mitogenesis. Oncogene expression in cultured cells leads to cellular transformation, characterized by the ability of cells to grow in soft agar and the growth of cells as dense foci lacking the contact inhibition exhibited by non-transformed cells. Mutation and/or overexpression of certain oncogenes is frequently associated with human cancer.

WO 95/10515 and WO 95/10516 disclose structurally related compounds for treatment of proliferative diseases.

To acquire transforming potential, the precursor of the Ras oncoprotein must undergo farnesylation of the cysteine residue located in a carboxyl-terminal tetrapeptide. Inhibitors of the enzyme that catalyzes this modification, farnesyl protein transferase, have therefore been suggested as anticancer agents for tumors in which Ras contributes to transformation. Mutated, oncogenic forms of Ras are frequently found in many human cancers, most notably in more than 50% of colon and pancreatic carcinomas (Kohl et al., Science, Vol. 260, 1834 to 1837, 1993).

In view of the current interest in inhibitors of famesyl protein transferase, a welcome contribution to the art would be additional compounds useful for the inhibition of famesyl protein transferase. Such a contribution is provided by this invention.

### SUMMARY OF THE INVENTION

Inhibition of famesyl protein transferase by tricyclic compounds of this invention has not been reported previously. Thus, this invention provides a method for inhibiting famesyl protein transferase using tricyclic compounds of this invention which: (i) potently inhibit famesyl protein transferase, but not geranylgeranyl protein transferase I, in vitro; (ii) block the phenotypic change induced by a form of transforming Ras which is a farnesyl acceptor but not by a form of transforming Ras engineered to be a geranylgeranyl acceptor, (iii) block intracellular processing of Ras which is a farnesyl acceptor but not of Ras engineered to be a geranylgeranyl acceptor; and (iv) block abnormal cell growth in culture induced by transforming Ras. Several compounds of this invention have been demonstrated to have anti-tumor activity in animal models.

This invention provides a method for inhibiting the abnormal growth of cells, including transformed cells, by administering an effective amount of a compound of this invention. Abnormal growth of cells refers to cell growth independent of normal regulatory mechanisms (e.g., loss of contact inhibition). This includes the abnormal growth of: (1) tumor cells (tumors) expressing an activated Ras oncogene; (2) tumor cells in which the Ras protein is activated as a result of oncogenic mutation in another gene; and (3) benign and malignant cells of other proliferative diseases in which aberrant Ras activation occurs.

Compounds useful in the claimed methods are represented by Formula 1.0: 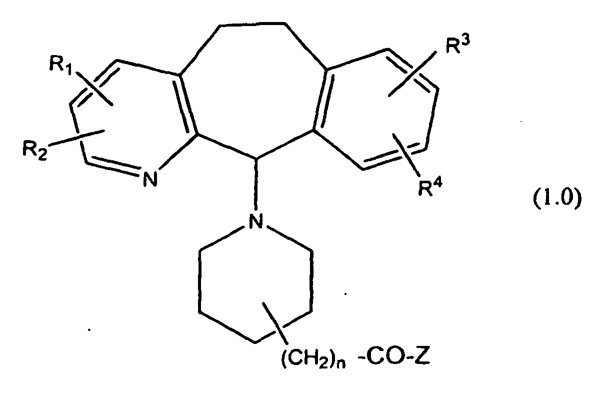 or a pharmaceutically acceptable salt thereof, wherein
n = 0, 1 or 2,
R¹ and R⁴ are H and R² and R³ are halo selected from chloro or bromo, or,
R¹ is H and R², R³ and R⁴ are halo selected from chloro or bromo,
Z is -NR⁴⁰R⁴² wherein R⁴⁰ and R⁴² independently represent H, aryl, alkyl, aralkyl, heteroaryl, heteroarylalkyl, heterocycloalkyl, heterocycloalkylalkyl, heteroalkyl, cycloalkyl or cycloalkylalkyl; or 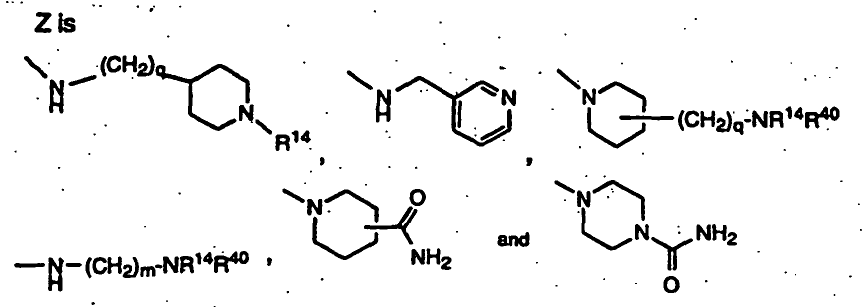 wherein R⁴⁰ is defined hereinbefore,
m is 2, 3 or 4;
q is 0 (zero), 1 or 2;
and R¹⁴ represents H, C₁₋₆ alkyl, aralkyl, heteroaryl, acyl, carboxamido, carboxamidoalkyl, cyano, alkoxycarbonyl, aralkyloxycarbonyl imido, imidamido, sulfamoyl, sulfonyl, dialkylphosphinyl, N-glycosyl or -C(NHCH₃)=CHNO₂.

In the compounds of formula (1.0), preferably R² and R³ are halo in the 3- and the 8-position on the ring structure; or R², R³ and R⁴ are in the 3-, 8- and 10-position on the ring structure. Also preferred is that R² is Br and R³ is Cl in the 3- and the 8-position on the ring structure; or R² is Br, R³ is Cl and R⁴ is Br in the 3-, 8- and 10- position on the ring structure. Also preferred is that each of R⁵, R⁶, R⁷ and R⁸ is H. Also preferred is that the moiety -(CH₂)-ₙ-CO-Z is bonded at the 2-, 3- or 4-position on the piperdinyl ring, more preferably at the 2- or 3-position on the piperdinyl ring.

Preferably R⁴⁰ is H and R⁴² is the heteroaryl moiety 3-pyridylmethyl.

In another embodiment, the present invention is directed toward a pharmaceutical composition for inhibiting the abnormal growth of cells comprising an effective amount of compound (1.0) in combination with a pharmaceutically acceptable carrier.

In another embodiment, the present invention is directed toward a method for inhibiting the abnormal growth of cells, including transformed cells, comprising administering an effective amount of compound (1.0) to a mammal (e.g., a human) in need of such treatment. Abnormal growth of cells refers to cell growth independent of normal regulatory mechanisms (e.g., loss of contact inhibition). This includes the abnormal growth of: (1) tumor cells (tumors) expressing an activated Ras oncogene; (2) tumor cells in which the Ras protein is activated as a result of oncogenic mutation in another gene; (3) benign and malignant cells of other proliferative diseases in which aberrant Ras activation occurs, and (4) benign or malignant cells that are activated by mechanisms other than the Ras protein. Without wishing to be bound by theory, it is believed that these compounds may function either through the inhibition of G-protein function, such as ras p21, by blocking G-protein isoprenylation, thus making them useful in the treatment of proliferative diseases such as tumor growth and cancer, or through inhibition of ras farnesyl protein transferase, thus making them useful for their antiproliferative activity against ras transformed cells.

The cells to be inhibited can be tumor cells expressing an activated ras oncogene. For example, the types of cells that maybe inhibited include pancreatic tumor cells, lung cancer cells, myeloid leukemia tumor cells, thyroid follicular tumor cells, myelodysplastic tumor cells, epidermal carcinoma tumor cells, bladder carcinoma tumor cells or colon tumors cells. Also, the inhibition of the abnormal growth of cells by the treatment with compound (1.0) may be by inhibiting ras famesyl protein transferase. The inhibition may be of tumor cells wherein the Ras protein is activated as a result of oncogenic mutation in genes other than the Ras gene. Alternatively, compounds (1.0) may inhibit tumor cells activated by a protein other than the Ras protein.

This invention also provides a method for inhibiting tumor growth by administering an effective amount of compound (1.0) to a mammal (e.g., a human) in need of such treatment. In particular, this invention provides a method for inhibiting the growth of tumors expressing an activated Ras oncogene by the administration of an effective amount of the above described compounds. Examples of tumors which may be inhibited include, but are not limited to, lung cancer (e.g., lung adenocarcinoma), pancreatic cancers (e.g., pancreatic carcinoma such as, for example, exocrine pancreatic carcinoma), colon.cancers (e.g., colorectal carcinomas, such as, for example, colon adenocarcinoma and colon adenoma), myeloid leukemias (for example, acute myelogenous leukemia (AML)), thyroid follicular cancer, myelodysplastic syndrome (MDS), bladder carcinoma and epidermal carcinoma.

It is believed that this invention also provides a method for inhibiting proliferative diseases, both benign and malignant, wherein Ras proteins are aberrantly activated as a result of oncogenic mutation in other genes--i.e., the Ras gene itself is not activated by mutation to an oncogenic form--with said inhibition being accomplished by the administration of an effective amount of the carbonyl piperazinyl and piperidinyl compounds (1.0) described herein, to a mammal (e.g., a human) in need of such treatment. For example, the benign proliferative disorder neurofibromatosis, or tumors in which Ras is activated due to mutation or overexpression of tyrosine kinase oncogenes (e.g., neu, src, abl, lck, and fyn), may be inhibited by the carbonyl piperazinyl and piperidinyl compounds (1.0) described herein.

In another embodiment, the present invention is directed toward a method for inhibiting ras famesyl protein transferase and the famesylation of the oncogene protein Ras by administering an effective amount of compound (1.0) to mammals, especially humans. The administration of the compounds of this invention to patients, to inhibit farnesyl protein transferase, is useful in the treatment of the cancers described above.

### DETAILED DESCRIPTION OF THE INVENTION

The following solvents and reagents are referred to herein by the abbreviations indicated:
tetrahydrofuran (THF);
ethanol (EtOH);
methanol (MeOH);
ethyl acetate (EtOAc);
N,N-dimethylformamide (DMF);
trifluoroacetic acid (TFA);
1-hydroxybenzotriazole (HOBT);
1-(3-dimethylaminopropyl)-3-ethyl carbodiimide hydrochloride (DEC);
dimethylsulfoxide (DMSO);
4-methylmopholine (NMM);
dimethylaminopyridine (DMAP); and
dimethoxyethane (DME).
t-butoxycarbonyl (BOC)
acetyl(OAc)

As used herein, the following terms are used as defined below unless otherwise indicated:
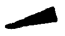 or 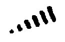 - indicates a pure isomer;
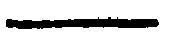 - when attached to a carbon atom labeled with an asterisk (*), indicates a separated isomer whose stereochemistry is not established;
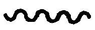 - indicates a racemic mixture;
M⁺ -represents the molecular ion of the molecule in the mass spectrum;
MH⁺ -represents the molecular ion plus hydrogen of the molecule in the mass spectrum;
Bu-represents butyl;
Et-represents ethyl;
Me-represents methyl;
Ph-represents phenyl;
benzotriazol-1-yloxy represents 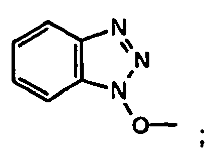
1-methyl-tetrazol-5-ylthio represents 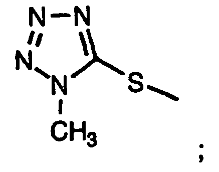
acyl-a moiety of the formula -COR¹⁵ wherein R¹⁵ represents H, C₁₋₆alkyl, aryl, aralkyl, heteroalkyl, heteroaryl, heteroarylalkyl, heterocycloalkyl, heterocycloalkylalkyl or -(CH₂)ₖNR⁸⁰R⁸¹ wherein k is 1 or 2, and R⁸⁰ and R⁸¹ may independently represent H, alkyl, cycloalkyl, cycloalkylalkyl, heteroaryl, heteroarylalkyl, aryl or aralkyl;
alkyl-(including the alkyl portions of alkoxy, alkylamino and dialkylamino)-represents straight and branched carbon chains and contains from one to twenty carbon atoms, preferably one to six carbon atoms (i.e. C₁₋₆ alkyl); for example methyl, ethyl, propyl, iso-propyl, n-butyl, t-butyl, n-pentyl, isopentyl, hexyl and the like; wherein said alkyl and said C₁₋₆ alkyl group may be optionally and independently substituted with one, two, three or more of the following: halo, alkyl, aryl, alkoxy, amino (-NH₂), alkylamino, cyano (-CN), -CF₃, dialkylamino, hydroxy, oxy (=O), phenoxy, -OCF₃, heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -NCOR¹⁰ or -COOR¹⁰.
alkoxy-an alkyl moiety of one to 20 carbon atoms covalently bonded to an adjacent structural element through an oxygen atom, for example, methoxy, ethoxy, propoxy, butoxy, pentoxy, hexoxy and the like; wherein said alkoxy group may be optionally and independently substituted with one, two, three or more of the following: halo, alkyl, aryl, alkoxy, amino, alkylamino, cyano, -CF₃, dialkylamino, hydroxy, oxy, phenoxy, -OCF₃, heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ or -COOR¹⁰;
alkoxycarbonyl - represents a alkoxy moiety, as defined above, covalantly bonded to a carbonyl moiety (-CO-) through an oxygen atom, for example, -COOCH₃, -COOCH₂CH₃ and -COOC(CH₃)₃;
alkenyl-represents straight and branched carbon chains having at least one carbon to carbon double bond and containing from 2 to 12 carbon atoms, preferably from 2 to 6 carbon atoms and most preferably from 3 to 6 carbon atoms; wherein said alkenyl group may be optionally and independently substituted with one, two, three or more of the following: halo, alkyl, aryl, alkoxy, amino, alkylamino, cyano, -CF₃, dialkylamino, hydroxy, oxy, phenoxy, -OCF₃, heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ or -COOR¹⁰;
alkynyl-represents straight and branched carbon chains having at least one carbon to carbon triple bond and containing from 2 to 12 carbon atoms, preferably from 2 to 6 carbon atoms; wherein said alkynyl group may be optionally and independently substituted with one, two, three or more of the following: halo, alkyl, aryl, alkoxy, amino, alkylamino, cyano, -CF₃, dialkylamino, hydroxy, oxy, phenoxy, -OCF₃, heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ or -COOR¹⁰;
amino acid- refers to organic compounds having both an amino group (-NH₂) and a carboxyl group (-COOH). Representative amino acids include glycine, serine, alanine, phenylalanine, tyrosine, S-methyl methionine and histidine;
aryl (including the aryl portion of aralkyl)-represents a carbocyclic group containing from 6 to 15 carbon atoms and having at least one aromatic ring (e.g., aryl is phenyl), wherein said aryl group optionally can be fused with aryl, cycloalkyl, heteroaryl or heterocycloalkyl rings; and wherein any of the available substitutable carbon and nitrogen atoms in said aryl group and/or said fused ring(s) may be optionally and independently substituted with one, two, three or more of the following: halo, alkyl, aryl, alkoxy, amino, alkylamino, cyano, -CF₃, dialkylamino, hydroxy, oxy, phenoxy, -OCF₃, heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ or -COOR¹⁰;
aralkyl - represents an alkyl group, as defined above, wherein one or more hydrogen atoms of the alkyl moiety have been substituted with one or more aryl groups; wherein said aralkyl group may be optionally and independently substituted with one, two, three or more of the following: halo, alkyl, aryl, alkoxy, amino, alkylamino, cyano, -CF₃, dialkylamino, hydroxy, oxy, phenoxy, -OCF₃, heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ or -COOR¹⁰; Representative aralkyl groups include benzyl and diphenylmethyl;
aralkyloxy - represents an aralkyl group, as defined above, covalently bonded to an adjacent structural element through an oxygen atom, for example, phenylmethyloxy and phenylethyloxy;
aralkyloxycarbonyl - represents an aralkyloxy group, as defined above, covalantly bonded to a carbonyl moiety (-CO-) through an oxygen atom, for example, -COOCH₂C₆H₅ and -COOCH₂CH₂C₆H₅;
carboxamido - represents a moiety of the formula -CONR⁴⁰R⁴², including -CONH₂;
carboxamidoalkyl - represents an alkyl group, as defined above, wherein a hydrogen atom of the alkyl moiety has been substituted with a carboxamido moiety, as defined above, through the carbonyl (-CO) portion of the carboxamido moiety, for example, -CH₂CONH₂ and -CH₂CH₂CONH₂;
cycloalkyl-represents saturated carbocyclic rings branched or unbranched of from 3 to 20 carbon atoms, preferably 3 to 7 carbon atoms; wherein said cycloalkyl group may be optionally and independently substituted with one, two, three or more of the following: halo, alkyl, aryl, alkoxy, amino, alkylamino, cyano, -CF₃, dialkylamino, hydroxy, oxy, phenoxy, -OCF₃, heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ or -COOR¹⁰;
cycloalkylalkyl - represents an alkyl group, as defined above, wherein one or more hydrogen atoms of the alkyl moiety have been substituted with one or more cycloalkyl groups; wherein said cycloalkylalkyl group may be optionally and independently substituted with one, two, three or more of the following: halo, alkyl, aryl, alkoxy, amino, alkylamino, cyano, -CF₃, dialkylamino, hydroxy, oxy, phenoxy, -OCF₃, heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ or -COOR¹⁰;
halo-represents fluoro, chloro, bromo and iodo;
heteroalkyl-represents straight and branched carbon chains containing from one to twenty carbon atoms, preferably one to six carbon atoms interrupted by 1 to 3 heteroatoms selected from -O-, -S- and -N-; wherein any of the available substitutable carbon and nitrogen atoms in said heteroalkyl chain may be optionally and independendently substituted with one, two, three or more of the following: halo, C₁-C₆ alkyl, aryl, cyano, hydroxy, alkoxy, oxy, phenoxy, -CF₃, -OCF₃, amino, alkylamino, dialkylamino, heterocycloalkyl, -SO₂NH₂, -NHSO₂R¹⁰, -SO₂NHR¹⁰, -SO₂R¹⁰, -SOR¹⁰, -SR¹⁰, or -NHSO₂, -NO₂, -CONR¹⁰, -NCOR¹⁰ or -COOR¹⁰;
heteroaryl-represents cyclic groups having at least one heteroatom selected from O, S and N, said heteroatom(s) interrupting a carbocyclic ring structure and having a sufficient number of delocalized pi electrons to provide aromatic character, with the aromatic heterocyclic groups containing from 2 to 14 carbon atoms,wherein said heteroaryl group optionally can be fused with one or more aryl, cycloalkyl, heteroaryl or heterocycloalkyl rings; and wherein any of the available substitutable carbon or nitrogen atoms in said heteroaryl group and/or said fused ring(s) may be optionally and independendently substituted with one, two, three or more of the following: halo, C₁-C₆ alkyl, aryl, cyano, hydroxy, alkoxy, oxy, phenoxy, -CF₃, -OCF₃, amino, alkylamino, dialkylamino, heterocycloalkyl, -SO₂NH₂, -NHSO₂R¹⁰, -SO₂NHR¹⁰, -SO₂R¹⁰, -SOR¹⁰, -SR¹⁰, or -NHSO₂, -NO₂, -CONR¹⁰, -NCOR¹⁰ or -COOR¹⁰. Representative heteroaryl groups can include, for example, furanyl, imidazoyl, pyrimidinyl, triazolyl, 2-, 3- or 4-pyridyl or 2-, 3- or 4-pyridyl N-oxide wherein pyridyl N-oxide can be represented as: 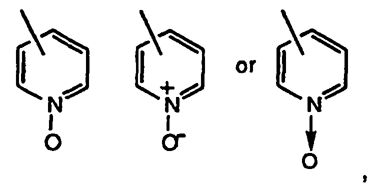
heteroarylalkyl - represents an alkyl group, as defined above, wherein one or more hydrogen atoms have been replaced by one or more heteroaryl groups; wherein said heteroarylalkyl group may be optionally and independently substituted with one, two, three or more of the following: halo, alkyl, aryl, alkoxy, amino, alkylamino, cyano, -CF₃, dialkylamino, hydroxy, oxy, phenoxy, -OCF₃, heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ or -COOR¹⁰; as exemplified by 2-, 3- or 4-pyridylmethyl or 2-, 3- or 4-pyridylmethyl N-oxide;
heterocycloalkyl-represents a saturated, branched or unbranched carbocylic ring containing from 3 to 15 carbon atoms, preferably from 4 to 6 carbon atoms, which carbocyclic ring is interrupted by 1 to 3 heteroatoms selected from -O-, -S- and -N- , wherein optionally, said ring may contain one or two unsaturated bonds which do not impart aromatic character to the ring; and wherein any of the available substitutable carbon and nitrogen atoms in the ring may be optionally and independently substituted with one, two, three or more of the following: halo, alkyl, aryl, alkoxy, amino, alkylamino, cyano, -CF₃, dialkylamino, hydroxy, oxy, phenoxy, -OCF₃, heterocycloalkyl, -SO₂NH₂, -NHSO₂R¹⁰, -SO₂NHR¹⁰, -SO₂R¹⁰, -SOR¹⁰, -SR¹⁰, -NHSO₂, -N0₂, -CONR¹⁰, -NCOR¹⁰ or -COOR¹⁰

Representative heterocycloalkyl groups can include morpholinyl, 2- or 3-tetrahydrofuranyl, 2- or 3- tetrahydrothienyl, 1-, 2-, 3- or 4-piperidinyl, 2- or 3-pyrrolidinyl, 1-, 2- or 3-piperizinyl, 2- or 4-dioxanyl, or 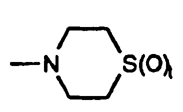 wherein t is 0, 1 or 2;
heterocycloalkylalkyl- represents an alkyl group, as defined above, wherein one or more hydrogen atoms have been replaced by one or more heterocycloalkyl groups; wherein optionally, said ring may contain one or two unsaturated bonds which do not impart aromatic character to the ring; and wherein said heterocycloalkylalkyl group may be optionally and independently substituted with one, two, three or more of the following: halo, alkyl, aryl, alkoxy, amino, alkylamino, cyano, -CF₃, dialkylamino, hydroxy, oxy, phenoxy, -OCF₃, heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ or -COOR¹⁰;
imido - represents a moiety of the formula 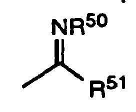 wherein and R⁵⁰ represents H, cyano, aryl, -SO₂NH₂, -SO₂NR⁴⁰R⁴² and carboxamido and R⁵¹ represents aryl and aryloxy. Representative imido groups can include, for example, 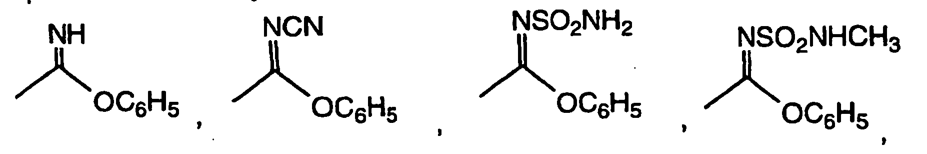 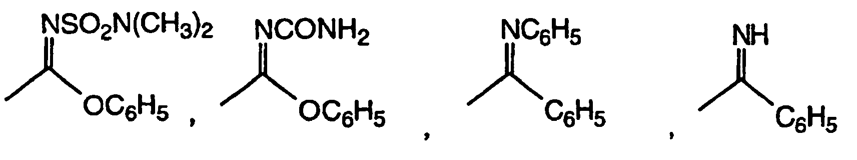 imidamido - represents a moiety of the formula 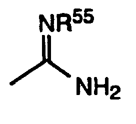 wherein and R⁵⁵ represents H, cyano, -SO₂NH₂, -SO₂NR⁴⁰R⁴², carboxamido, hydroxy and alkoxy. Representative imidamido groups can include, for example, 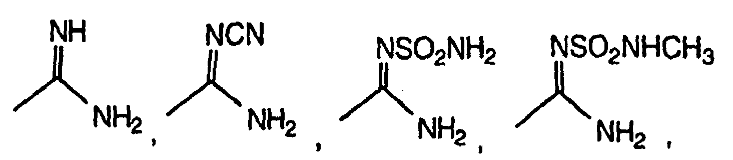 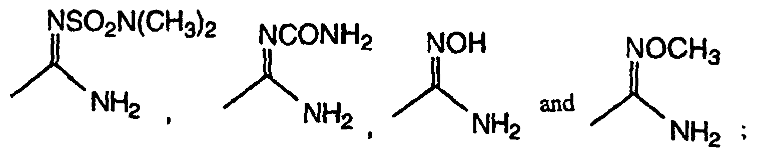 N-glycosyl- represents a pyranosyl or furanosyl monosaccaride. Representative N-glycosyl groups include (N → 1)-tetra-O-acetyl-D-giucosyl, (N → 1)-tetra-O-acetyl-D-galactosyl and (N → 1) -tri-O-acetyl-D-ribosyl, e.g. 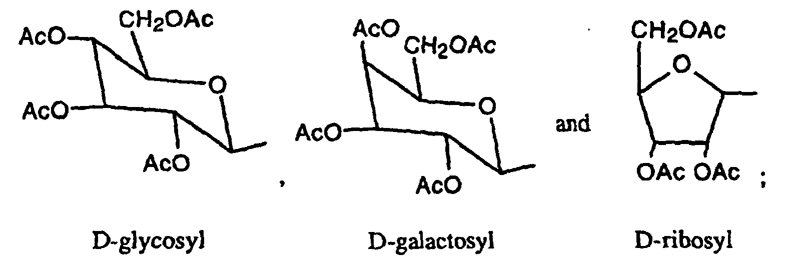 1-amino-2-nitroethenyl represents the formula: -C(NHCH₃)=CHNO₂;
dialkylphosphinyl - represents a phosphine (-PO) moiety covalently bonded to two alkyl groups. A representative dialkylphosphinyl group is -PO(CH₃)₂.
sulfamoyl - represents a moiety of the formula -SO₂R⁶⁰ wherein R⁶⁰ represents amino, alkylamino and dialkylamino. Representative sulfamoyl groups can include, for example, -SO₂NH₂, -SO₂NHCH₃, -SO₂N(CH₃)₂.
sulfonyl - represents a moiety of the formula -SO₂R⁶⁰ wherein R⁶⁰ represents alkyl, aryl and arylalkyl. Representative sulfonyl groups can include, for example, -SO₂CH₃, -SO₂C₆H₅, -SO₂C₆H₄CH₃, and -SO₂CH₂C₆H₅,

Reference to the position of the substituents R¹, R², R³, and R⁴ is based on the numbered ring structure:

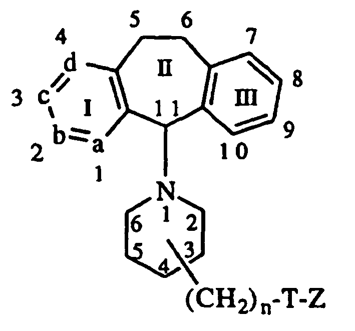

Certain compounds of the invention may exist in different stereoisomeric forms (e.g., isomers such as enantiomers and diastereoisomers). The invention contemplates all such stereoisomers both in pure form and in mixture, including racemic mixtures. For example, the carbon atom at the C-11 position can be in the S or R stereoconfiguration. Also, the carbon atom at the C-2, C-3, C-5 and C-6 positions of the piperidinyl moiety bonded at C-11 can also be in the S or R stereoconfiguration.

Certain tricyclic compounds will be acidic in nature, e.g. those compounds which possess a carboxyl or phenolic hydroxyl group. These compounds may form pharmaceutically acceptable salts. Examples of such salts may include sodium, potassium, calcium, aluminum, gold and silver salts. Also contemplated are salts formed with pharmaceutically acceptable amines such as ammonia, alkyl amines, hydroxyalkylamines, N-methylglucamine and the like.

Certain basic tricyclic compounds also form pharmaceutically acceptable salts, e.g., acid addition salts. For example, the pyrido-nitrogen atoms may form salts with strong acid, while compounds having basic substituents such as amino groups also form salts with weaker acids. Examples of suitable acids for salt formation are hydrochloric, sulfuric, phosphoric, acetic, citric, oxalic, malonic, salicylic, malic, fumaric, succinic, ascorbic, maleic, methanesulfonic and other mineral and carboxylic acids well known to those in the art. The salts are prepared by contacting the free base form with a sufficient amount of the desired acid to produce a salt in the conventional manner. The free base forms may be regenerated by treating the salt with a suitable dilute aqueous base solution such as dilute aqueous NaOH, potassium carbonate, ammonia and sodium bicarbonate. The free base forms differ from their respective salt forms somewhat in certain physical properties, such as solubility in polar solvents, but the acid and base salts are otherwise equivalent to their respective free base forms for purposes of the invention.

All such acid and base salts are intended to be pharmaceutically acceptable salts within the scope of the invention and all acid and base salts are considered equivalent to the free forms of the corresponding compounds for purposes of the invention.

### Compounds of the present invention can be prepared according to the following Scheme 1:

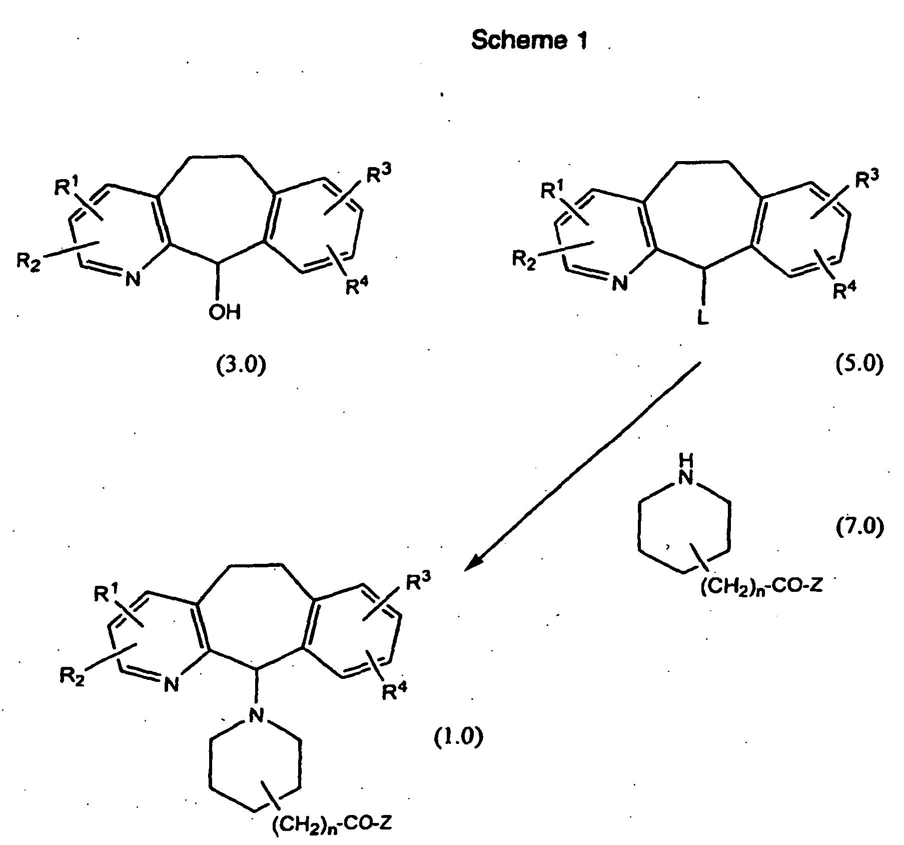

wherein L represents a leaving group such as halo, preferably chloro or a leaving group such as o-tosyl and o-mesyl; R¹, R², R³, R⁴, n, and Z are as defined hereinbefore.

Referring to the Scheme 1, compounds of formula (5.0) can be prepared by reacting the compounds of formula (3.0) with a halogenating agent or a sulfonylating agent in the presence of a suitable base, and optional aprotic solvent, in amounts and under conditions effective to give compounds (5.0). Suitable bases include organic bases such as pyridine and triethylamine; or inorganic bases of alkali and alkaline earth metals including carbonates such as sodium, lithium, potassium and cesium carbonates, hydroxides such as sodium, lithium and potassium hydroxides; hydrides such as sodium or potassium hydride; and sodium t-butoxide, preferably sodium hydride. Suitable aprotic solvents include ethers, DMF, DMSO, THF, DME and mixtures thereof, preferably DMF. Preferably the halogenating agent is a chlorinating agent, such as thionyl chloride. The sulfonylating can be methane sulfonyl chloride or toluene sulfonyl chloride. The amounts of the halogenating agent or the sulfonylating agent can range from about one to about 10 moles per mole of compound (3.0). Temperatures can range from 0° to 50°C, or reflux of the reaction mixture.

The desired tricylic piperidinyl compounds of formula (1.0) can be prepared by reacting the compounds of formula (5.0) with a suitably substituted piperidinyl compound of formula (7.0) in the presence of a suitable base and optional aprotic solvent, such as those described above, to give compounds (1.0). The amounts of the substituted piperidinyl compound of formula (7.0) to compound (5.0) can range from about one to about 10 moles per mole of compound (5.0) Temperatures can range from about room temperature to about 80°C.

The tricylic piperidinyl compounds of fomula (1.0) can be isolated from the reaction mixture using conventional procedures, such as, for example, extraction of the reaction mixture from water with organic solvents, evaporation of the organic solvents, followed by chromatography on silica gel or other suitable chromatographic media.

Selected compounds of formula (1.0) can be prepared in accordance with Scheme 2. 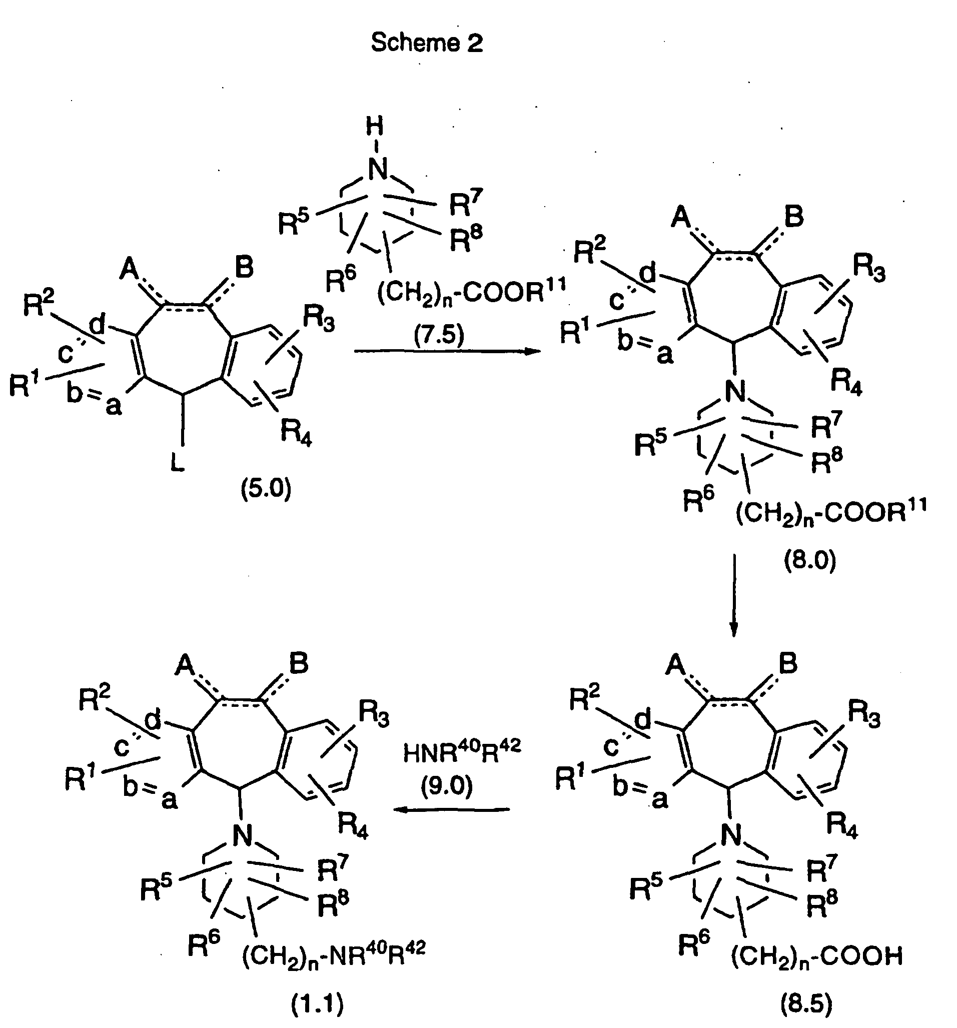 wherein L represents a leaving group, preferably chloro; the dotted line represents a single or double bond; and a, b, c, d, A, B, R¹, R², R³, R⁴, R⁵, R⁶, R⁷, R⁸, R⁸, R¹¹, R⁴⁰, R⁴² and n are as defined hereinbefore.

Referring to the Scheme 2, compounds of formula (8.0) can be prepared by reacting the compounds of formula (5.0) with a piperdinyl carboxylic acid ester of formula (7.5) in the presence of a base and optional aprotic solvent, in amounts and under conditions effective to give compounds (8.0). Suitable bases and aprotic solvents are described hereinbefore. The amounts of piperidinyl compound (7.5) can range from about 1 to about 10 moles per mole of compound (5.0). Temperatures can range from room temperature to about 80°C. Compound (8.0) can be isolated as described hereinbefore.

Carboxylic acid compounds of formula (8.5) can be prepared by hydrolyzing carboxylic acid ester (8.0) with an excess amount of acid or base. Suitable acids include inorganic acids, organic acids or a mixture thereof. Inorganic acids include hydrogen chloride, hydrogen bromide, sulfuric acid, nitric acid, phosphoric acid, perchloric acid and the like. Organic acids include acetic, citric, formic, maleic, tartaric, methanesulfonic acid and arylsulfonic acids. Suitable bases, such as sodium hydroxide or lithium hydroxide, preferably in an aqueous alcohol, have been described hereinbefore. The temperature can range from about 0°C to about 100°C.

The desired amide compounds of formula (1.1) can be prepared by reacting the compounds of formula (8.5) with a suitable amine of formula (9.0) in the presence of a coupling agent such as DEC/HOBT, a base such as NMM and a suitable aprotic solvent effective to give amide compound (1.1).
Suitable bases and aprotic solvents are described hereinbefore. The amounts of amine (9.0) can range from about 1 to about 10 moles per mole of carboxylic acid (8.5). Temperatures can range from 0° to 100°C. Compound (1.1) can be isolated as described hereinbefore.

Compounds of the present invention and preparative starting materials therof, are exemplified by the following examples, which should not be construed as limiting the scope of the disclosure.

### EXAMPLE 1. 1-[8-Chloro-6,11-dihydro-5H-benzo[5,6]cyclohepta[1,2-b] pyridin-11-yl]-N-(4-pyridinyl)-4-piperidinecarboxamide

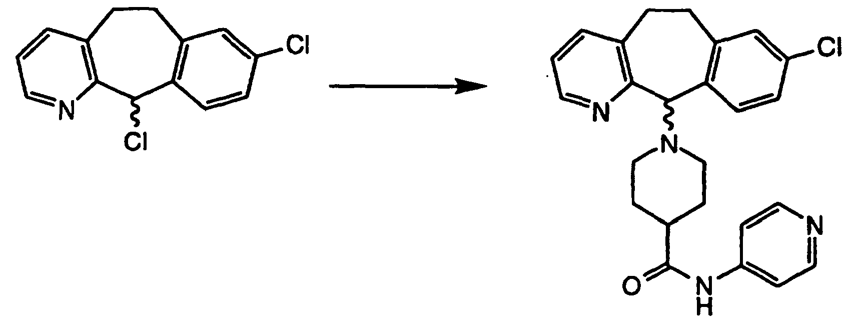

8,11-Dichloro-6,11-dihydro-5H-benzo[5,6]cyclohepta[1,2-b] pyridine (prepared as described in Preparative Example 7, Step B in INO291K) (0.088g; 1 equivalent) in anhydrous toluene (0.819 ml) is added to anhydrous DMSO (1.5ml). 4-Piperidinyl-N-(4-pyridinyl)carboxmide (0.0684g; 1 equivalent) (prepared as described in Preparative Example 1, Step C below) is added and the mixture is stirred at 25°C for 22h. The mixture is diluted with dichloromethane and washed with water. The dichloromethane layer is dried over magnesium sulfate, filtered and evaporated to dryness. The product is chromatographed on a silica gel column (15X2.5cm) using 1% increasing to 8% (10% concentrated ammonium hydroxide in methanol)-dichloromethane as the eluant to give the title compound (0.0272g; 19% yield), CIMS: m/z 433 (MH⁺).
FPT IC₅₀ = 9.24 µM

| δ_{c} (CDCl₃) | | |
|---|---|---|
| Tricyclic | CH₂: | 30.4, 30.2 |
| | CH: | 146.1, 139.5, 130.9, 123.4, 126.1, 132.4, 80.2 |
| | C: | 141.3, 135.2, 136.7, 134.0, 158.1 |
| Piperidine | CH₂: | 29.0, 51.4, 51.2, 28.7 |
| | CH: | 44.3 |
| | C: | 175.3 |
| Piperidine N-substituent | CH: | 150.6, 113.9, 113.9, 150.6 |
| | C: | 146.1 |

### EXAMPLE 2. 1-(3-Bromo-8-chloro-6,11-dihydro-5H-benzo[5,6]cyclohepta[1,2-b]pyridin-11-yl)-N-(4-pyridinyl)-4-piperidinecarboxamide

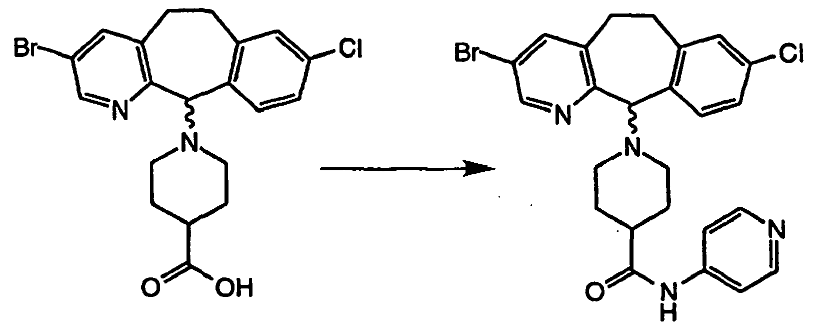

1-[3-Bromo-8-chloro-6,11-dihydro-5H-benzo[5,6]cyclohepta[1,2-b] pyridin-11-yl]-4-piperidinecarboxylate (0.25g) (1 equivalent) (prepared as described in Preparative Example 2 below) dissolved in anhydrous DMF (9ml) is added to a solution of 4-aminopyridine (0.0761g) (1.5 equivalents), DEC (0.155g) (1.5 equivalents), HOBT (0.1093g) (1.5 equivalents) and N-methylmorpholine (0.0889ml) (1.5 equivalents) in anhydrous DMF (4ml) and the mixture is stirred at 25°C for 42h. The solution is evaporated to dryness and the residue is taken up in dichloromethane and washed with 1.0N sodium hydroxide. The dichloromethane layer is dried over magnesium sulfate, filtered and evaporated to dryness. The product is chromatographed on a silica gel column (60X2.5cm) using 1.5% increasing to 3% (10% concentrated ammonium hydroxide in methanol)-dichloromethane as the eluant to give the title compound (0.0308g; 12%), CIMS: m/z 511 (MH⁺).
FPT Inhibition = 0% @ 0.39 µM

| δ_{c} (CDCl₃) | | |
|---|---|---|
| Tricyclic | CH₂: | 30.4, 29.9 |
| | CH: | 146.9, 141.3, 132.2, 126.1, 130.5, 79.4 |
| | C: | 119.8, 140.7, 134.0, 136.1, 136.7, 156.5 |
| Piperidine | CH₂: | 29.0, 51.2, 51.5, 30.4 |
| | CH: | 44.5 |
| | C: | 174.6 |
| Piperidine N-substituent | CH: | 150.5, 113.8, 113.8, 150.5 |
| | C: | 145.9 |

### EXAMPLE 3. 1-(3-Bromo-8-chloro-6,11-dihydro-5H-benzo[5,6]cyclohepta[1,2-b]pyridin-11-yl)-N-(4-pyridinylmethyl)-4-piperidinecarboxamide

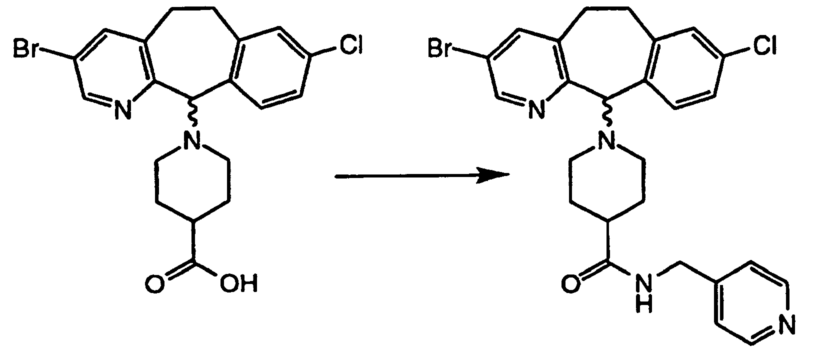

1-(3-Bromo-8-chloro-6,11-dihydro-5H-benzo[5,6]cyclohepta[1,2-b] pyridin-11-yl)-4-piperidinecarboxylate (0.25g; 1 equivalent) (prepared as described in Preparative Example 2 below) dissolved in anhydrous DMF (9ml) is added to a solution of 4-aminomethylpyridine (0.0821 ml; 1.5 equivalents), DEC (0.155g; 1.5 equivalents), HOBT (0.1093g; 1.5 equivalents) and N-methylmorpholine (0.0889ml; 1.5 equivalents) in anhydrous DMF (4ml) and the mixture is stirred at 25°C for 19h. The solution is evaporated to dryness and the residue is taken up in dichloromethane and washed with 1.0N sodium hydroxide. The dichloromethane layer is dried over magnesium sulfate, filtered and evaporated to dryness. The product is chromatographed on a silica gel column (30X2.5cm) using 2% increasing to 3% (10% concentrated ammonium hydroxide in methanol)-dichloromethane as the eluant to give the title compound (0.2128g; 75% yield), FABMS: m/z 524.9 (MH⁺).
FPT Inhibition = 21% @ 1.1 µM

| δ_{c} (CDCl₃) | | |
|---|---|---|
| Tricyclic | CH₂: | 30.4, 30.3 |
| | CH: | 146.9, 141.2, 132.2, 126.1, 130.6, 79.4 |
| | C: | 119.9, 140.7, 134.0, 136.1, 136.7, 156.5 |
| Piperidine | CH₂: | 29.2, 51.4, 51.6, 29.2 |
| | CH: | 43.3 |
| | C: | 175.3 |
| Piperidine N-substituent | CH₂: | 42.1 |
| | CH: | 122.3, 149.9, 149.9, 122.3 |
| | C: | 147.7 |

### EXAMPLE 4. 1-(3-Bromo-8-chloro-6,11-dihydro-5H-benzo[5,6]cyclohepta[1,2-b]pyridin-11-yl)-N-(3-pyridinylmethyl)-4-piperidinecarboxamide

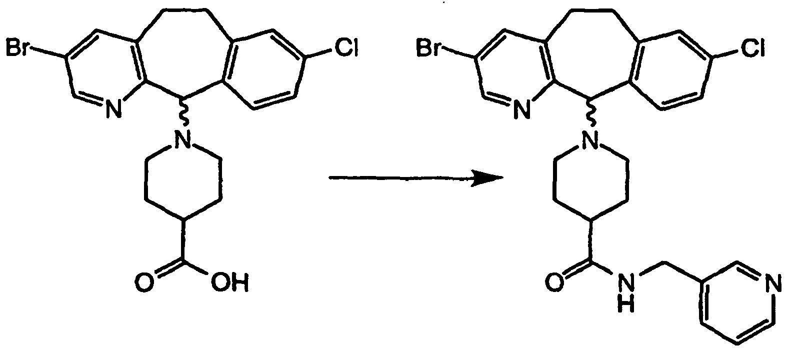

1-(3-Bromo-8-chloro-6,11-dihydro-5H-benzo[5,6]cyclohepta[1,2-b] pyridin-11-yl)-4-piperidinecarboxylate (0.25g; 1 equivalent) (prepared as described in Preparative Example 2 below) dissolved in anhydrous DMF (9ml) is added to a solution of 3-aminomethylpyridine (0.0823ml; 1.5 equivalents), DEC (0.155g; 1.5 equivalents), HOBT (0.1093g; 1.5 equivalents) and N-methylmorpholine (0.0889ml; 1.5 equivalents) in anhydrous DMF (4ml) and the mixture is stirred at 25°C for 18h. The solution is evaporated to dryness and the residue is taken up in dichloromethane and washed with 1.0N sodium hydroxide. The dichloromethane layer is dried over magnesium sulfate, filtered and evaporated to dryness. The product is chromatographed on a silica gel column (60X2.5cm) using 2% (10% concentrated ammonium hydroxide in methanol)-dichloromethane as the eluant to give the title compound (0.246g; 87%), FABMS: m/z 525 (MH⁺).
FPT IC₅₀ = 1.3 µM

| δ_{c} (CDCl₃) | | |
|---|---|---|
| Tricyclic | CH₂: | 30.4, 30.3 |
| | CH: | 146.9, 141.3, 132.3, 126.1, 130.6, 79.4 |
| | C: | 119.9, 140.7, 134.0, 136.2, 136.7, 156.6 |
| Piperidine | CH₂: | 29.2, 51.4, 51.7, 29.2 |
| | CH: | 43.4 |
| | C: | 175.2 |
| Piperidine N-substituent | CH₂: | 40.9 |
| | CH: | 149.1, 135.7, 123.7, 148.8 |
| | C: | 134.2 |

### EXAMPLE 5. 1-(3-Bromo-8-chloro-6,11-dihydro-5H-benzo[5,6]cyclohepta[1,2-b]pyridin-11-yl)-N-(2-pyridinyimethyl)-4-piperidinecarboxamide

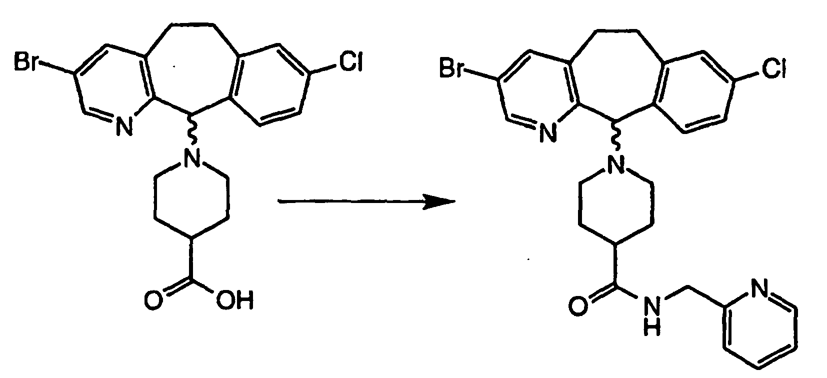

1-(3-Bromo-8-chloro-6,11-dihydro-5H-benzo[5,6]cyclohepta[1,2-b] pyridin-11-yl)-4-piperidinecarboxylate (0.25g; 1 equivalent) (prepared as described in Preparative Example 2 below) dissolved in anhydrous DMF (9ml) is added to a solution of 2-aminomethylpyridine (0.0834ml; 1.5 equivalents), DEC (0.155g; 1.5 equivalents), HOBT (0.1093g; 1.5 equivalents) and N-methylmorpholine (0.0889ml; 1.5 equivalents) in anhydrous DMF (4ml) and the mixture is stirred at 25°C for 18h.
The solution is evaporated to dryness and the residue is taken up in dichloromethane and washed with 1.0N sodium hydroxide. The dichloromethane layer is dried over magnesium sulfate, filtered and evaporated to dryness. The product is chromatographed on a silica gel column (60X2.5cm) using 0.85% (10% concentrated ammonium hydroxide in methanol)-dichloromethane as the eluant to give the title compound (0.2475g; 87% yield), FABMS: m/z 525 (MH⁺).
FPT IC₅₀ = 1.8 µM

| δ_{c} (CDCl₃) | | |
|---|---|---|
| Tricyclic | CH₂: | 30.4, 30.3 |
| | CH: | 146.9, 141.2, 132.3, 126.1, 130.6, 79.5 |
| | C: | 119.8, 140.7, 133.9, 136.3, 136.7, 156.7 |
| Piperidine | CH₂: | 29.1, 51.5, 51.7, 29.1 |
| | CH: | 43.4 |
| | C: | 175.1 |
| Piperidine N-substituent | CH₂: | 44.2 |
| | CH: | 122.4, 137.1, 122.2, 148.9 |
| | C: | 156.2 |

### EXAMPLE 6. 1-(3-Bromo-8-chloro-6,11-dihydro-5H-benzo[5,6]cyclohepta[1,2-b]pyridin-11-yl)-N-(2-pyridiriylethyl)-4-piperidinecarboxamide

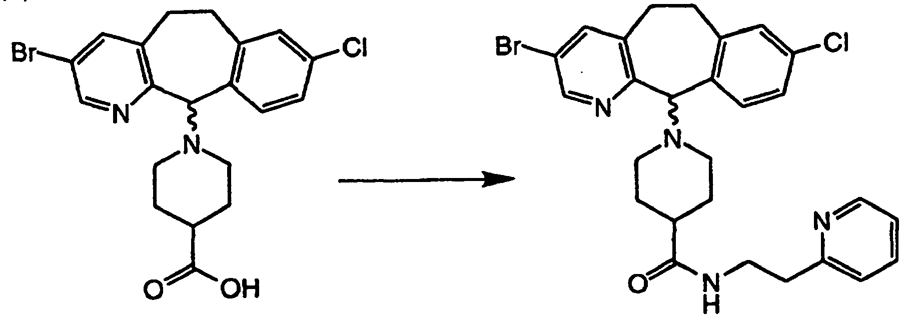

1-(3-Bromo-8-chloro-6,11-dihydro-5H-benzo[5,6]cyclohepta[1,2-b] pyridin-11-yl)-4-piperidinecarboxylate (0.4g; 1 equivalent) (prepared as described in Preparative Example 2 below) dissolved in anhydrous DMF (14ml) is added to a solution of 2-aminoethylpyridine (0.134ml; 1.3 equivalents), DEC (0.215g; 1.3 equivalents), HOBT (0.1515g; 1.3 equivalents) and N-methylmorpholine (0.123m1; 2.6 equivalents) in anhydrous DMF (6ml) and the mixture is stirred at 25°C for 67h. The solution is evaporated to dryness and the residue is taken up in dichloromethane and washed with 1.0N sodium hydroxide. The dichloromethane layer is dried over magnesium sulfate, filtered and evaporated to dryness. The product is chromatographed on a silica gel column (60X2.5cm) using 1% increasing to 2% (10% concentrated ammonium hydroxide in methanol)-dichloromethane as the eluant to give the title compound (0.4003g; 86% yield), FABMS: m/z 539.2 (MH⁺).
FPT Inhibition = 9% @ 1.1 µM

| δ_{c} (CDCl₃) | | |
|---|---|---|
| Tricyclic | CH₂: | 30.4, 30.3 |
| | CH: | 146.9, 141.2, 132.3, 126.1, 130.6, 79.5 |
| | C: | 119.8, 140.7, 133.9, 136.4, 136.7, 156.8 |
| Piperidine | CH₂: | 29.1, 29.1, 51.5, 51.7 |
| | CH: | 43.5 |
| | C: | 174.9 |
| Piperidine N-substituent | CH₂: | 38.6,36.7 |
| | CH: | 123.6, 136.9, 121.7, 149.0 |
| | C: | 159.7 |

### EXAMPLE 7. 1-(3-Bromo-8-chloro-6,11-dihydro-5H-benzo[5,6]cyclohepta[1,2-b]pyridin-11-yl)-N-[4-(N-carboxamidopiperidinyl)]-4-piperidinecarboxamide

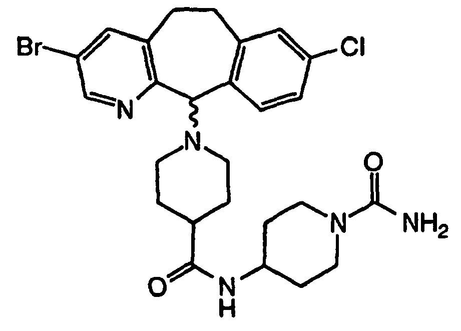

### Step A:

### 1-(3-Bromo-8-chloro-6,11-dihydro-5H-benzo[5,6]cyclohepta[1,2-b]pyridin-11-yl)-N-[4-(N-benzylpiperidinyl)]-4-piperidinecarboxamide

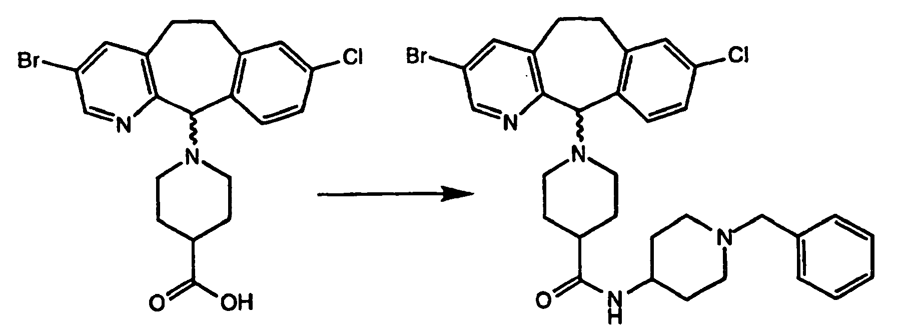

1-(3-Bromo-8-chloro-6,11-dihydro-5H-benzo[5,6]cyclohepta[1,2-b] pyridin-11-yl)-4-piperidinecarboxylate (0.8g; 1 equivalent) (prepared as described in Preparative Example 2 below) dissolved in anhydrous DMF (29ml) is added to a solution of 1-N-benzyl-4-aminopiperidine (0.4573ml) (1.3 equivalents), DEC (0.43g; 1.3 equivalents), HOBT (0.303g; 1.3 equivalents) and N-methylmorpholine (0.494ml; 2.6 equivalents) in anhydrous DMF (12.8ml) and the mixture is stirred at 25°C for 18h. The solution is evaporated to dryness and the residue is taken up in dichloromethane and washed with 1.0N sodium hydroxide. The dichloromethane layer is dried over magnesium sulfate, filtered and evaporated to dryness. The product is chromatographed on a silica gel column (60X2.5cm) using 2% (10% concentrated ammonium hydroxide in methanol)-dichloromethane as the eluant to give the title compound (0.8143g; 78% yield), FABMS: m/z 607.1 (MH⁺).

| δ_{c} (CDCl₃) | | |
|---|---|---|
| Tricyclic | CH₂: | 30.4, 30.3 |
| | CH: | 146.9, 141.2, 132.2, 126.1, 130.6, 79.5 |
| | C: | 119.8, 140.7, 133.9, 136.3, 136.7, 156.8 |
| Piperidine | CH₂: | 29.2, 51.4, 51.7, 29.2 |
| | CH: | 43.6 |
| | C: | 174.3 |
| Piperidine N-substituent | CH₂: | 52.3, 52.3, 32.3, 32.3, 63.0 |
| | CH: | 46.3, 128.3, 128.3, 129.2, 129.2, 127.2 |
| | C: | 138.1 |

### Step B:

### 1-(3-Bromo-8-chloro-6,11-dihydro-5H-benzo[5,6]cyclohepta[1,2-b]pyridin-11-yl)-N-(4-piperidinyl)-4-piperidinecarboxamide

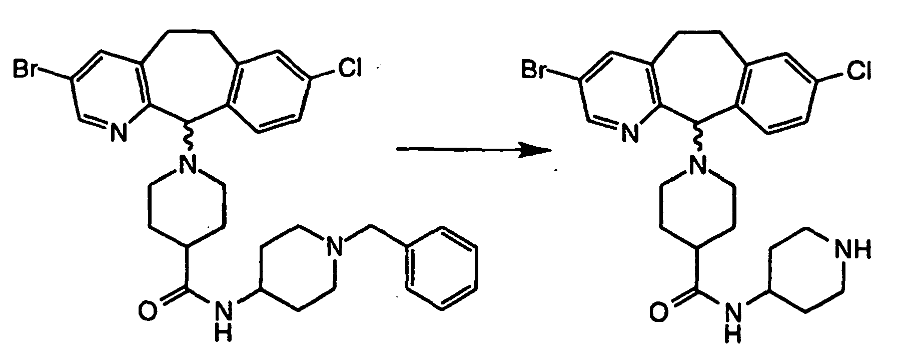

1-(3-Bromo-8-chloro-6,11-dihydro-5H-benzo[5,6]cyclohepta[1,2-b] pyridin-11-yl)-N-[4-(N-benzylpiperidinyl)]-4-piperidinecarboxamide (0.51g; 1 equivalent) (prepared as described in Step A above) is dissolved in anhydrous dichloromethane (3ml) and the solution is cooled to 0°C. α-Chloroethoxycarbonyl chloride (0.09027ml; 1 equivalent) is added over 5 minutes and the solution is allowed to warm to 25°C over 1 h. The dichloromethane is removed *in vacuo* and anhydrous methanol (14ml) is added. The solution is heated under reflux for 1 h. The solution is evaporated to dryness and the residue is taken up in dichloromethane and washed with 1.0N sodium hydroxide. The dichloromethane layer is dried over magnesium sulfate, filtered and evaporated to dryness. The product is chromatographed on a silica gel column (60X2.5cm) using 2.5% (10% concentrated ammonium hydroxide in methanol)-dichloromethane as the eluant to give unreacted 1-(3-bromo-8-chloro-6,11-dihydro-5H-benzo[5,6]cyclohepta [1,2-b]pyridin-11-yl)-N-[4-(N-benzylpiperidinyl)]-4-piperidinecarboxamide (0.1921 g; 38%yield) and the title compound (0.199g;46%), FABMS: m/z 517.5 (MH⁺).
FPT Inhibition = 8.75% @ 0.39 µM

| δ_{c} (CDCl₃+CD₃OD) | | |
|---|---|---|
| Tricyclic | CH₂: | 30.3, 30.2 |
| | CH: | 146.6, 141.4, 132.2, 126.1, 130.5, 79.2 |
| | C: | 119.8, 140.6, 133.9, 136.1, 136.9, 156.7 |
| Piperidine | CH₂: | 29.0, 51.4, 51.7,29.0 |
| | CH: | 43.4 |
| | C: | 175.0 |
| Piperidine N-substituent | CH₂: | 44.9, 44.9, 32.0, 32.0 |
| | CH: | 46.0 |

### Step C:

### 1-(3-Bromo-8-chloro-6,11-dihydro-5H-benzo[5,6] cyclohepta[1,2-b]pyridin-11-yl)-N-[4-(N-carboxamidopiperidinyl)]-4-piperidinecarboxamide

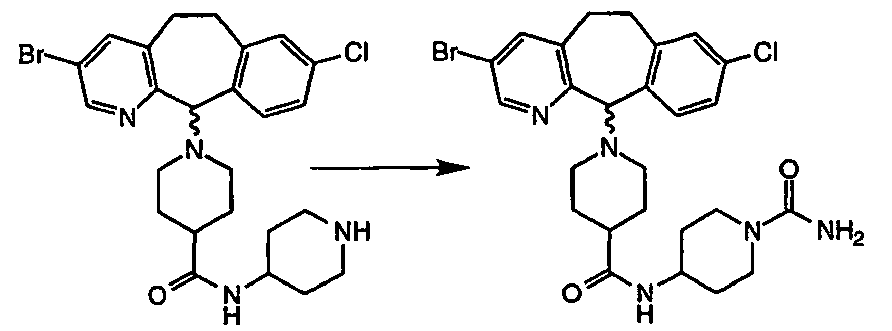

1-(3-Bromo-8-chloro-6,11-dihydro-5H-benzo[5,6]cyclohepta[1,2-b] pyridin-11-yl)-N-(4-piperidinyl)-4-piperidinecarboxamide (0.1191g; 1 equivalent) (prepared as described in Step B above) is dissolved in anhydrous dichloromethane (3.3ml). Trimethylsilylisocyanate (0.467ml; 15 equivalents) is added and the mixture is stirred under argon at 25°C for 22h. Additional trimethylsilylisocyanate (0.156ml; 5 equivalents) is added and the mixture is stirred for a total of 27h. The solution is diluted with dichloromethane and washed with saturated aqueous sodium bicarbonate. The dichloromethane layer is dried over magnesium sulfate, filtered and evaporated to dryness. The product is chromatographed on a silica gel column (15X2.5cm) using 4% (10% concentrated ammonium hydroxide in methanol)-dichloromethane as the eluant to give the title compound (0.0678g; 53% yield), FABMS: m/z 560 (MH⁺). FPT Inhibition =15% @ 0.36 µM

| δ_{c} (CDCl₃) | | |
|---|---|---|
| Tricyclic | CH₂: | 30.4, 30.3 |
| | CH: | 146.8, 141.2, 132.2, 126.1, 130.6, 79.4 |
| | C: | 119.8, 140.7, 133.9, 136.2, 136.7, 156.7 |
| Piperidine | CH₂: | 29.1, 51.4, 51.7, 29.1 |
| | CH: | 43.5 |
| | C: | 174.7 |
| Piperidine N-substituent | CH₂: | 43.3, 43.3, 31.9, 31.9 |
| | CH: | 46.3 |
| | C: | 158.1 |

### EXAMPLE 8. 1-(3-Bromo-8-chloro-6,11-dihydro-5H-benzo[5,6]cyclohepta[1,2-b] pyridin-11-yl)-N-[4-(N-carboxamidopiperidinyl)methyl]-4-piperidinecarboxamide

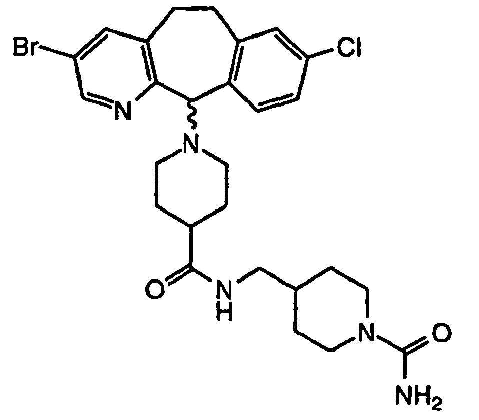

### Procedure 1, Step A:

### 1-(3-Bromo-8-chloro-6,11-dihydro-5H-benzo[5,6] cyclohepta[1,2-b]pyridin-11-yl)-N-[4-(N-benzylpiperidinyl)methyl]-4-piperidinecarboxamide

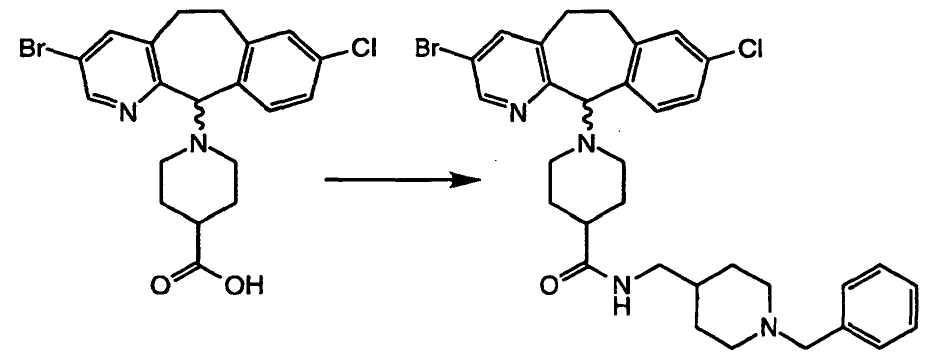

1-(3-Bromo-8-chloro-6,11-dihydro-5H-benzo[5,6]cyclohepta[1,2-b] pyridin-11-yl)-4-piperidinecarboxylate (0.8g; 1 equivalent) (prepared as described in Preparative Example 2 below) dissolved in anhydrous DMF (29ml) is added to a solution of 1-N-benzyl-4-aminomethylpiperidine (0.4581g; 1.3 equivalents) (prepared as described in Preparative Example 4, Step B below), DEC (0.43g) (1.3 equivalents), HOBT (0.303g; 1.3 equivalents) and N-methylmorpholine (0.493ml; 2.6 equivalents) in anhydrous DMF (12.8ml) and the mixture is stirred at 25°C for 24h. The solution is evaporated to dryness and the residue is taken up in dichloromethane and washed with 1.0N sodium hydroxide. The dichloromethane layer is dried over magnesium sulfate, filtered and evaporated to dryness. The product is chromatographed on a silica gel column (60X2.5cm) using 2% (10% concentrated ammonium hydroxide in methanol)-dichloromethane as the eluant to give the title compound (0.7475g; 70% yield), FABMS: m/z 621.6 (MH⁺).

| δ_{c} (CDCl₃) | | |
|---|---|---|
| Tricyclic | CH₂: | 30.4, 30.3 |
| | CH: | 146.9, 141.2, 132.2, 126.1, 130.6, 79.5 |
| | C: | 119.8, 140.7, 133.9, 136.3, 136.7, 156.8 |
| Piperidine | CH₂: | 29.3, 51.5, 51.8, 29.3 |
| | CH: | 43.7 |
| | C: | 175.1 |
| Piperidine N-substituent | CH₂: | 53.3, 29.9, 29.9, 53.3, 63.4, 44.9 |
| | CH: | 36.0, 128.2, 129.2, 127.0, 129.2, 128.2 |
| | C: | 138.3 |

### Step B:

### 1-(3-Bromo-8-chloro-6,11-dihydro-5H-benzo[5,6]cyclohepta[1,2-b]pyridin-11-yl)-N-(4-piperidinyl)-4-piperidinecarboxamide

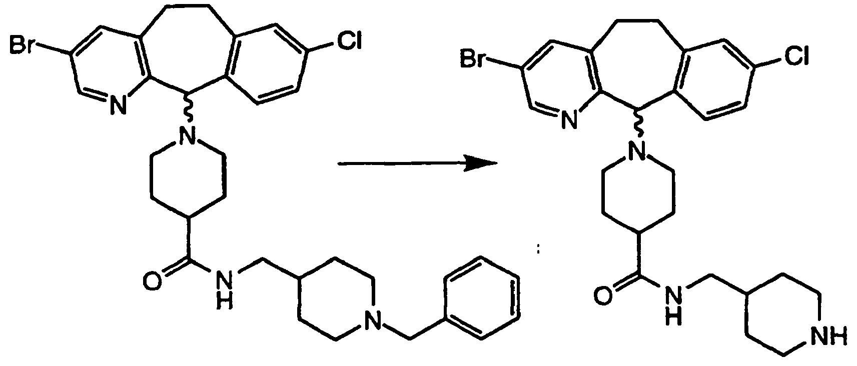

1-(3-Bromo-8-chloro-6,11-dihydro-5H-benzo[5,6]cyclohepta[1,2-b] pyridin-11-yl)-N-[4-(N-benzylpiperidinylmethyl)]-4-piperidinecarboxamide (0.568g; 1 equivalent) (prepared as described in Step A above) is dissolved in anhydrous dichloromethane (5.9ml) and the solution is cooled to 0°C. α-Chloroethoxycarbonyl chloride (0.487ml; 5 equivalents) is added over 30 minutes and the solution is allowed to warm to 25°C over 2.5h. The dichloromethane is removed *in vacuo* and anhydrous methanol (14.2ml) is added. The solution is heated under reflux for 1.25h. The solution is evaporated to dryness and the residue is taken up in dichloromethane and washed with 1.0N sodium hydroxide. The dichloromethane layer is dried over magnesium sulfate, filtered and evaporated to dryness. The product is chromatographed on a silica gel column (30X2.5cm) using 2% (10% concentrated ammonium hydroxide in methanol)-dichloromethane as the eluant to give unreacted 1-(3-bromo-8-chloro-6,11-dihydro-5H-benzo[5,6]cyclohepta [1,2-b]pyridin-11-yl)-N-[4-(N-benzylpiperidinyl)]-4-piperidinecarboxamide (0.1487g; 23% yield) and the title compound (0.1932g; 34% yield), FABMS: m/z 531.0 (MH⁺). The title compound is identical to that prepared in Procedure 2, Step B below. FPT Inhibition = 12% @ 0.38 µM

| δ_{c} (CDCl₃) | | |
|---|---|---|
| Tricyclic | CH₂: | 30.4, 30.3 |
| | CH: | 146.9, 141.2, 132.2, 126.1, 130.6, 79.5, |
| | C: | 119.8, 140.7, 133.9, 136.3, 136.7, 156.7 |
| Piperidine | CH₂: | 29.3,51.5,51.7,29.3 |
| | CH: | 43.7 |
| | C: | 175.2 |
| Piperidine N-substituent | CH₂: | 30.9, 30.9, 46.2, 46.2, 45.2 |
| | CH: | 36.5 |

### Step C:

### 1-(3-Bromo-8-chloro-6,11-dihydro-5H-benzo[5,6]cyclohepta[1,2-b]pyridin-11-yl)-N-[4-(N-carboxamidopiperidinyl)methyl]-4-piperidinecarboxamide

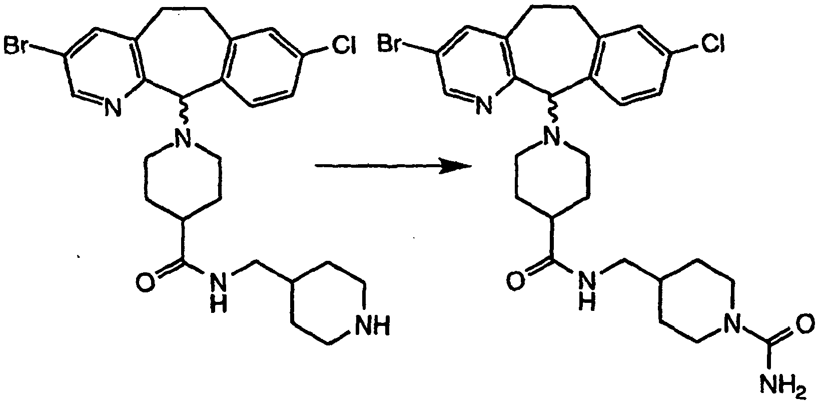

1-(3-Bromo-8-chloro-6,11-dihydro-5H-benzo[5,6]cyclohepta[1,2-b]pyridin-11-yl)-N-(4-piperidinylmethyl)-4-piperidinecarboxamide (0.005g; 1 equivalent) (prepared as described in Step B above) is dissolved in anhydrous dichloromethane (0.161ml). Trimethylsilylisocyanate (0.0038ml; 3 equivalents) is added and the mixture is stirred at 25°C under argon for 16h.
The mixture is diluted with dichloromethane and washed with saturated aqueous sodium bicarbonate. The dichloromethane layer is dried over magnesium sulfate, filtered and evaporated to dryness to give the title compound which is identical on thin layer chromatography (TLC) to that prepared in Procedure 2, Step C below.

### Procedure 2, Step A:

### 1-(3-Bromo-8-chloro-6,11-dihydro-5H-benzo[5,6]cyclohepta[1,2-b]pyridin-11-yl)-N-[4-(N-tert-butoxycarbonylpiperidine)methyl]-4-piperidinecarboxamide

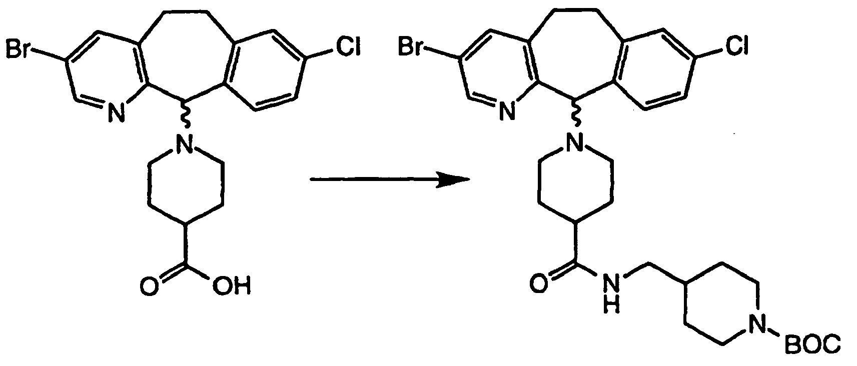

1-(3-Bromo-8-chloro-6,11-dihydro-5H-benzo[5,6]cyclohepta[1,2-b]pyridin-11-yl)-4-piperidinecarboxylate (0.5g; 1 equivalent) (prepared as described in Preparative Example 2 below) dissolved in anhydrous DMF (18ml) is added to a solution of 1-N-*tert*-butoxycarbonyl-4-aminomethylpiperidine (0.1778g; 1 equivalent) (prepared as described in Preparative Example 3, Step C below), DEC (0.2067g; 1.3 equivalents), HOBT (0.1457g; 1.3 equivalents) and N-methylmorpholine (0.1185ml; 1.3 equivalents) in anhydrous DMF (8ml) and the mixture is stirred at 25°C for 19h. The solution is evaporated to dryness and the residue is taken up in dichloromethane and washed with 1.0N sodium hydroxide. The dichloromethane layer is dried over magnesium sulfate, filtered and evaporated to dryness. The product is chromatographed on a silica gel column (60X2.5cm) using 0.75% (10% concentrated ammonium hydroxide in methanol)-dichloromethane as the eluant to give the title compound (0.4805g; 71% yield), FABMS: m/z 631 (MH⁺).

| δ_{c} (CDCl₃) | | |
|---|---|---|
| Tricyclic | CH₂: | 30.4, 30.3 |
| | CH: | 146.9, 141.2, 132.2, 126.1. 130.6. 79.5 |
| | C: | 119.8, 140.7, 133.9, 136.3. 136.7, 156.7 |
| Piperidine | CH₂: | 29.8,51.7,51.4,29.3 |
| | CH: | 43.6 |
| | C: | 175.2 |
| Piperidine N-substituent | CH₃: | 28.5 |
| | CH₂: | 43.6, 43.6, 29.3, 29.3, 44.7 |
| | CH: | 36.4 |
| | C: | 79.5, 154.8 |

### Step B:

### 1-(3-Bromo-8-chloro-6,11-dihydro-5H-benzo[5,6]hepta[1,2-b]pyridin-11-yl)-N-(4-piperidinylmethyl)-4-piperidinecarboxamide

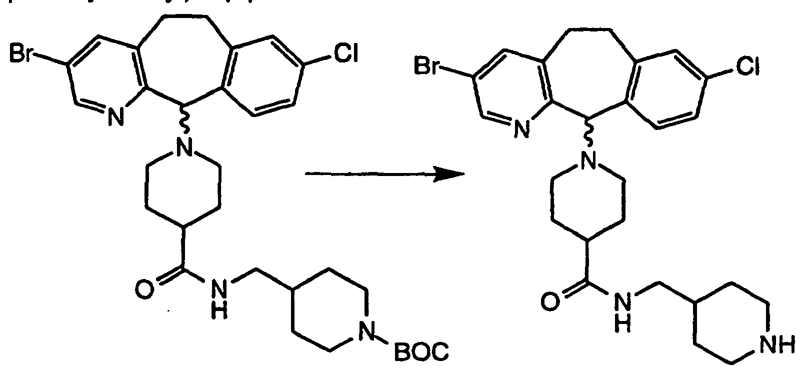

1-(3-Bromo-8-chloro-6,11-dihydro-5H-benzo[5,6]cyclohepta[1,2-b] pyridin-11-yl)-N-[4-(N-*tert* -butoxycarbonylpiperidine)methyl]-4-piperidine carboxamide (0.3936g; 1 equivalent) is dissolved in anhydrous dichloromethane (30ml). Trifluoroacetic acid (6.039ml; 127 equivalents) is added to the stirred solution at 0°C under argon. The mixture is stirred at 0°C for 1.5h and then allowed to warm to 25°C for 1 h. The mixture is diluted with dichloromethane and washed with 1.0N sodium hydroxide. The dichloromethane layer is dried over magnesium sulfate, filtered and evaporated to dryness. The product is chromatographed on a silica gel column (60X2.5cm) using 7% increasing to 10% (10% concentrated ammonium hydroxide in methanol)-dichloromethane as the eluant to give the title compound (0.2023g; 61% yield), FABMS: m/z 531.1 (MH⁺).

| δ_{c} (CDCl₃) | | |
|---|---|---|
| Tricyclic | CH₂: | 30.4, 30.3 |
| | CH: | 146.9, 141.2, 132.2, 126.1, 130.6, 79.5 |
| | C: | 119.8, 140.7, 133.9, 136.3, 136.7, 156.7 |
| Piperidine | CH₂: | 29.3, 51.4, 51.7, 29.3 |
| | CH: | 43.7 |
| | C: | 175.1 |
| Piperidine N-substituent | CH₂: | 30.7, 30.7, 46.1, 46.1, 45.2 |
| | CH: | 36.4 |

### Step C.

### 1-(3-Bromo-8-chloro-6,11-dihydro-5H-benzo[5,6] cyclohepta[1,2-b]pyridin-11-yl)-N-[4-(N-carboxamidopiperidinyl)methyl]-4-piperidinecarboxamide

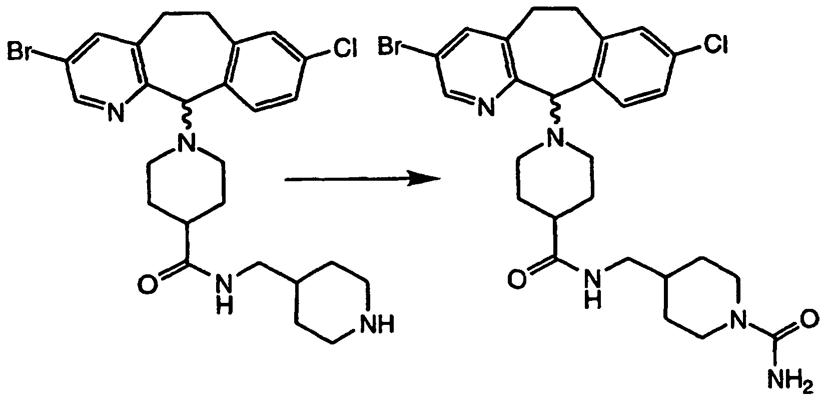

1-(3-Bromo-8-chloro-6,11-dihydro-5H-benzo[5,6]cyclohepta[1,2-b]pyridin-11-yl)-N-(4-piperidinylmethyl)-4-piperidinecarboxamide (0.140g; 1 equivalent) (prepared as described in Step B above) is dissolved in anhydrous dichloromethane (4.5ml). Trimethylsilylisocyanate (0.534ml) (15 equivalents) is added and the mixture is stirred at 25°C under argon for 18h. The mixture is diluted with dichloromethane and washed with saturated aqueous sodium bicarbonate. The dichloromethane layer is dried over magnesium sulfate, filtered and evaporated to dryness. The product is chromatographed on a silica gel column (30X2.5cm) using 4% (10% concentrated ammonium hydroxide in methanol)-dichloromethane as the eluant to give the title compound (0.1084g; 72% yield), FABMS: m/z 573.9 (MH⁺). FPT Inhibition = 41% @ 1.04 µM

| δ_{c} (CDCl₃) | | |
|---|---|---|
| Tricyclic | CH₂: | 30.4, 30.3 |
| | CH: | 146.8, 141.2, 132.2, 126.1, 130.6, 79.5 |
| | C: | 119.8, 140.7, 133.9, 136.3, 136.7, 156.7 |
| Piperidine | CH₂: | 29.3, 51.4, 51.7, 29.3 |
| | CH: | 43.5 |
| | C: | 175.4 |
| Piperidine N-substituent | CH₂: | 29.6, 29.6, 44.6, 44.6, 44.1 |
| | CH: | 36.1 |
| | C: | 158.1 |

### EXAMPLE 9. 1-[1-(3-Bromo-8-chloro-6,11-dihydro-5H-benzo[5,6]cyclohepta[1,2-b]pyridin-11-yl)-4-piperidinylcarbonyl]-4-[(1-aminomethanamido)methyl]piperidine

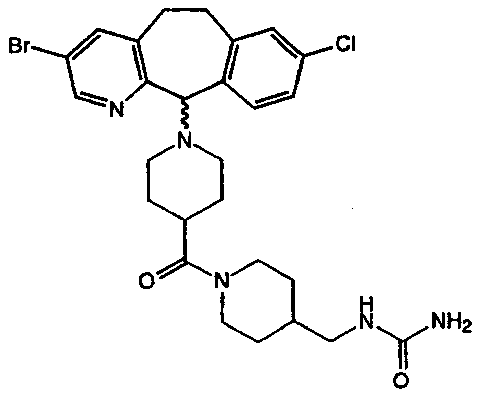

### Step A:

### 1-[1-(3-Bromo-8-chloro-6,11-dihydro-5H-benzo[5,6]cyclohepta[1,2-b]pyridin-11-yl)-4-piperidinylcarbonyl]-4-[(N-tert -butoxycarbonylamino)methyl]piperidine

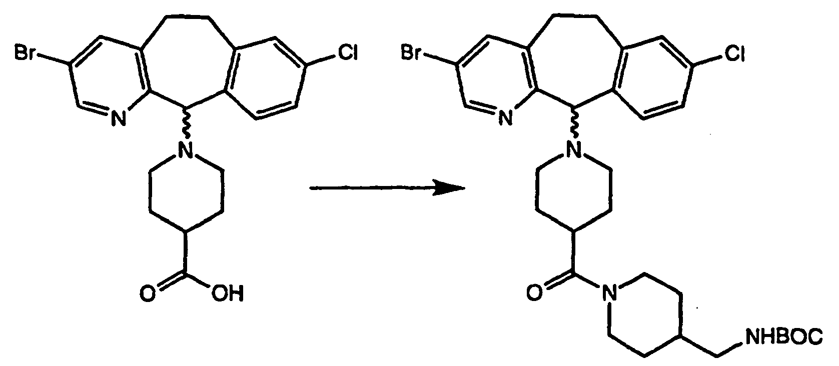

1-(3-Bromo-8-chloro-6,11-dihydro-5H-benzo[5,6]cyclohepta[1,2-b]pyridin-11-yl)-4-piperidinecarboxylate (0.3195g; 1 equivalent) (prepared as described in Preparative Example 2 below) dissolved in anhydrous DMF (11.5ml) is added to a solution of 4-[(N-*tert* -butoxycarbonylamino)-methyl]piperidine (0.1904g; 1.3 equivalents) (prepared as described in Preparative Example 5, Step C below), DEC (0.1703g; 1.3 equivalents), HOBT (0.1201g; 1.3 equivalents) and N-methylmorpholine (0.195ml; 2.6 equivalents) in anhydrous DMF and the mixture is stirred at 25°C for 19h. The solution is evaporated to dryness and the residue is taken up in dichloromethane and washed with 1.0N sodium hydroxide. The dichloromethane layer is dried over magnesium sulfate, filtered and evaporated to dryness. The product is chromatographed on a silica gel column (60X2.5cm) using 0.8% (10% concentrated ammonium hydroxide in methanol)-dichloromethane as the eluant to give the title compound (0.3701 g; 86% yield), FABMS: m/z 631.3 (MH⁺).

| δ_{c} (CDCl₃) | | |
|---|---|---|
| Tricyclic | CH₂: | 30.7,30.4 |
| | CH: | 146.8, 141.2, 132.2, 126.0, 130.6, 79.6 |
| | C: | 119.7, 140.7, 133.8, 136.4, 136.7, 156.9 |
| Piperidine | CH₂: | 51.6, 29.1, 28.9, 51.9 |
| | CH: | 38.8 |
| | C: | 173.4 |
| Piperidine N-substituent | CH₃: | 28.4 |
| | CH₂: | 45.3/45.9, 28.9/29.1, 29.5/30.2, 45.3/45.9, 41.7 |
| | CH: | 37.0 |
| | C: | 79.4, 156.1 |

### Step B:

### 1-[1-(3-Bromo-8-chloro-6,11-dihydro-5H-benzo[5,6]cyclohepta[1,2-b]pyridin-11-yl)-4-[piperidinyl]-4-aminomethylpiperidine

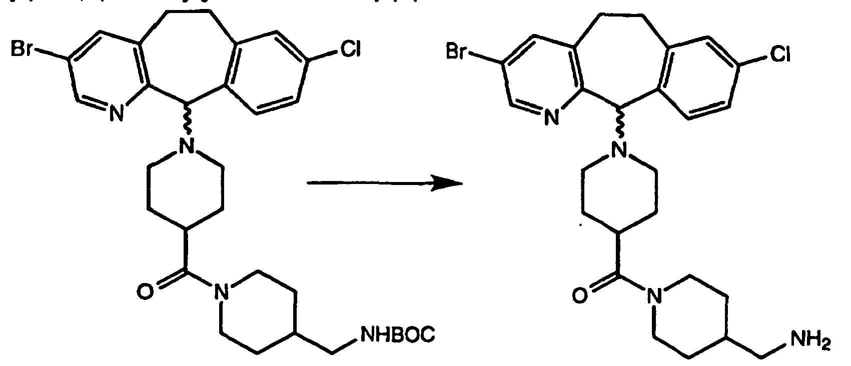

1-[1-(3-Bromo-8-chloro-6,11-dihydro-5H-benzo[5,6]cyclohepta[1,2-b]pyridin-11-yl)-4-piperidinylcarbonyl)-4-[(N-*tert* -butoxycarbonylamino) methyl]piperidine (0.35g) (1 equivalent) is dissolved in methanol (3.1ml). A 10% (v/v) solution of concentrated sulfuric acid in dioxane (7.568ml) is added and the mixture is stirred at 25°C under argon for 1.5h. The mixture is stirred at 0°C for 1.5h and then allowed to warm to 25°C for 1 h. The mixture is diluted with dichloromethane and washed with 1.0N sodium hydroxide. The dichloromethane layer is dried over magnesium sulfate, filtered and evaporated to dryness. The product is chromatographed on a silica gel column (30X2.5cm) using 4% (10% concentrated ammonium hydroxide in methanol)-dichloromethane as the eluant to give the title compound (0.226g; 77% yield), FABMS: m/z 531.4 (MH⁺). FPT Inhibition = 16% @ 0.38 µM

| δ_{c} (CDCl₃) | | |
|---|---|---|
| Tricyclic | CH₂: | 30.4, 30.2 |
| | CH: | 146.8, 141.2, 132.2, 126.0, 130.6, 79.6 |
| | C: | 119.8, 140.7, 133.8, 136.4, 136.7, 156.9 |
| Piperidine | CH₂: | 51.6, 29.1, 28.9, 51.9 |
| | CH: | 38.8 |
| | C: | 173.3 |
| Piperidine N-substituent | CH₂: | 42.0, 29.7/30.9, 29.0/29.2, 42.0, 45.5 |
| | CH: | 38.8 |

### Step C:

### 1-[1-(3-Bromo-8-chloro-6,11-dihydro-5H-benzo[5,6]cyclohepta[1,2-b]pyridin-11-yl)-4-piperidinylcarbonyl]-4-[(1-aminomethanamido)methyl]piperidine

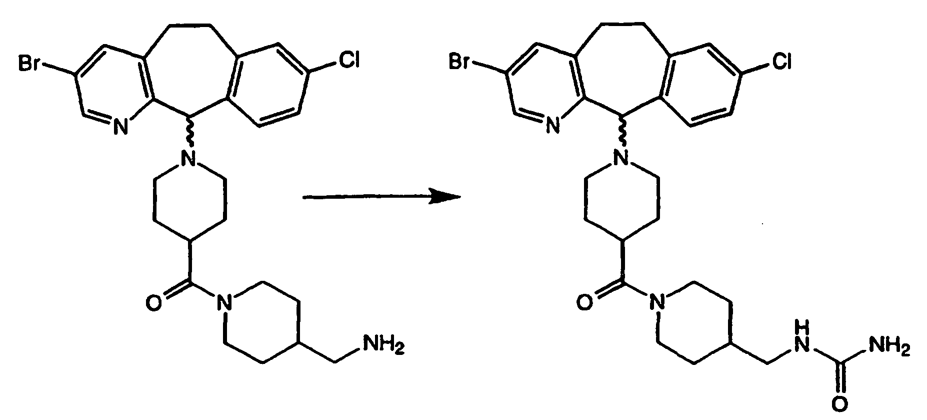

1-[1-(3-Bromo-8-chloro-6,11-dihydro-5H-benzo[5,6]cyclohepta [1,2-b] pyridin-11-yl)-4-piperidinylcarbonyl]-4-[(N-*tert*-butoxycarbonylamino) methyl]piperidine (0.185g) (1 equivalent) (prepared as described in Example 9, Step B above) is dissolved in anhydrous dichloromethane (5ml). Trimethylsilylisocyanate (0.706ml) (15 equivalents) is added and the mixture is stirred at 25°C under argon for 22h. Additional trimethylsilylisocyanate (0.235ml) (5 equivalents) is added and the stirring is continued for a total of 26.75h. The mixture is diluted with dichloromethane and washed with saturated aqueous sodium bicarbonate. The dichloromethane layer is dried over magnesium sulfate, filtered and evaporated to dryness. The product is chromatographed on a silica gel column (30X2.5cm) using 3.5% (10% concentrated ammonium hydroxide in methanol)-dichloromethane to give the title compound (0.1502g; 75% yield), FABMS: m/z 574.2 (MH⁺).
FPT IC₅₀ = 0.66 µM

| δ_{c} (CDCl₃) | | |
|---|---|---|
| Tricyclic | CH₂: | 30.4, 30.2 |
| | CH: | 146.8, 141.2, 132.2, 126.1, 130.6, 79.5 |
| | C: | 119.8, 140.7, 133.9, 136.2, 136.7, 156.7 |
| Piperidine | CH₂: | 51.5, 29.0, 28.9, 51.8 |
| | CH: | 38.8 |
| | C: | 173.6 |
| Piperidine N-substituent | CH₂: | 41.9, 41.9, 30.7, 29.6, 45.4 |
| | CH: | 36.9 |
| | C: | 159.2 |

### EXAMPLE 10. 1-(8-chloro-3-bromo-5,6-dihydro-11H-benzo[5,6]-cyclohepta[1,2-b]pyridin-11-yl)piperidine-3-(N-3-pyridylmethylamino)carboxamide

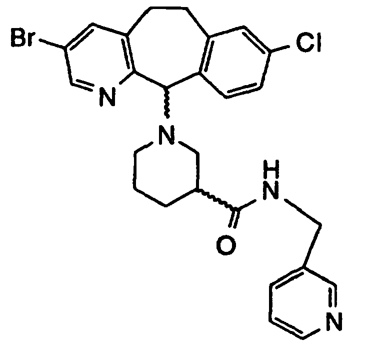

### Procedure 1, Step A.

### Ethyl 1-(3-bromo-8-chloro-6,11-dihydro-5H-benzo[5,6]cyclohepta[1,2-b]pyridin-11-yl)-3-piperidinecarboxylate

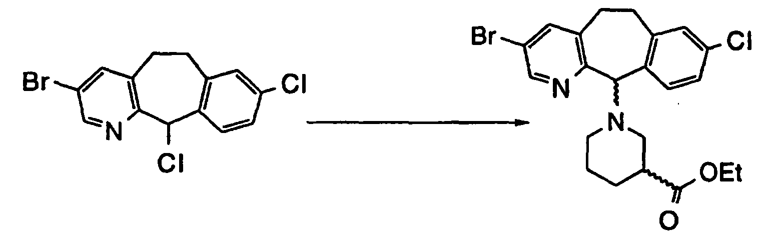

3-Bromo-8,11-dichloro-6,11-dihydro-5H-benzo[5,6]cyclohepta[1,2-b]pyridine (1 g, 2.5 mmol) is dissolved in 10 mL of N,N-dimethylformamide (DMF). Ethylnipecotate (0.6 ml, 3.7 mmol) and N-methylmorpholine (0.69 mL, 6.2 mmol) are added and the reaction mixture is stirred at ambient temperature for 18 hours. The reaction mixture is poured into water and extracted with dichloromethane two times. The combined extracts are dried over magnesium sulfate and the mixture filtered and evaporated to obtain an oil. The oil is chromatographed on silica gel using 10% ethyl acetate/hexanes as the eluent to obtain 0.55 gm of the title compound. FABMS (MH⁺)=464

### Step B.

### 1-(3-Bromo-8-chloro-6,11-dihydro-5H-benzo[5,6]cyclohepta[1,2-b]pyridin-11-yl)-3-piperidinecarboxylate

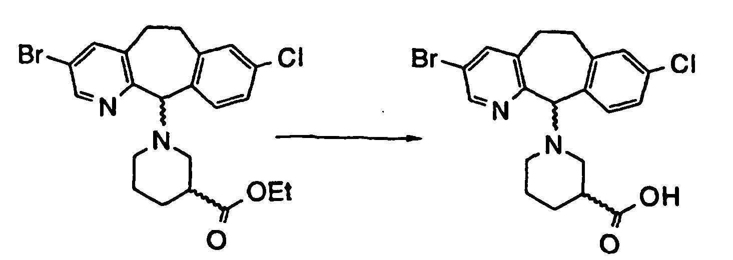

Ethyl 1-(3-bromo-8-chloro-6,11-dihydro-5H-benzo[5,6]cyclohepta[1,2-b]pyridin-11-yl)-3-piperidinecarboxylate (1.9 gm) is refluxed in 25 ml of 6N hydrochloric acid for 8 hours. The HCI and water is evaporated to obtain the title compound, as a solid.

### Step C.

### 1-(3-Bromo-8-chloro-6,11-dihydro-5H-benzo[5,6]cyclohepta[1,2-b]pyridin-11-yl)-N-(3-pyridinylmethyl)-3-piperidinecarboxamide

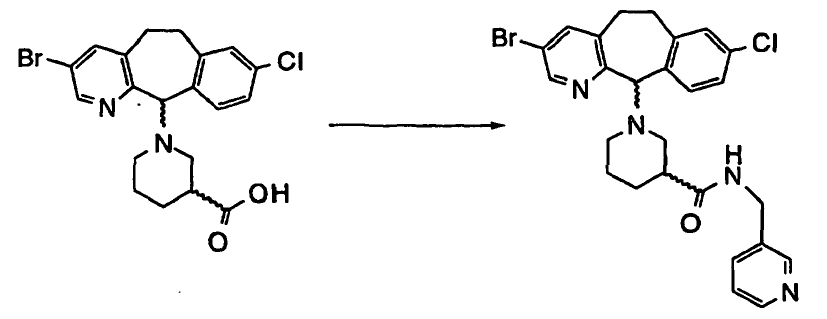

The compound from Example 10, Step B is dissolved in 12 mL of DMF and 1-(3-dimethylaminopropyl)-3-ethylcarbodiimide hydrochloride (DEC) (0.37 g 1.9 mmol), 1-hydroxybenzotriazole (HOBT) (0.36g), NMM (0.5 mL) and (0.216 g, 2.0 mmol) of 3-aminomethylpyridine are added and the reaction mixture is stirred at ambient temperature. After 17 hours the reaction mixture is poured into water and extracted with dichloromethane two times. The combined extracts are dried over magnesium sulfate and the mixture filtered and evaporated to obtain an oil. The oil is chromatographed on silica gel using 5% methanol/dichloromethane as the eluent to obtain 0.44 gm of the title compound.
FABMS (MH⁺)=603
FPT IC₅₀ = 0.21 µM

### Step D. Separation of Isomers

The compound of Example 10, Procedure 1, Step C, is separated into its four optical isomers by HPLC chromatography with a Chiralpak® AD analytical (0.46cmX25cm) chiral column (amylose tris(3,5-dimethylphenyl carbamate) coated on a 10 µM silica-gel substrate (trademark of of Chiral Technologies, Exton, Pennsylvania), using as the eluting solvent, 20% isopropanlol/hexanes/.02% diethylamine at 1mL/minute, the four compounds elute at 10.27 (Isomer A), 11.43 (Isomer B), 11.57 (Isomer C) and 18.37 (Isomer D) minutes.

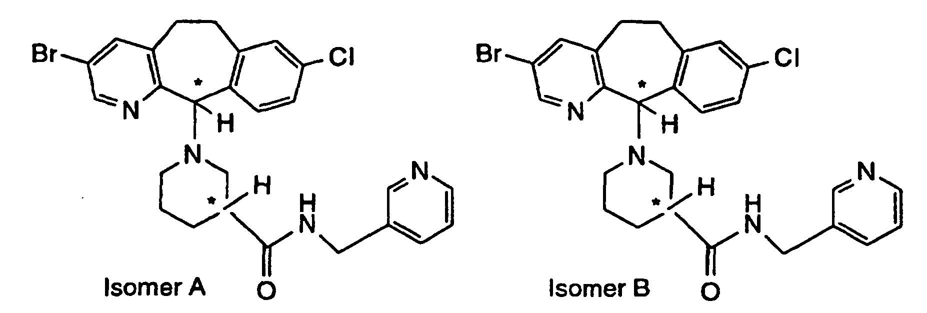

| | |
|---|---|
| FABMS(MH⁺)=526.8 | FABMS(MH⁺)=526.8 |
| FPT Inhibition = 6.1%@1.14µM | FPT IC₅₀ = 0.194µM |

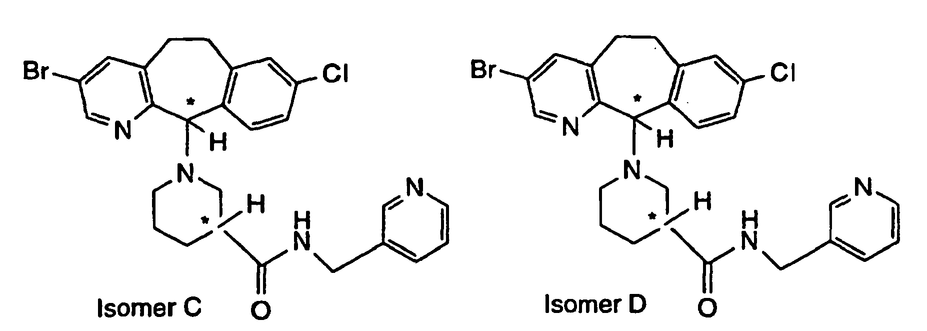

| | |
|---|---|
| FABMS(MH⁺)=526.8 | FABMS(MH⁺)=526.8 |
| FPT IC₅₀ = 0.179µM | FPT IC₅₀ = 0.187µM |

### Procedure 2. Step A.

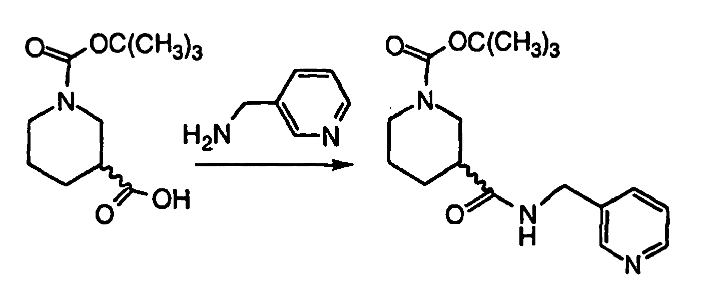

To a solution of N-(tert-butoxycarbonyl)nipecotic acid (0.50 g, 2.41 mmol) in dichloromethane (10 mL) is added 3-(aminomethyl)pyridine (0.27 mL, 2.65 mmol), 1-hydroxybenzotriazole monohydrate (HOBT) and 1,3-dicyclohexylcarbodiimide (0.547 g, 2.65 mmol). The mixture is stirred at room temperature for 16 hr and is then filtered. The solution is purified by flash chromatography (SiO₂, 2% methanol in CH₂Cl₂) affording 0.67g of the product.

### Step B.

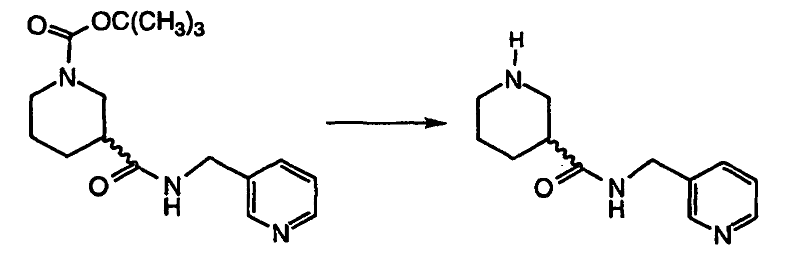

A solution of the product of Example 10, Procedure 2, Step A, (0.04g, 0.125 mmol) in CH₂Cl₂ (3 mL) is treated with trifluoroacetic acid (TFA) (0.5 mL) for 1 hr. The mixture is then evaporated to dryness in vacuo and azeotroped with methanol (3 x 5mL portions).

### Step C

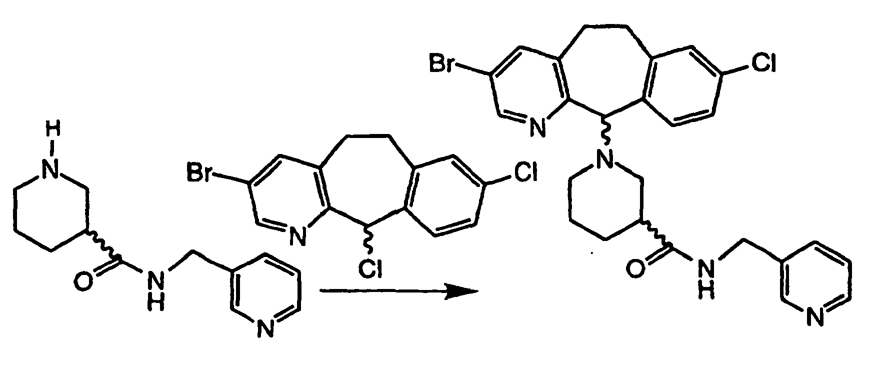

The residue from Example 10, Procedure 2, Step B above, is then dissolved in CH₃CN (1 mL) and a solution of 3-bromo-8,11-dichloro-6,11-dihydro-5H-benzo[5,6]cyclohepta[1,2-b]pyridine (0.07 g, 0.2 mmol) in CH₃CN is added, followed by 1,2,2,6,6-pentamethylpiperidine (0.2 mL, 1.1 mmol). The solution is heated at 45°C for 16 hr and then evaporated to dryness in vacuo. The residue is purified by flash chromatography (SiO₂, 3% methanol in CH₂Cl₂) affording 0.04g of the title compound.
1H NMR (300 Mhz) CDCl3; d=1.45-1.75 (m, 2H); 1.8-1.92 (m, 1H); 2.02-2.15 (m, 1 H); 2.16-2.30 (m, 1 H); 2.38-2.52 (m, 2H); 2.58-2.72 (m, 2H); 3.16-3.3 (m, 1H); 3.46-3.70 (m, 2H); 3.70-3.82 (m, 1 H); 4.32 (br. s, 2H); 4.39 (d, 1H); 4.45-4.52 (m, 1 H); 6.93 (br. s, 0.5 H); 7.06-7.17 (m, 3H); 7.22-7.28 (m, 1 H); 7.35 (br. s, 0.5H); 7.45-7.52 (m, 1H); 7.53 (d, 0.5H); 8.38 (d, 0.5H); 8.49 (br. s, 1H); 1.58 (m, 1H).

### EXAMPLE 11. 1-(3-bromo-8-chloro-6,11-dihydro-5H-benzo[5,6]cyclohepta [1,2-b]pyridin-11-yl)-N-(3-pyridinyl)-3-piperidinecarboxamide

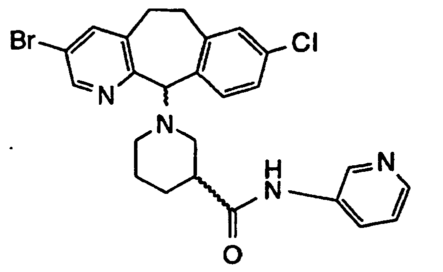

1-(3-Bromo-8-chloro-6,11-dihydro-5H-benzo[5,6]cyclohepta[1,2-b]pyridin-11-yl)-3-piperidinecarboxytate (0.12 gm, 0.27 mmol) from Example 10, Procedure 1, Step B, is dissolved in 4 ml of DMF. 1-(3-Dimethylaminopropyl)-3-ethyl carbodiimide hydrochloride (DEC), (79 mg, 0.41 mmol), 1-hydroxybenzotriazole (HOBT) (55 mg, 0.41 mmol), N-methyl morpholine (NMM) (0.29 ml, 2.7 mmol), and 3-amino pyridine (0.05 gm) are added and the reaction mixture is stirred for 18 hours. The reaction mixture is poured into water and extracted with ethyl acetate three times. The combined extracts are dried over magnesium sulfate, filtered and chromatographed on silica gel to obtain 42 mg of title compound. FABMS (MH⁺)=512.8
FPT IC₅₀ = 0.065 µM

### EXAMPLE 12. 1-(3-Bromo-8-chloro-6,11-dihydro-5H-benzo[5,6]cyclohepta[1,2-b]pyridin-11-yl)-N-(4-pyridinylmethyl)-2S-piperidinecarboxamide- Isomer A1 amide

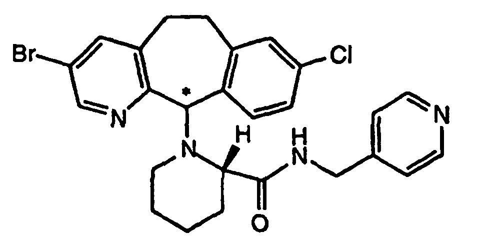

### Step A1. L-Pipecolinic Acid Ethyl Ester Hydrochloride.

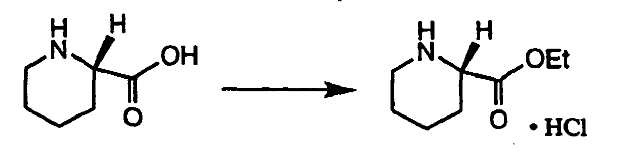

L-Pipecolinic acid (0.9g, 6.97 mmol) is dissolved in 40 mL of absolute EtOH. HCl gas is bubbled for -1 minute. The reaction mixture is refluxed for 20 min, cooled and the solvents removed by rotary evaporation to give 1.34g of the title compound, a wax that is used without further purification.

### Step A2. D-Pipecolinic Acid Ethyl Ester Hydrochloride.

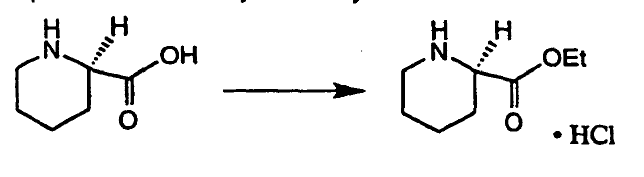

Using essentially the same conditions as described in Example 12, Step A1, but replacing L-Pipecolinic acid with D-Pipecolinic acid, the title compound is obtained.

### Step B.

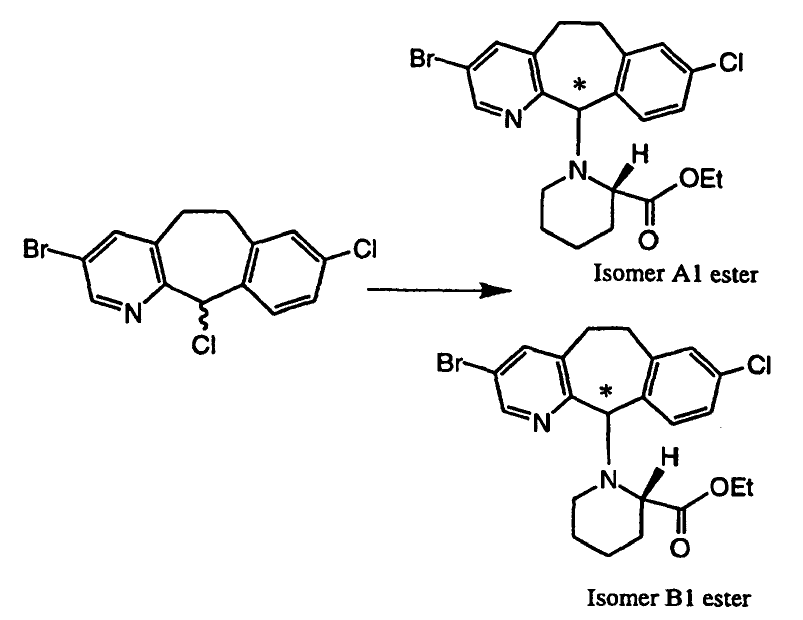

3-Bromo-8,11-dichloro-6,11-dihydro-5H-benzo[5,6]cyclohepta[1,2-b]pyridine (1.10 g, 3.00 mmol) and L-pipecolinic acid ethyl ester hydrochloride from Example 12, Step A1 (1.34 g, 6.98 mmol), triethylamine (2.91 µL, 21 mmol) are dissolved in dry CH₂Cl₂ (20ml) and the mixture is stirred at 25°C under nitrogen for 72h. The reaction mixture is washed with saturated NaHCO₃, H₂O, brine and then filtered through Na₂SO₄ and evaporated to dryness. The product is chromatographed on a silica gel column using 1% ethyl acetate-dichloromethane as the eluant to separate the two separable diastereomeric isomers (i.e. Isomers A1 ester and B1 ester), the less polar being referred to as Isomer A1 ester and the more polar isomer being referred to as Isomer B1 ester. FABMS MH⁺ = 464.

### Step C.

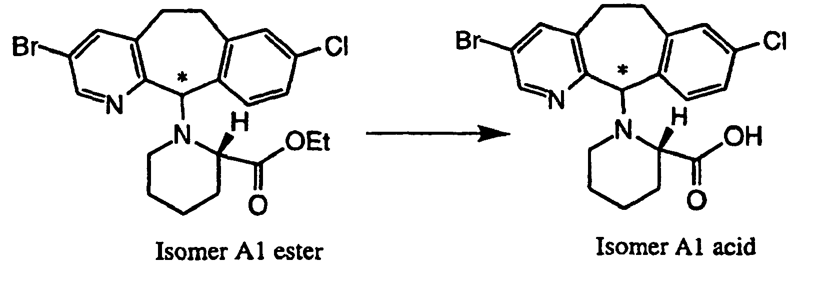

Isomer A1 ester from Example 12, Step B (0.26g, 0.6 mmol) is dissolved in 6 mL of ethanol and 1.4 mL of 1 M LiOH (1.4 mmol) is added. The reaction mixture is heated on oil bath at 80°C for 10h, cooled and 1.5 mL of 1N HCl is then added to adjust the pH to - 4.5. Solvents are then removed by evaporation and the resulting crude acid is used in the next reaction without further purification.

### Step D.

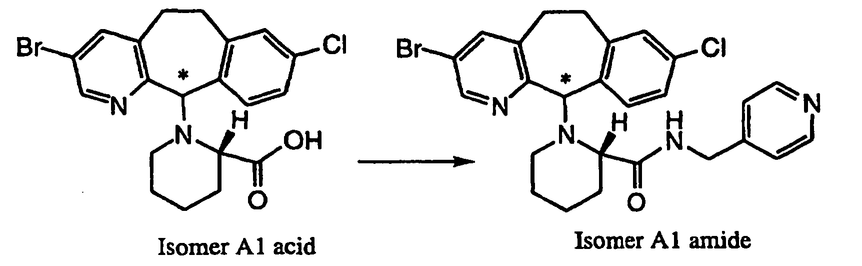

Isomer A1 acid from Example 12, Step C (from 0.26g, 0.6 mmol of Isomer A ester) is dissolved in 3 mL of DMF and NMM (184 µL, 1.6 mmol), 4-(aminomethyl)pyridine (74 µL, 0.078g, 0.73 mmol), HOBT(0.098 g, 0.72 mmol), DEC(0.139g, 0.72 mmol) are then added. The reaction mixture is stirred at room temperature for 16h. DMF is removed by rotary evaporation and the resulting crude mixture is partitioned between EtOAc-NaHCO₃. The organic phase is washed with H₂O, brine and filtered though Na₂SO₄ to give crude product that is purified by flash chromatography eluting with 3% (10% NH₄OH-CH₃OH)-CH₂Cl₂ solvent system to obtain the title compound, a white solid FAB-MS MH⁺ = 527
FPT Inhibition = 18% @ 1.1 µM

### Example 13. 1-(3-Bromo-8-chloro-6,11-dihydro-5H-benzo[5,6]cyclohepta[1,2-b]pyridin-11-yl)-N-(4-pyridinylmethyl)-2S-piperidinecarboxamide - Isomer B1 amide

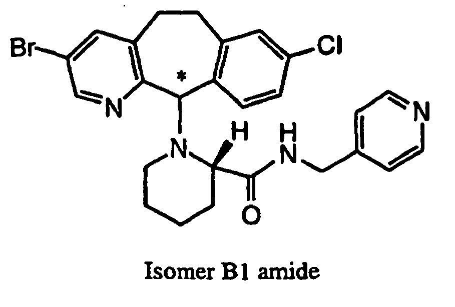

Using the method of Example 12, Steps C and D, except that Isomer B1 ester from Example 12, Step B, is used instead of Isomer A1 ester, the title compound is obtained. MH⁺ =527
FPT Inhibition = 21% @ 1.1 µM

### Example 14. Using the method of Examples 12 and 13, except that D-pipecolinic acid ethyl ester hydrochloride is used in place of L-pipecolinic acid ethyl ester hydrochloride, the following two diasteriomers are obtained:

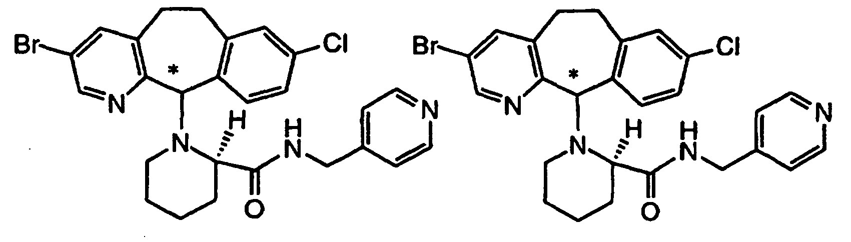

| Isomer A2 amide | Isomer B2 amide |
|---|---|
| MH⁺ = 527 | MH⁺ = 527 |
| FPT Inhibition = 0% @ 1.1 µM | FPT Inhibition = 13% @ 1.1 µM |

### Example 15. Using the method of Examples 12-14, except that in Example 12, Step D, 3-(aminomethyl)pyridine is used in place of 4-(aminomethyl)pyridine, the following four diasteriomers are prepared.

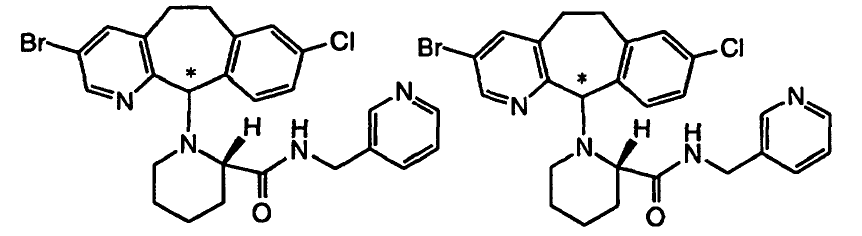

| Isomer C1 amide | Isomer D1 amide |
|---|---|
| MH⁺=527 | MH⁺ = 527 |
| melting point (m.p.) = 198.5-199°C | m.p. = 180.9-181.5°C |
| FPT IC₅₀ = 0.3 µM | FPT IC₅₀ = 0.16 µM |

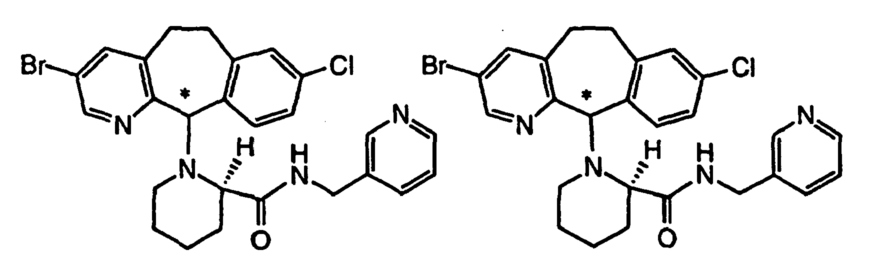

| Isomer C2 amide | Isomer D2 amide |
|---|---|
| MH⁺=527 | MH⁺ = 527 |
| m.p. = 168.2-168.4°C | m.p. = 205.5-206.4°C |
| FPT Inhibition =11% @ 0.38 µM | FPT Inhibition = 0% @ 0.38 µM |

### Example 16. 1-(3,10-Dibromo-8-chloro-6,11-dihydro-5H-benzo[5,6]cyclohepta[1,2-b]pyridin-11-yl)-N-(3-pyridinylmethyl)-3-piperidinecarboxamide

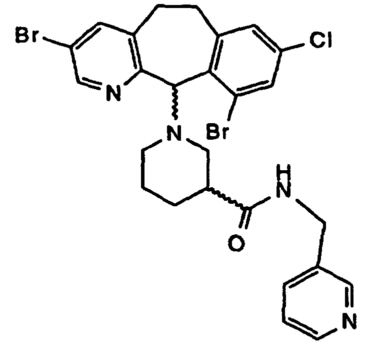

Using the method of Example 10, Procedure 1, except that the compound 3,10-dibromo-8,11-dichioro-6,11-dihydro-5H-benzo[5,6]cyclohepta[1,2-b]pyridine is substituted for 3-bromo-8,11-dichloro-6,11-dihydro-5H-benzo[5,6]cyclohepta[1,2-b]pyridine, the title compound is obtained. FABMS MH⁺=605.7
FPT IC₅₀ = 0.027µM

### EXAMPLE 17 1-(3,10-Dibromo-8-chloro-6,11-dihydro-5H-benzo[5,6]cyclohepta[1,2-b]pyridin-11-yl)-N-(3-pyridinylmethyl)-3-piperidinecarboxamide

Using the method of Example 10, Procedure 1, except that the compound 3,10-dibromo-8,11-dichloro-6,11-dihydro-5H-benzo[5,6]cyclohepta[1,2-b]pyridine is substituted for 3-bromo-8,11-dichloro-6,11-dihydro-5H-benzo[5,6]cyclohepta[1,2-b]pyridine and optically pure ethylnipecotate is used, the title compound is obtained. Optically pure ethylnipecotate can be prepared from L-tartaric acid according to (Recl. Trav. Chim. P. 899, 1951).

Separation of the resulting two isomers by HPLC chromatography is performed on a Chiral Technlogies AD analytical (0.46cmX25cm) chiral column using 10% isopropanol/hexanes/0.02% diethylamine at 1mL/minute. The two compounds are eluted at 14.85 (Isomer A) and 24.7 (Isomer B) minutes.

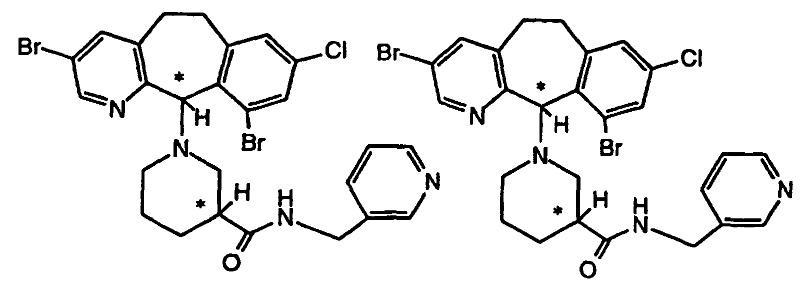

| Isomer A | Isomer B |
|---|---|
| MH⁺ = 605.7 | MH⁺ = 605.7 |
| FPT IC₅₀ < 0.0099 µM | FPT Inhibition = 3.4% at 0.1 µM |

### EXAMPLE 18. 1-(3-Bromo-8-chloro-6,11-dihydro-5H-benzo[5,6]cyclohepta[1,2-b]pyridin-11-yl)-N-(3-pyridinylmethyl)-3-piperidineacetamide

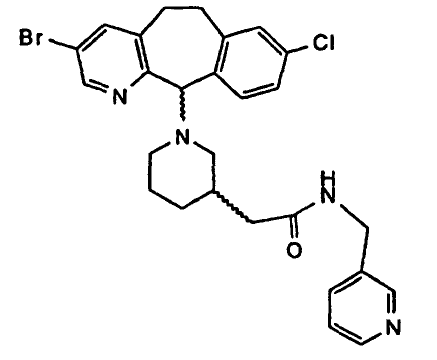

Using the method of Example 10, Procedure 1, except that 3-ethylpiperidineacetate is substituted for ethylnipecotate, the title compound is obtained. FABMS(MH⁺) = 541.0
FPT Inhibition = 9% @ 1.1 µM

### EXAMPLE 19. 1-(3-Bromo-8-chloro-6,11-dihydro-5H-benzo[5,6]cyclohepta[1,2-b]pyridin-11-yl)-N-(3-pyridinyl)-3-piperidineacetamide

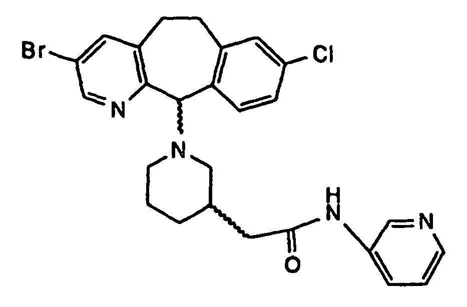

Using the method of Example 10, Procedure 1, except that 3-ethylpiperidineacetate is substituted for ethylnipecotate, and Example 11, except that nicotinic acid is substituted for 3-pyridylacetic acid, the title compound is obtained. FABMS(MH⁺)= 526.9
FPT Inhibition = 15% @ 1.1 µM

### EXAMPLE 20.

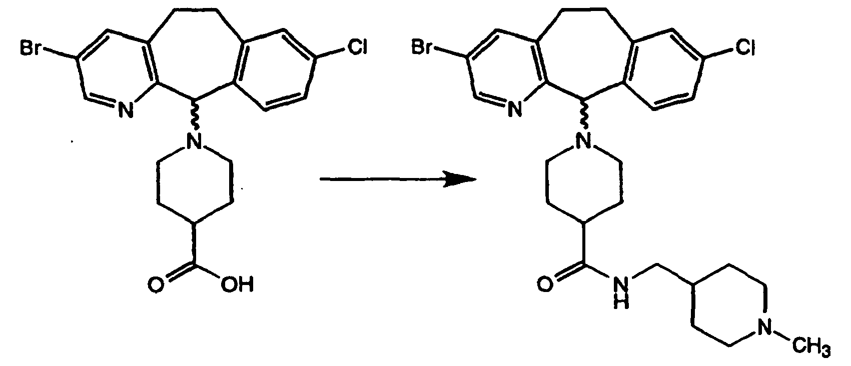

1-(3-Bromo-8-chloro-6,11-dihydro-5H-benzo[5,6]cyclohepta[1,2-b]pyridin-11-yl)-4-piperidinecarboxylate (1 equivalent) (prepared as described in Preparative Example 2) is reacted with 1-N-methyl-4-(aminomethyl)-piperidine (1.3 equivalents) (prepared by reductive formylation of 4-ethoxycarbonylaminomethylpyridine, followed by hydrolysis of the protecting group under standard conditions) under similar conditions to those described in Preparative Example 2, below, to give the title compound.

### EXAMPLE 21

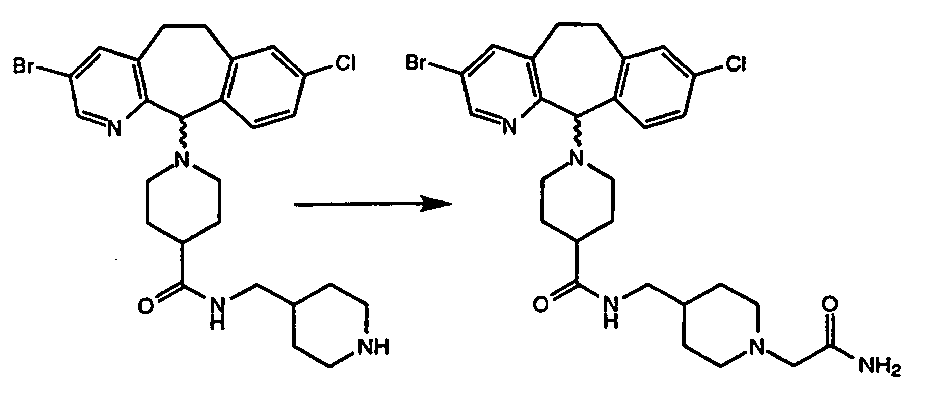

1-(3-Bromo-8-chloro-6,11-dihydro-5H-benzo[5,6]cyclohepta[1,2-b]pyridin-11-yl)-N-(4-piperidinylmethyl)-4-piperidinecarboxamide (1 equivalent) (prepared as described in Example 8, Procedures 1 or 2, Step B above) is reacted with 2-bromoacetamide (1.1 equivalents) and sodium carbonate in anhydrous DMF at 25°C to give the title compound.

### EXAMPLE 22

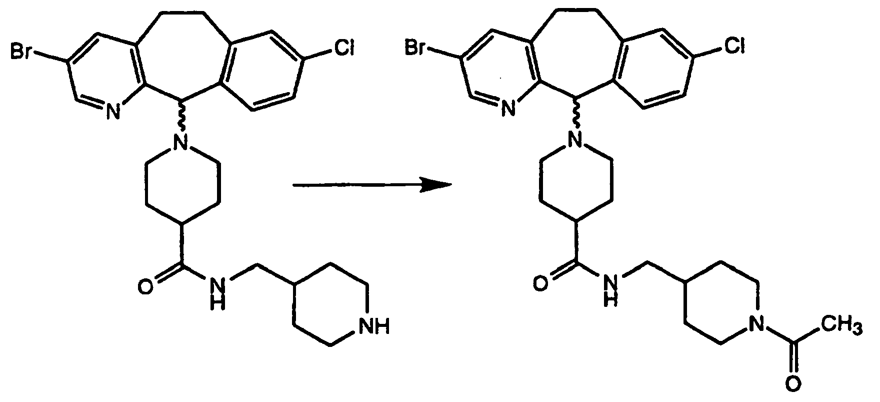

1-(3-Bromo-8-chloro-6,11-dihydro-5H-benzo[5,6]cyclohepta[1,2-b]pyridin-11-yl)-N-(4-piperidinylmethyl)-4-piperidinecarboxamide (1 equivalent) (prepared as described in Example 8, Procedures 1 or 2, Step B above) is reacted with an excess of acetic anhydride in methanol at 25°C for 24h to give the title compound.

### EXAMPLE 23

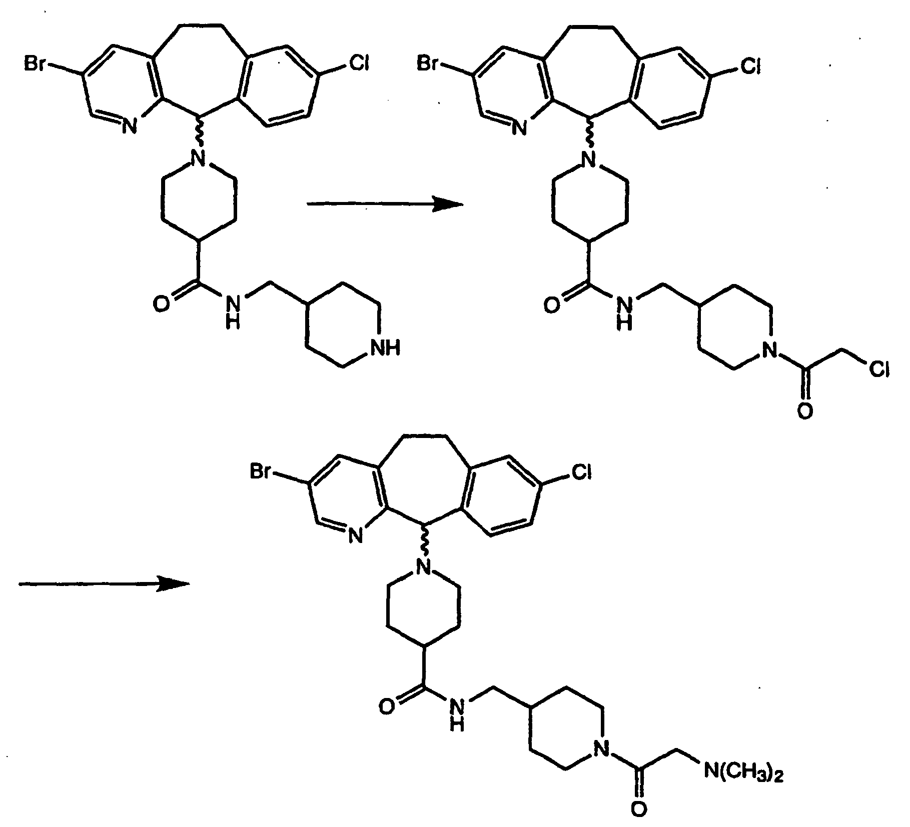

1-(3-Bromo-8-chloro-6,11-dihydro-5H-benzo[5,6]cyclohepta[1,2-b]pyridin-11-yl)-N-(4-piperidinylmethyl)-4-piperidinecarboxamide (1 equivalent) (prepared as described in Example 8, Procedures 1 or 2, Step B above) is reacted with chloroacetyl chloride (1.1 equivalents) and triethylamine (2 equivalents) in dichloromethane to give the intermediate chloroacetate. The latter is reacted with an excess of dimethylamine in the presence of sodium carbonate in DMF at 25°C to give the title compound.

### EXAMPLE 24

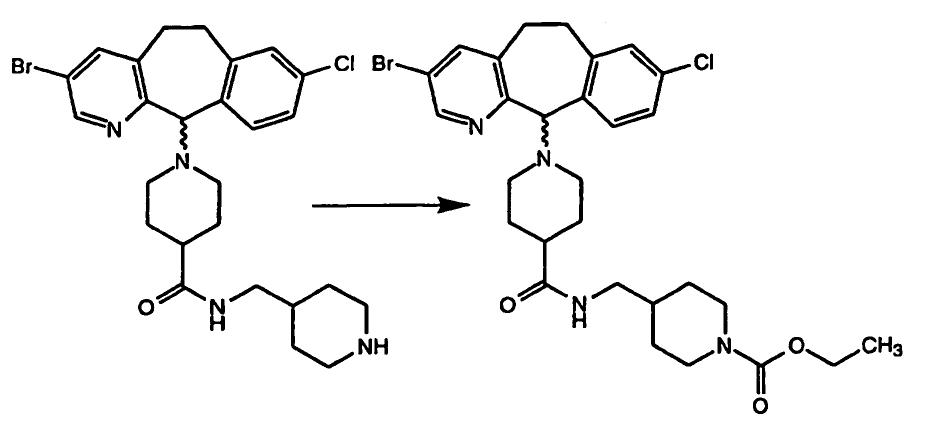

1-(3-Bromo-8-ch)oro-6,11-dihydro-5H-benzo[5,6]cyclohepta[1,2-b]pyridin-11-yl)-N-(4-piperidinylmethyl)-4-piperidinecarboxamide (1 equivalent) (prepared as described in Example 8, Procedures 1 or 2, Step B above) is reacted with ethylchloroformate (1.1 equivalents) in anhydrous dichloromethane at 25°C for 24h to give the title compound.

### EXAMPLE 25

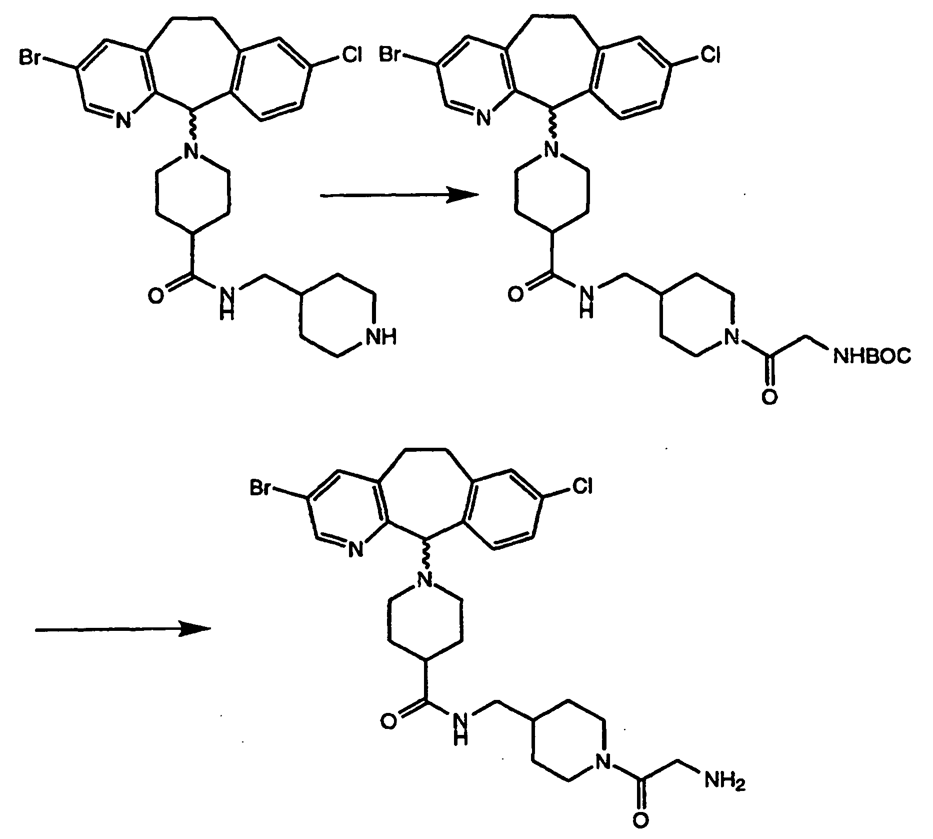

1-(3-Bromo-8-chloro-6,11-dihydro-5H-benzo[5,6]cyclohepta[1,2-b]pyridin-11-yl)-N-(4-piperidinylmethyl)-4-piperidinecarboxamide (1 equivalent) (prepared as described in Example 8, Procedures 1 or 2, Step B above) is reacted with N-(*tert*-butoxycarbonyl)glycine (1.3 equivalents), DEC.HCl (1.3 equivalents), HOBT (1.3 equivalents) and N-methylmorpholine (1.3 equivalents) in anhydrous DMF at 25°C for 24h to give the N-BOC intermediate. The latter is dissolved in methanol and reacted with 10% concentrated sulfuric acid in dioxane at 25°C for 2h to give after basification and chromatography on silica gel, the title compound.

### EXAMPLE 26

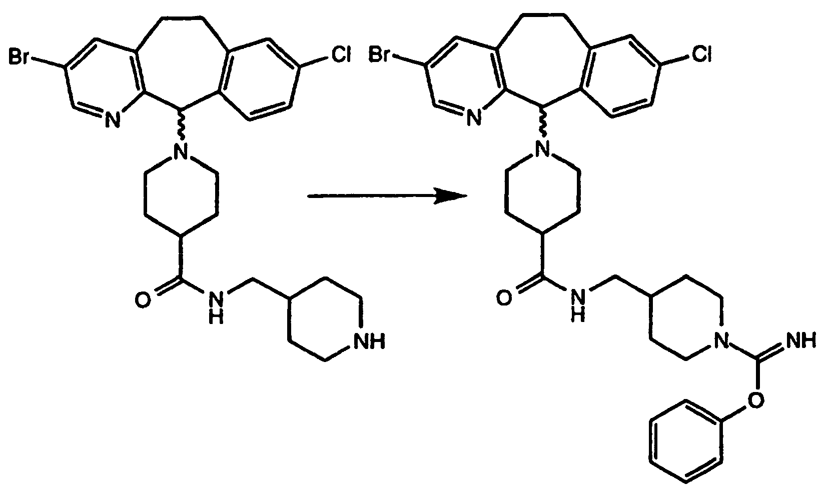

1-(3-Bromo-8-chloro-6,11-dihydro-5H-benzo[5,6]cyclohepta[1,2-b]pyridin-11-yl)-N-(4-piperidinylmethyl)-4-piperidinecarboxamide (1 equivalent) (prepared as described in Example 8, Procedures 1 or 2, Step B above) in dichloromethane is reacted with phenyl cyanate (2 equivalents) and diisopropylethylamine at 25°C for 15 minutes to give the title compound.

### EXAMPLE 27

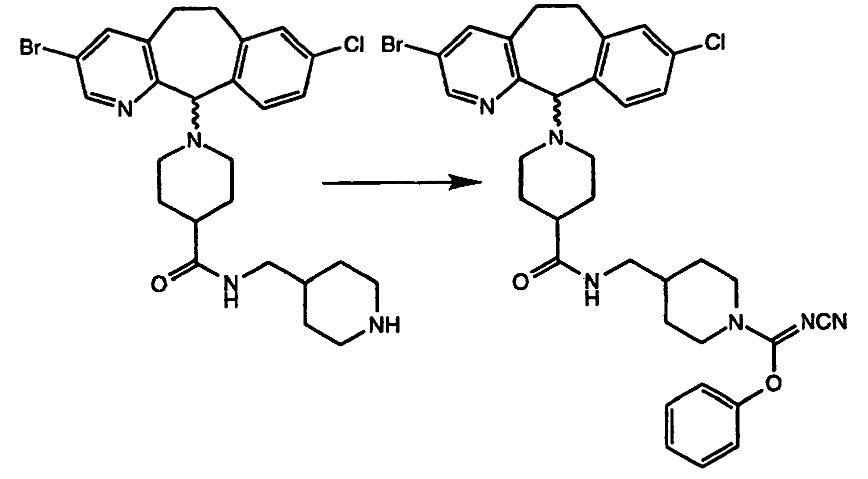

1-(3-Bromo-8-chloro-6,11-dihydro-5H-benzo[5,6]cyclohepta[1,2-b]pyridin-11-yl)-N-(4-piperidinylmethyl)-4-piperidinecarboxamide (1 equivalent) (prepared as described in Example 8, Procedures 1 or 2, Step B above) and diphenylcyanocarbonimidate (1.2 equivalents) are dissolved in 2-propanol and the mixture is heated at 80°C for 24h to give the title compound.

### EXAMPLE 28

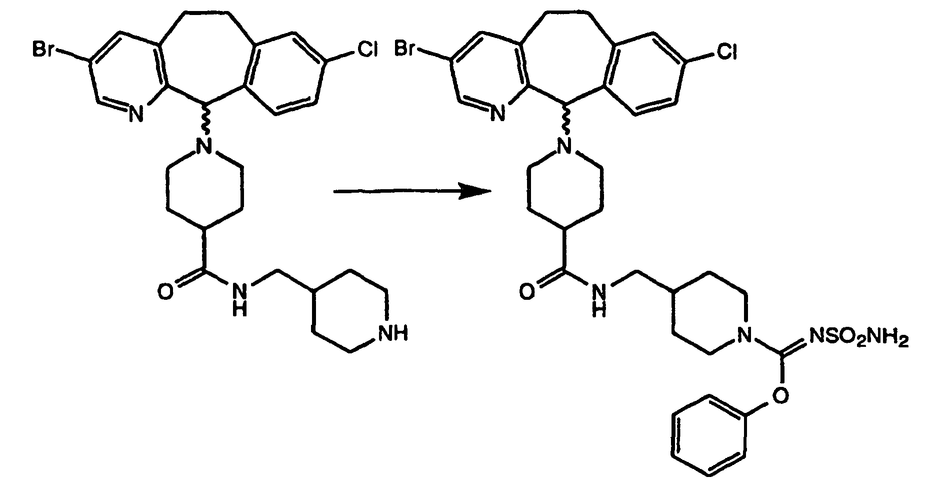

1-(3-Bromo-8-chloro-6,11-dihydro-5H-benzo[5,6]cyciohepta[1,2-b]pyridin-11-yl)-N-(4-piperidinylmethyl)-4-piperidinecarboxamide (1 equivalent) (prepared as described in Example 8, Procedures 1 or 2, Step B above) and diphenylsulfamoylcarbonimidate (1.2 equivalents) [prepared as described in: M. Haake and B. Schummelfeder, Synthesis, 753-758 (1991)] are dissolved in 2-propanol and the mixture is heated at 80°C for 24h to give the title compound.

### EXAMPLE 29

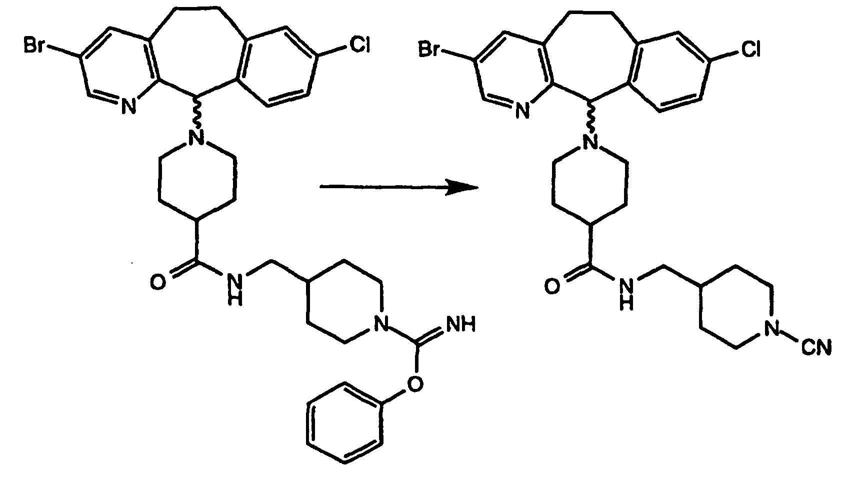

The phenoxyimidate (1 equivalent) (prepared as described in Example 26 above) is dissolved in anhydrous THF. A 60% sodium hydride dispersion in oil 4 equivalents) is added and the mixture is stirred at 25°C for 2h. The mixture is diluted with dichloromethane and washed with 1N sodium hydroxide. Chromatography on silica gel affords the title compound.

### EXAMPLE 30

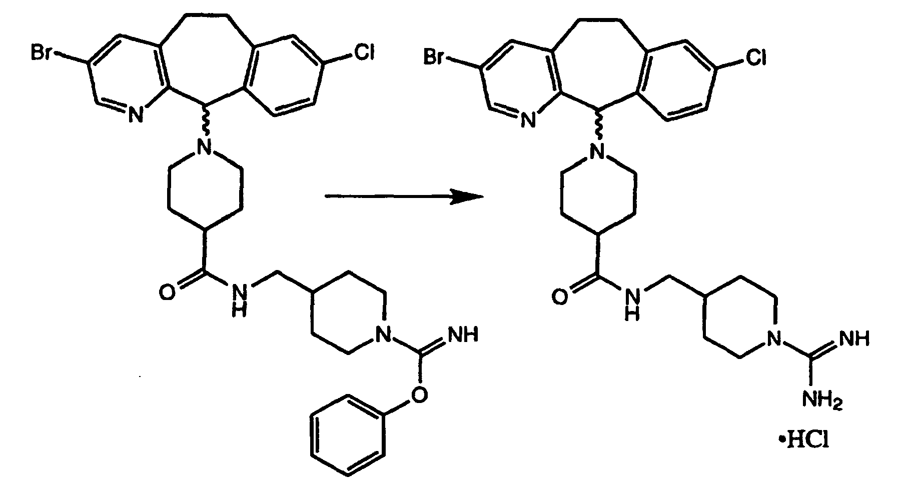

The phenoxyimidate (1 equivalent) (prepared as described in Example 26 above) is dissolved in concentrated ammonium hydroxide and ammonium chloride (1 equivalent) is added. The mixture is heated in a sealed tube at 90°C to give the title compound.

### EXAMPLE 31

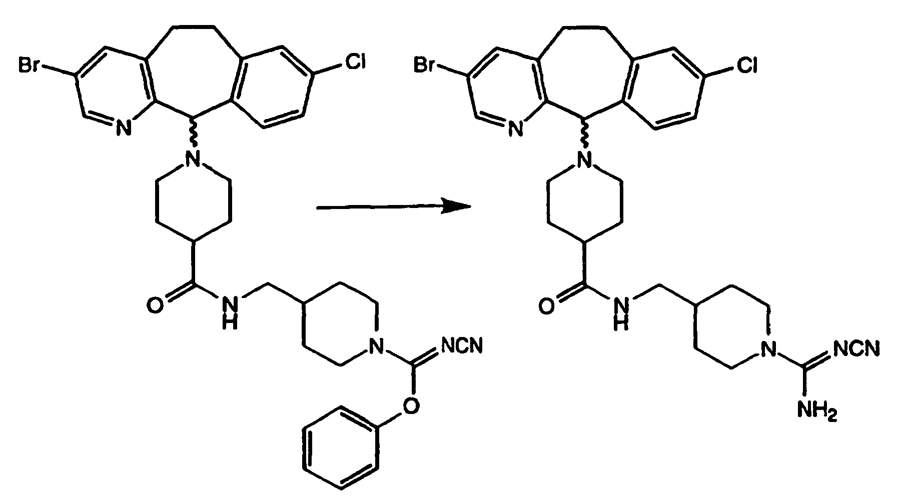

The N-cyanophenoxyimidate (1 equivalent) (prepared as described in Example 27 above) is dissolved in concentrated ammonium hydroxide and the mixture is stirred at 25°C for 24h to give the title compound.

### EXAMPLE 32

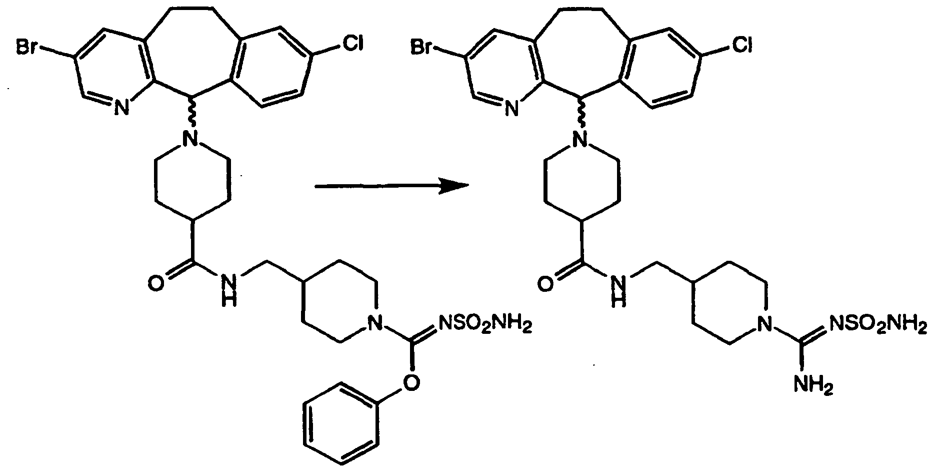

The N-sulfamoylphenoxyimidate (1 equivalent) (prepared as described in Example 28 above) is dissolved in concentrated ammonium hydroxide and the mixture is stirred at 25°C for 24h to give the title compound.

### EXAMPLE 33

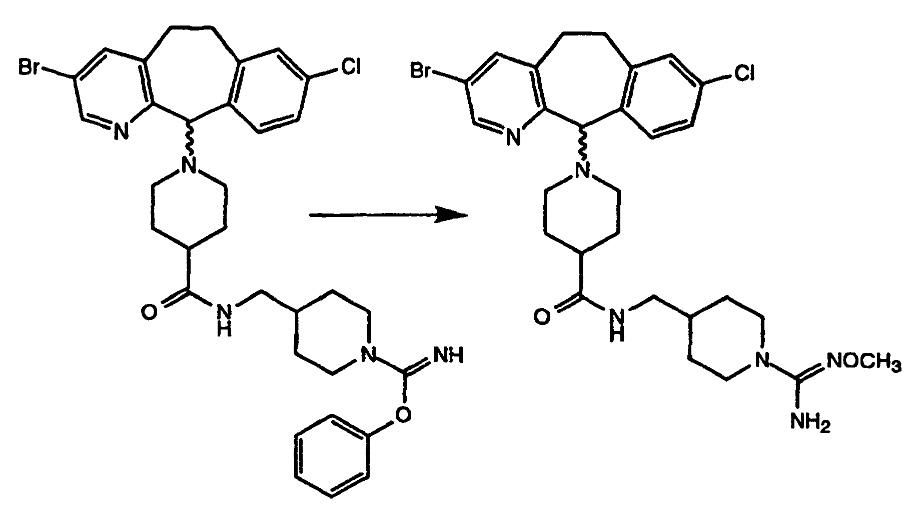

The phenoxyimidate (1 equivalent) (prepared as described in Example 26 above) is dissolved in methanol. An aqueous solution of methoxylamine (1 equivalent) [prepared by dissolving methoxylamine hydrochloride (1 equivalent) in 50% (w/v) sodium hydroxide (1 equivalent)] is added and the mixture is stirred at 25°C to give the title compound.

### EXAMPLE 34

Using the method of Example 14 except that 3,10-dibromo-8,11-dichloro-6,11-dihydro-5H-benzo[5,6]cyclohepta[1,2-b]pyridine is substituted for 3-bromo-8,11-dichloro-6,11-dihydro-5H benzo[5,6]cyclohepta[1,2-b]pyridine, the following two compounds are obtained:

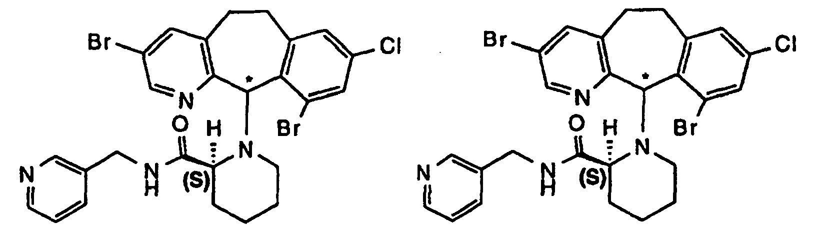

| Isomer C3 amide | Isomer D3 amide |
|---|---|
| MH⁺ = 605 | MH⁺ = 605 |
| FPT IC₅₀ = 0.3 µM | FPT IC₅₀ = 0.0.0042 µM |

### EXAMPLE 35

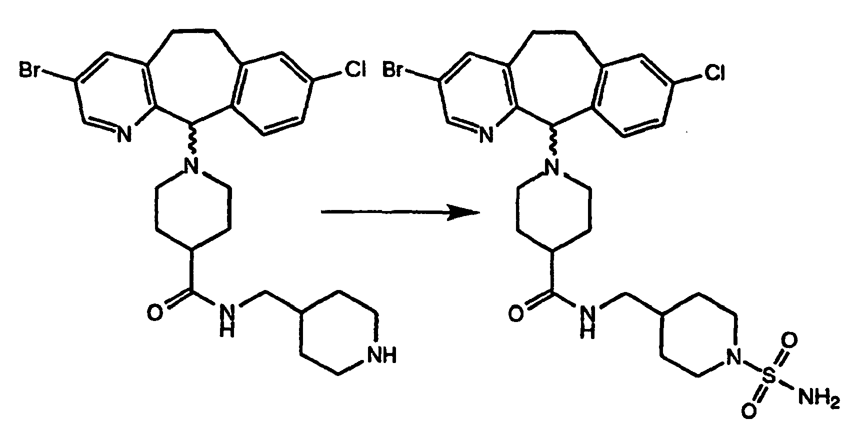

1-(3-Bromo-8-chloro-6,11-dihydro-5H-benzo[5,6]cyctohepta[1,2-b]pyridin-11-yl)-N-(4-piperidinylmethyl)-4-piperidinecarboxamide (1 equivalent) (prepared as described in Example 8, Procedures 1 or 2, Step B above) and sulfamide (10 equivalents) are added to water and the mixture is stirred under reflux at 100°C for 43h to give the title compound.

### EXAMPLE 36

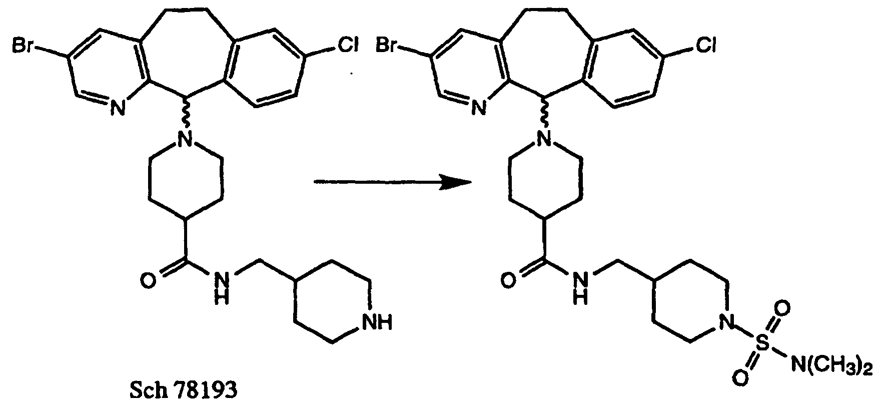

1-(3-Bromo-8-chloro-6,11-dihydro-5H-benzo[5,6]cyclohepta[1,2-b] pyridin-11-yl)-N-(4-piperidinylmethyl)-4-piperidinecarboxamide (1 equivalent) (prepared as described in Example 8, Procedures 1 or 2, Step B above) in dichloromethane is reacted with dimethylsulfamoyl chloride (1.1 equivalents) in the presence of triethylamine (2 equivalents) at from 0°C to 25°C to give the title compound.

### EXAMPLE 37

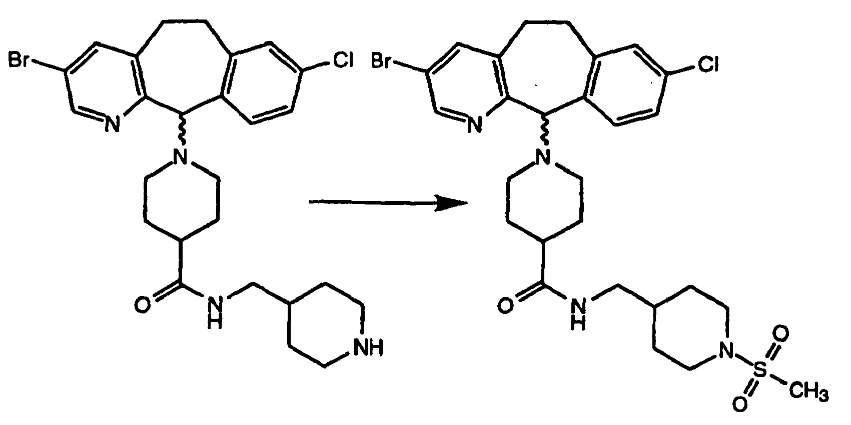

1-(3-Bromo-8-chloro-6,11-dihydro-5H-benzo[5,6]cyclohepta[1,2-b]pyridin-11-yl)-N-(4-piperidinylmethyl)-4-piperidinecarboxamide (1 equivalent) (prepared as described in Example 8, Procedures 1 or 2, Step B above) in dichloromethane is reacted with methanesulfonyl chloride (1.1 equivalents) in the presence of triethylamine (2 equivalents) at 25°C to give the title compound.

### EXAMPLE 38

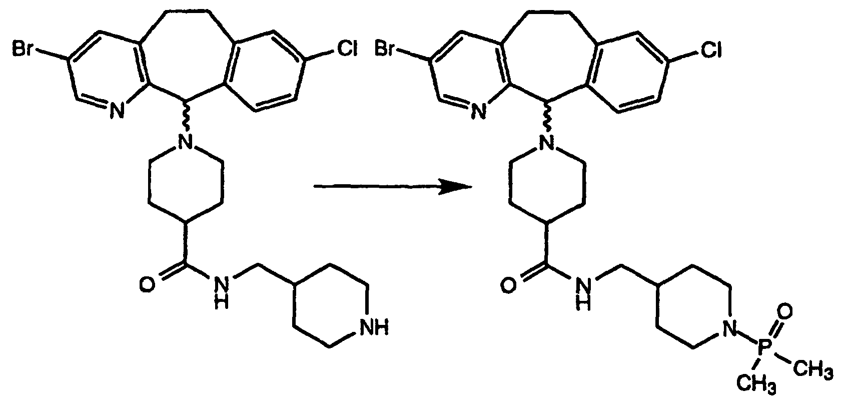

1-(3-Bromo-8-chloro-6,11-dihydro-5H-benzo[5,6]cyclohepta[1,2-b]pyridin-11-yl)-N-(4-piperidinylmethyl)-4-piperidinecarboxamide (1 equivalent) (prepared as described in Example 8, Procedures 1 or 2, Step B above) in DMF is reacted with dimethylphosphinic chloride (1.1 equivalents) and sodium carbonate at 25°C to give the title compound.

### EXAMPLE 39

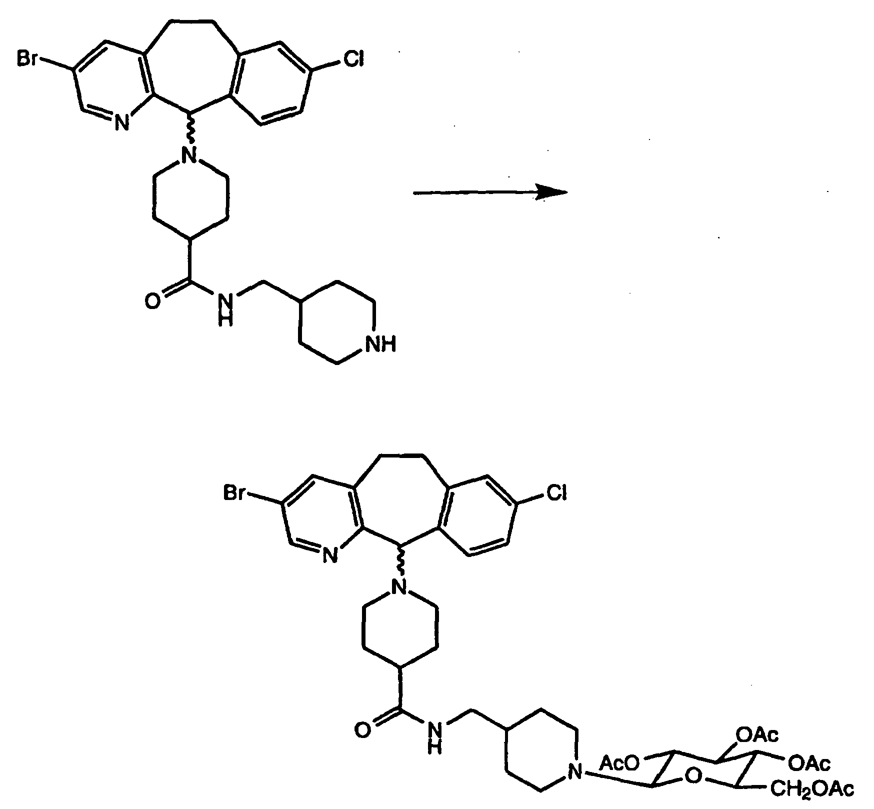

1-(3-Bromo-8-chloro-6,11-dihydro-5H-benzo[5,6]cyclohepta[1,2-b]pyridin-11-yl)-N-(4-piperidinylmethyl)-4-piperidinecarboxamide (1 equivalent) (prepared as described in Example 8, Procedures 1 or 2, Step B above) in DMF is reacted with tetra-O-acetyl-D-glucopyranosyl bromide (1.1 equivalents) in the presence of sodium carbonate to give the title compound.

### EXAMPLE 40

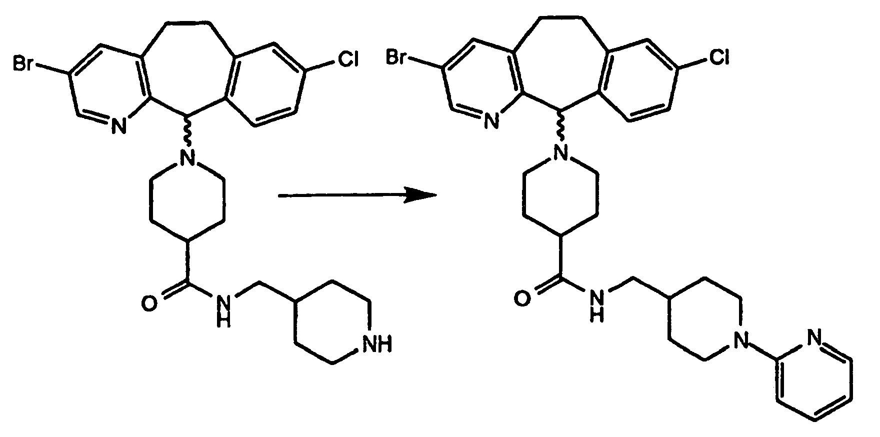

1-(3-Bromo-8-chloro-6,11-dihydro-5H-benzo[5,6]cyclohepta[1,2-b]pyridin-11-yl)-N-(4-piperidinylmethyl)-4-piperidinecarboxamide (1 equivalent) (prepared as described in Example 8, Procedures 1 or 2, Step B above) in DMF is reacted with 2-chtoropyridine (1.1 equivalents) in the presence of sodium carbonate to give the title compound.

### EXAMPLE 41

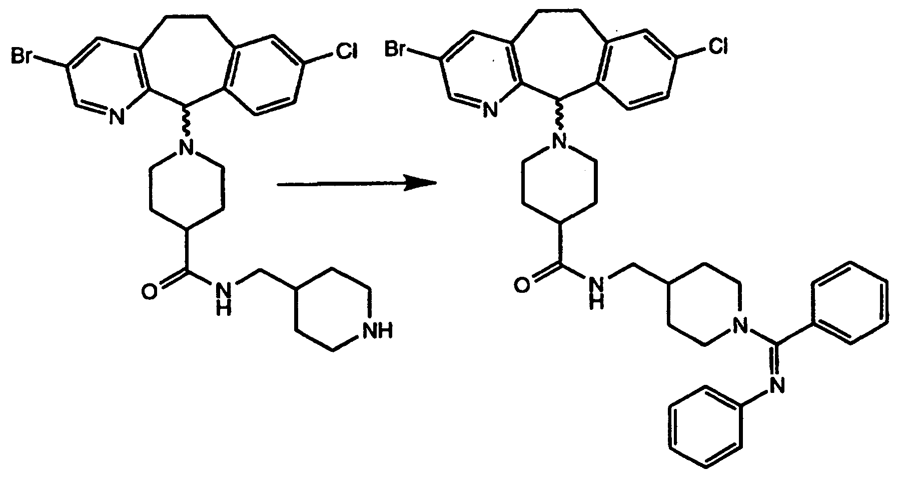

Benzanilide is converted into the choloroimidate (as described in: A. C. Honz and E. C. Wagner, Org. Syn. Coll. Vol. 4, 383-386 (1963) (1.1 equivalents) and this is reacted with 1-(3-Bromo-8-chloro-6,11-dihydro-5H-benzo[5,6]cyctohepta[1,2-b]pyridin-11-yl)-N-(4-piperidinylmethyl)-4-piperidine-carboxamide (1 equivalent) (prepared as described in Example 8, Procedures 1 or 2, Step B above) in pyridine at reflux temperature to give the title compound.

### Example 42

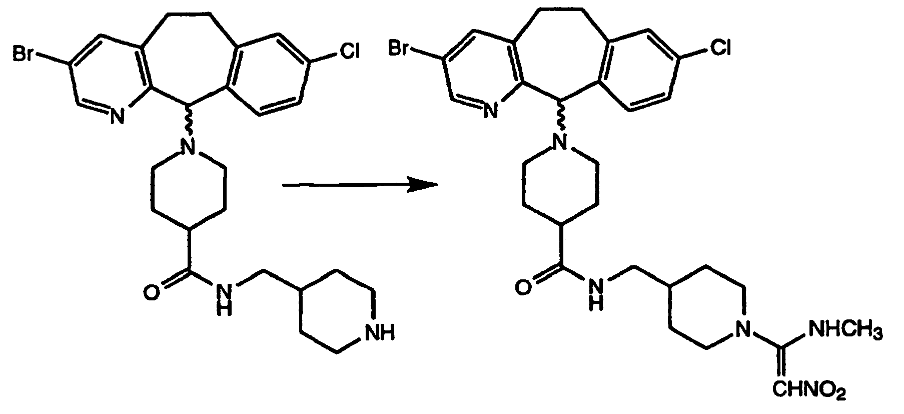

Copper(I)chloride (1 equivalent) is dissolved in anhydrous acetonitrile. To this solution, a solution of 1-(3-Bromo-8-chloro-6,11-dihydro-5H-benzo[5,6]cyclohepta[1,2-b]pyridin-11-yl)-N-(4-piperidinylmethyl)-4-piperidine-carboxamide (1 equivalent) (prepared as described in Example 8, Procedures 1 or 2, Step B above), 1-methylthio-1-methylamino-2-nitroethene (1 equivalent) (prepared as described in Canadian Patent 1,178,289 (1984)) and triethylamine in anhydrous acetonitrile is added dropwise over 10 minutes with stirring. The solid is filtered off The volume is reduced and dichloromethane is added. The mixture is washed with aqueous sodium bicarbonate and the dichloromethane layer is dried over magnesium sulfate, filtered and evaporated to dryness. The residue is purified on silica gel to give the title compound.

### Example 43. 1-(3-Bromo-8-chloro-6,11-dihydro-5H-benzo[5,6]cyclohepta[1,2-b]pyridin-11-yl)-N-(3-pyridylmethyl)-4-piperidineacetamide

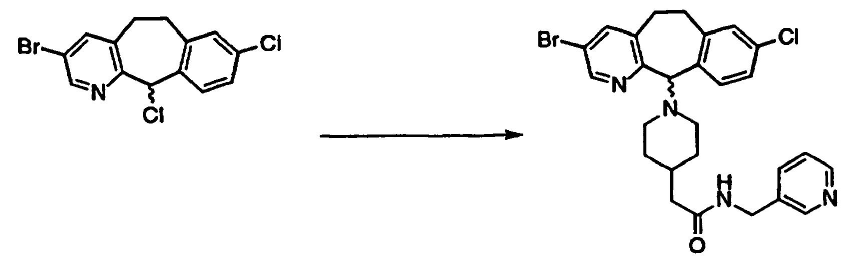

3-Bromo-8,11-dichloro-6,11-dihydro-5H-benzo[5,6]cyclohepta[1,2-b]pyridine (0.317g, 0.924mmoles) is dissolved in anhydrous THF (4.6ml). N-(3-pyridylmethyl)-4-piperidineacetamide (prepared as described in Preparative Example 7, Step B) (0.2803g, 1.2mmoles) and triethylamine (0.386ml, 2.77mmoles) in anhydrous dichloromethane (5ml) are added and the mixture is stirred at 25°C for 18h. The solution is diluted with dichloromethane and washed with 1N NaOH, dried over magnesium sulfate, filtered and evaporated to dryness. The product is chromatographed on silica gel using 4% (10% conc. ammonium hydroxide in methanol)-dichloromethane as the eluant to give the title compound (0.219g, 44%), SIMS: m/z 539 (MH⁺), FPT INH 43% @ 0.22µM.

| | | d_{c} (CDCl₃) |
|---|---|---|
| Tricyclic: | CH₂: | 30.3, 30.5 |
| | CH: | 79.5, 126.1, 130.5, 132.4, 141.1, 146.9 |
| | C: | 120.2, 126.4, 134.1, 135.5, 136.4, 143.5, 154.0 |
| Piperidine: | CH₂: | 41.0, 46.8, 47.0, 50.9, 50.9 |
| | CH: | 33.5 |
| | C: | 172.0 |
| Pyridine substituent: | CH₂: | 43.6 |
| | CH: | 123.7, 135.7, 148.9, 149.1 |
| | C: | 133.8 |

### Example 44. 1-(3-Bromo-8-chloro-6,11-dihydro-5H-benzo[5,6]cyclohepta[1,2-b]pyridin-11-yl)-N-(3-pyridylmethyl)-4-piperidinepropanamide

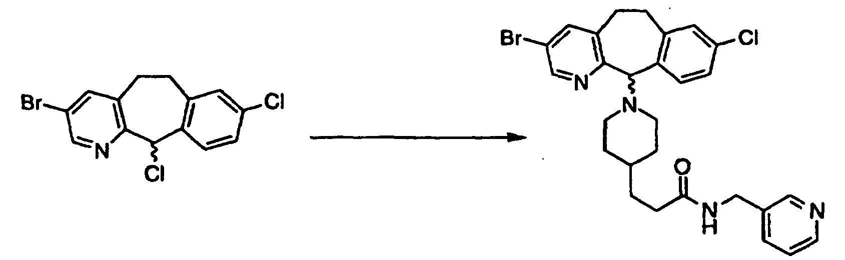

3-Bromo-8,11-dichtoro-6,11-dihydro-5H-benzo[5,6]cydohepta[1,2-b]pyridine (0.317g, 0.924mmoles) is dissolved in anhydrous THF (5ml). N-(3-pyridylmethyl)-4-piperidinepropanamide (prepared as described in Preparative Example 8, Step C) (0.2972g, 1.2mmoles) and triethylamine (0.386ml, 2.77mmoles) in anhydrous dichloromethane (20m1) are added and the mixture is stirred at 25°C for 20h. The solution is diluted with dichloromethane and washed with 1N NaOH , dried over magnesium sulfate, filtered and evaporated to dryness. The product is chromatographed on silica gel using 2.5% (10% conc. ammonium hydroxide in methanol)-dichloromethane as the eluant to give the title compound (0.3022g, 59%), ESIMS: m/z 553.2 (MH⁺), FPT INH 39% @ 0.35µM.

| δ_{c} (CDCl₃) | | |
|---|---|---|
| Tricyclic: | CH₂: | 30.3, 30.5 |
| | CH: | 79.6, 126.0, 130.5, 132.3, 141.1, 146.8 |
| | C: | 119.6, 133.7, 136.6, 136.6, 140.6, 157.1 |
| Piperidine: | CH₂: | 32.1, 32.4, 32.4, 34.0, 52.0, 52.3 |
| | CH: | 35.6 |
| | C: | 173.2 |
| Pyridine substituent: | CH₂: | 41.0 |
| | CH: | 123.6, 136.6, 148.9, 149.2 |
| | C: | 134.2 |

### Example 45. 1-(8-Chloro-3,10-dibromo-6,11-dihydro-5H-benzo[5,6]cycloheptal[1,2-b]pyridin-11-yl)-N-(3-pyridylmethyl)-4-piperidineacetamide

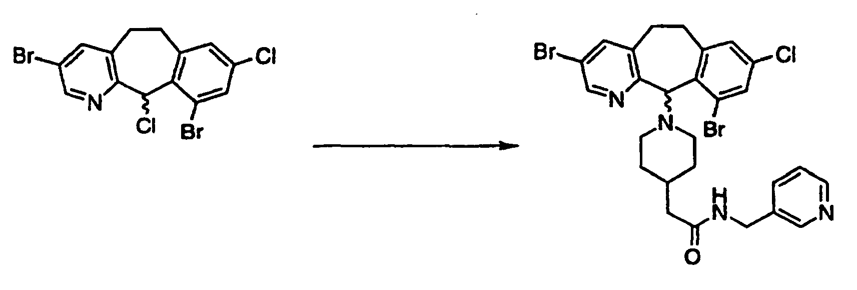

3,10-Dibromo-8,11-dichioro-6,11-dihydro-5H-benzo[5,6]cyclohepta[1,2-b]pyridine (prepared as described in Preparative Example 6, Step F) (0.2426g, 0.575mmoles) is dissolved in anhydrous THF (2.86ml). N-(3-pyridylmethyl)-4-piperidineacetamide (prepared as described in Preparative Example 7, Step B) (0.175g, 0.748mmoles) and triethylamine (0.24ml. 1.725mmoles) in anhydrous THF (5ml) are added and the mixture is stirred at 25°C for 138h. The solution is evaporated to dryness and the residue is dissolved in dichloromethane and washed with 1N NaOH, dried over magnesium sulfate, filtered and evaporated to dryness. The product is chromatographed on silica gel using 5% (10% conc. ammonium hydroxide in methanol)-dichloromethane as the eluant to give the title compound (0.021 g, 6%), ESIMS: m/z 617.2 (MH⁺), FPT IC₅₀ =0.042µM.

| δ_{c} (CDCl₃) | | |
|---|---|---|
| Tricyclic: | CH₂: | 32.4, 32.4 |
| | CH: | 75.6, 129.6, 130.7, 141.6, 147.2 |
| | C: | 119.9, 126.2, 133.8, 136.2, 136.2, 143.2, 155.0 |
| Piperidine: | CH₂: | 29.9, 31.2, 41.0, 51.0, 51.5 |
| | CH: | 33.4 |
| | C: | 171.9 |
| Pyridine substituent: | CH₂: | 43.6 |
| | CH: | 123.7, 135.7, 148.9, 149.1 |
| | C: | 134.1 |

### Example 46. 4-Carboxamido-1-[1-(8-chloro-3,10-dibromo-6,11-dihydro-5H-benzo[5,6]cyclohepta[1,2-b]pyridin-11-yl)-4-piperidineacetyl]piperidine

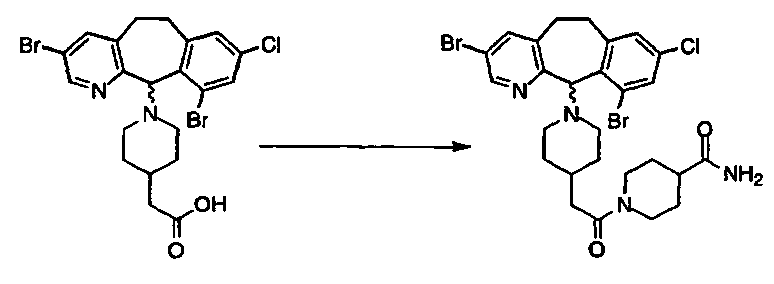

1-(8-Chloro-3,10-dibromo-6,11-dihydro-5H-benzo[5,6]cyclohepta[1,2-b]pyridin-11-yl)-4-piperidineacetic acid (0.5287g, 1mmole) (prepared as described in Preparative Example 10, Step B), isonipecotamide (0.1666g, 1.3mmoles), DEC.HCl (0.2492g, 1.3mmoles), HOBT (0.1757g, 1.3mmoles) and NMM (0.1315g, 1.3mmoles) are dissolved in anhydrous DMF (10ml) and the mixture is stirred at 25°C under argon for 24h. The solution is evaporated to dryness and the residue is dissolved in dichloromethane, washed with 1N NaOH, dried over magnesium sulfate, filtered and evaporated to dryness. The product is chromatographed on silica gel using 0.75% (10% conc. ammonium hydroxide in methanol)-dichloromethane as the eluant to give the title compound.

### Example 47. 3-Carboxamido-1-[1-(8-chloro-3,10-dibromo-6,11-dihydro-5H-benzo[5,6]cyclohepta[1,2-b]pyridin-11-yl)-4-piperidineacetyl]piperidine

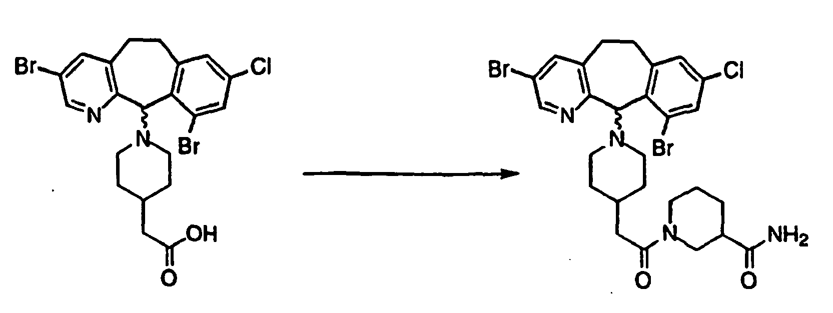

1-(8-Chloro-3,10-dibromo-6,11-dihydro-5H-benzo[5,6]cyclohepta[1,2-b]pyridin-11-yl)-4-piperidineacetic acid (0.5287g, 1mmole) (prepared as described in Preparative Example 10, Step B) and nipecotamide (0.1666g, 1.3mmoles), DEC.HCl (0.2492g, 1.3mmoles), HOBT (0.1757g, 1.3mmoles) and NMM (0.1315g, 1.3mmoles) are dissolved in anhydrous DMF (10ml) and the mixture is stirred at 25°C under argon for 24h. The solution is evaporated to dryness and the residue is dissolved in dichloromethane, washed with 1N NaOH, dried over magnesium sulfate, filtered and evaporated to dryness. The product is chromatographed on silica gel using 0.75% (10% conc. ammonium hydroxide in methanol)-dichloromethane as the eluant to give the title compound.

### Example 48. 4-[1-(8-Chloro-3,10-dibromo-6,11-dihydro-5H-benzo[5,6]cyclohepta[1,2-b]pyridin-11-yl)-4-piperidineacetyl]-1-piperazinecarboxamide

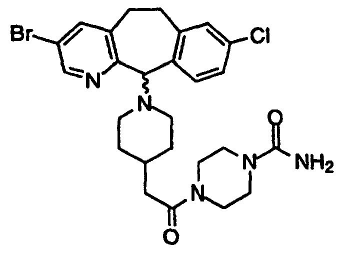

### Step A. 4-[1-(8-Chloro-3,10-dibromo-6,11-dihydro-5H-benzo[5,6]cyclohepta[1,2-b]pyridin-11-yl)-4-piperidineacetyl]-1-N-tert -butoxycarbonylpiperazine

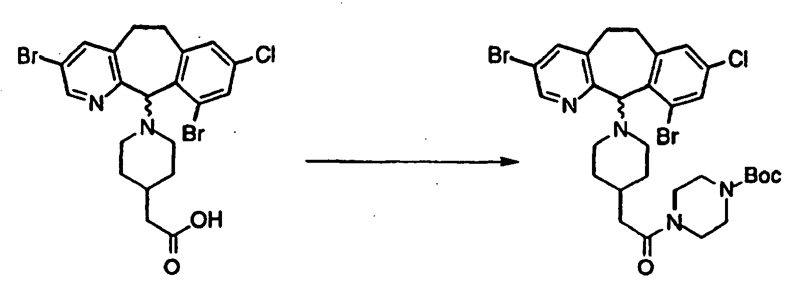

1-(8-Chloro-3,10-dibromo-6,11-dihydro-5H-benzo[5,6]cyclohepta[1,2-b]pyridin-11-yl)-4-piperidineacetic acid (0.5287g, 1 mmole) (prepared as described in Preparative Example 10, Step B) and 1-N-*tert-*butoxycarbonylpiperazine (0.1667g, 1.3mmoles), DEC.HCl (0.2492g, 1.3mmoles), HOBT (0.1757g, 1.3mmoles) and NMM (0.1315g, 1.3mmoles) are dissolved in anhydrous DMF (10ml) and the mixture is stirred at 25°C under argon for 24h. The solution is evaporated to dryness and the residue is dissolved in dichloromethane, washed with 1N NaOH, dried over magnesium sulfate, filtered and evaporated to dryness. The product is chromatographed on silica gel using 0.75% (10% conc. ammonium hydroxide in methanol)-dichloromethane as the eluant to give the title compound.

### Step B. 1-[1-(8-Chloro-3,10-dibromo-6,11-dihydro-5H-benzo[5,6]cyclohepta[1,2-b]pyridin-11-yl)-4-piperidineacetyl]piperazine

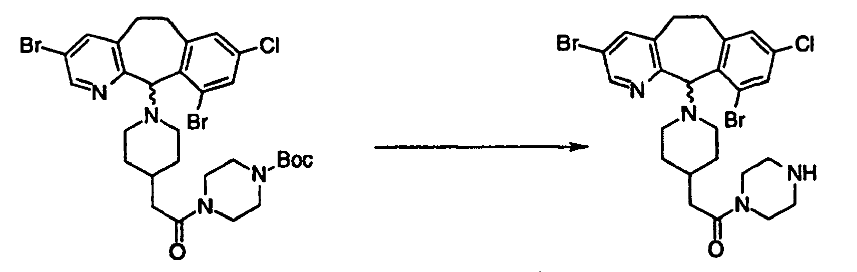

4-[1-(8-Chloro-3,10-dibromo-6,11-dihydro-5H-benzo[5,6]cyclohepta[1,2-b]pyridin-11-yl)-4-piperidineacetyl]-1-N-*tert*-butoxycarbonylpiperazine (prepared as described in Step A above) is converted into 1-[1-(8-Chloro-3,10-dibromo-6,11-dihydro-5H-benzo[5,6]cyclohepta[1,2-b]pyridin-11-yl)-4-piperidineacetyl]piperazine by essentially the same procedure as described in Example 8, Procedure 2, Step B.

### Step C. 4-[1-(8-Chloro-3,10-dibromo-6,11-dihydro-5H-benzo[5,6]cyclohepta[1,2-b]pyridin-11-yl)-4-piperidineacetyl]-1-piperazinecarboxamide

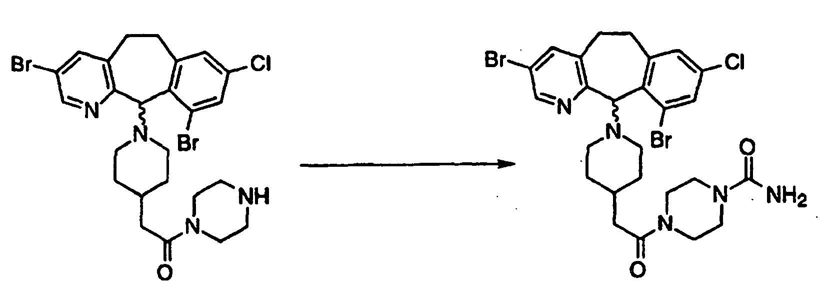

1-[1-(8-Chloro-3,10-dibromo-6,11-dihydro-5H-benzo[5,6]cyclohepta[1,2-b]pyridin-11-yl)-4-piperidineacetyl]piperazine (prepared as described in Step B above) is converted into 4-[1-(8-Chloro-3,10-dibromo-6,11-dihydro-5H-benzo[5,6]cyclohepta[1,2-b]pyridin-11-yl)-4-piperidineacetyl]-1-piperazinecarboxamide by essentially the same procedure as decribed in Example 8, Procedure 2, Step C above.

### Example 49. N-cyclopropyl-1-(3,10-dibromo-8-chloro-6,11-dihydro-5H-benzo[5,6]cyclohepta[1,2-b]pyridin-11-yl)-3-piperidinecarboxamide

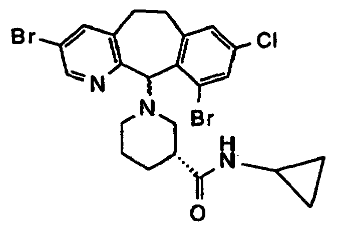

Following the method of Example 10, Procedure 1, except that (a) the compound 3,10-dibromo-8,10-dichloro-6,11-dihydro-5H-benzo[5,6]cyclohepta[1,2-b]pyridine is substituted for 3-bromo-8,10-dichloro-6,11-dihydro-5H-benzo[5,6]cyclohepta[1,2-b]pyridine in Step A, and (b) cyclopropyl amine is substituted for 3-aminomethylpyridine in Step C, the title compound is obtained. FABMS (MH+) = 554. FPT IC₅₀ = 0.58 uM.

### Example 50. 1-(3,10-Dibromo-8-chloro-6,11-dihydro-5H-benzo[5,6]cyclohepta[1,2-b]pyridin-11-yl)-N-methyl-3-piperidinecarboxamide

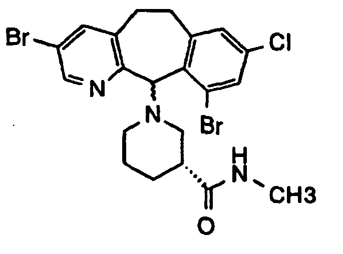

Following the method of Example 10, Procedure 1, except that (a) the compound 3,10-dibromo-8,10-dichloro-6,11-dihydro-5H-benzo[5,6]cyclohepta[1,2-b]pyridine is substituted for 3-bromo-8,10-dichtoro-6,11-dihydro-5H-benzo[5,6]cyclohepta[1,2-b]pyridine in Step A, and (b) methylamine is substituted for 3-aminomethylpyridine in Step C, the title compound is obtained. FABMS (MH+) = 528. FPT IC₅₀ = 0.96 uM.

### Example 51. 1-(3,10-Dibromo-8-chioro-6,11-dihydro-5H-benzo[5,6]cyclohepta[1,2-b]pyridin-11-yl)-N-(3-pyridinylmethyl)-3-piperidinecarboxamide N1-oxide (Isomer B)

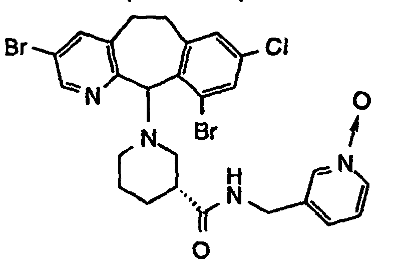

Following the method of Example 10, Procedure 1, except that (a) the compound 3,10-dibromo-8,10-dichloro-6,11-dihydro-5H-benzo[5,6]cyciohepta[1,2-b]pyridine is substituted for 3-bromo-8,10-dichloro-6,11-dihydro-5H-benzo[5,6]cyclohepta[1,2-b]pyridine in Step A, and (b) 3-aminomethylpyridine-N-oxide is substituted for 3-aminomethylpyridine in Step C, the title compound is obtained. FABMS (MH+) = 619.
FPT IC₅₀ = 0.1 uM.

### Example 52. 1-(3,10-Dibrorno-8-chforo-6,11-dihydro-5H-benzo[5,6]cyclohepta[1,2-b]pyridin-11-yl)-N-[2-(3-pyridinyl)ethyl]-3-piperidinecarboxamide

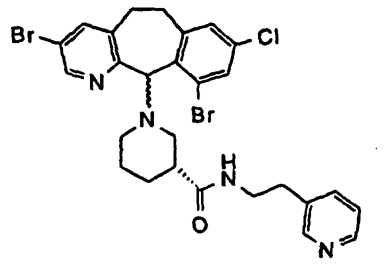

Following the method of example 10, procedure 1, except that (a) the compound 3,10-dibromo-8,10-dichtoro-6,11-dihydro-5H-benzo[5,6]cyclohepta[1,2-b]pyridine is substituted for 3-bromo-8,10-dichloro-6,11-dihydro-5H-benzo[5,6]cyclohepta[1,2-b]pyridine in Step A,and (b) 3-aminoethylpyridine is substituted for 3-aminomethylpyridine in Step C, the title compound is obtained. FABMS (MH+) = 617.
FPT IC₅₀ = 0.081 uM

### Example 53. 1-(3,10-Dibromo-8-chloro-6,11-dihydro-5H-benzo[5,6]cyclohepta[1,2-b]pyridin-11-yl)-N-[[1-[(dimethylamino)acetyl]-4-piperidinyl]methyl]-3-piperidinecarboxamide

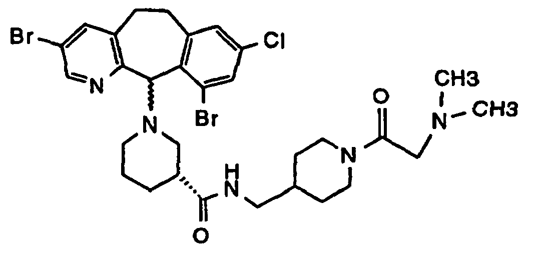

Following the method of Example 10, Procedure 1, except that (a) the compound 3,10-dibromo-8,10-dichloro-6,11-dihydro-5H-benzo[5,6]cyclohepta[1,2-b]pyridine is substituted for 3-bromo-8,10-dichloro-6,11-dihydro-5H-benzo[5,6]cyclohepta[1,2-b]pyridine in Step A,and (b) dimethylaminoacetyl-4-piperidinylmethyl-3-amine is substituted for 3-aminomethylpyridine in Step C, the title compound is obtained.
FABMS (MH+) = 694. FPT IC₅₀ = 0.11 uM

### Example 54. 1-(3,10-Dibromo-8-chloro-6,11-dihydro-5H-benzo[5,6]cyclohepta[1,2-b]pyridin-11-yl)-N-[3-(2-oxo-1-pyrrolidinyl)propyl]-3-piperidinecarboxamide

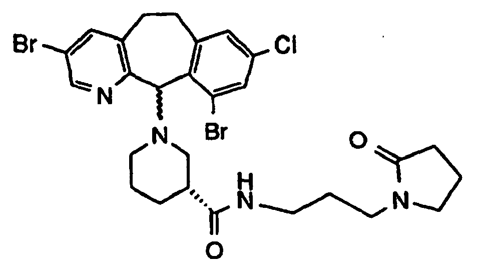

Following the method of Example 10, Procedure 1, except that (a) the compound 3,10-dibromo-8,10-dichloro-6,11-dihydro-5H-benzo[5,6]cyciohepta[1,2-b]pyridine is substituted for 3-bromo-8,10-dichloro-6,11-dihydro-5H-benzo[5,6]cyclohepta[1,2-b]pyridine in Step A, and (b) 3-aminopropylpyrrolidinone amine is substituted for 3-aminomethylpyridine in Step C, the title compound is obtained. FABMS (MH+) = 637.
FPT IC₅₀ = 0.1 uM

### PREPARATION OF STARTING MATERIALS

Starting materials useful in preparing the compounds of the present invention are exemplified by the following preparative examples, which should not be construed to limit the scope of the disclosure. The tricylic compounds (3.0) and substituted piperidinyl compounds (7.0) used as starting materials are known in the art and/or can be prepared using known methods, such as taught in U.S. Patents 5,089,496; 5,151,423; 4,454,143; 4,355,036; PCT /US94/11390 (WO95/10514); PCT/US94/11391 (WO 95/10515); PCT/US94/11392 (WO95/10516); Stanley R. Sandler and Wolf Karo, Organic Functional Group Preparations, 2nd Edition, Academic Press, Inc., San Diego, California, Vol. 1-3, (1983); in J. March, Advanced Organic Chemistry, Reactions & Mechanisms, and Structure, 3rd Edition, John Wiley & Sons, New York, 1346 pp. (1985); in G. R. Newkome (Ed.), Pyrdine and its Derivatives, John Wiley and Sons Inc., New York, N.Y., Vol. 1-5, (1984); A. J. Boulton and A. McKillop (Eds.), Comprehensive Heterocyclic Chemistry, Volume 2, Part 2A, Six Membered Rings With One Nitrogen Atom, Pergamon Press, Elmsford, New York, (1960-1985); and Chia-Lin J. Wang and Mark A. Wuonola, Recent Progress in the Synthesis and Reactions of Substituted Piperidines. A Review, Organic Preparations and Procedures International Vol 24, p. 585, (1992).The starting materials may also be prepared as taught in copending U.S. Application Serial No. 08/410,187 filed March 24, 1995, copending U.S. Application Serial No. 08/577,951 filed December 22, 1995, and copending U.S. Application Serial No. 08/615,760 filed March 13, 1996; the disclosures being incorporated herein by reference. Alternative mechanistic pathways and analogous structures within the scope of the invention may be apparent to those skilled in the art.

For example, piperidinyl compounds of formula (7.0), wherein T = -CO- or -CR³⁰R³¹- can be prepared by initially preparing a pyridine compound substituted with the requisite 2-, 3-, or 4 -(CH₂)ₙCR³⁰R³¹Z or -(CH₂)ₙCOZ moiety, together with any optional -R⁵, -R⁶, -R⁷ and/or -R⁸ moieties, as described, in the references cited above. The 2-, 3- or 4-substituted pyridine compound can subsequently be reduced using conventional reduction procedures, such as catalytic hydrogenation, to give the desired piperidinyl compound (7.0). One skilled in the art will appreciate that in cases where -R⁵, -R⁶, -R⁷, -R⁸ and/or Z moieties also contain reducible groups, it may useful to utilize alternative methods.

The sulfonylpiperidinyl compounds of formula (7.0), wherein T= -SO₂- can be prepared by reacting the appropriate 2-, 3-, or 4-hydroxy-N-blocked-piperidine with a suitable chlorinating agent such as thionyl chloride to obtain the 2-, 3-, or 4-chloro-N-blocked piperidine, using N-blocking groups such as benzyloxycarbonyl or tert-butoxycarbonyl. The 2-,3-, or 4-chloro-N-blocked piperidine can then be reacted with sodium bisulfite to obtain the corresponding 2-, 3-, or 4-sulfonic acid N-blocked piperidine sodium salt. This salt is then reacted with an appropriate chlorinating agent such as phosphorus pentachloride or phosphorus oxychloride to obtain the corresponding 2-, 3- or 4-sulfonylchloride-N-blocked piperidine. This sulfonyl chloride is then reacted with the corresponding agent containing the desired Z group (i.e. amines, alkylating agents and the like) to obtain the desired sulfonylpiperidine (7.0).

The sulfoxylpiperidine wherein T= -SO- (with the proviso that Z is not -NR⁴⁰R⁴²) can be prepared by reacting the appropriate 2-, 3-, or 4-hydroxy-N-blocked-piperidine with a suitable chlorinating agent such as thionyl chloride to obtain the 2-, 3-, or 4-chloro-N-blocked piperidine, using N-blocking groups such as benzyloxycarbonyl or tert-butoxycarbonyl. The 2-, 3-, or 4-chloro-N-blocked piperidine can then reacted with the corresponding substituted sulfide (i.e. arylsulfide, alkylsulfides and the like) to obtain the appropriate 2-, 3-, or 4-sulfide-N-blockedpiperidine. This compound can then be reacted with an oxidizing agent such as meta-chloroperbenzoic acid to obtain the desired sulfoxylpiperidine (7.0).

### PREPARATIVE EXAMPLE 1. 4-Piperidinyl-N-(4-pyridinyl)carboxamide

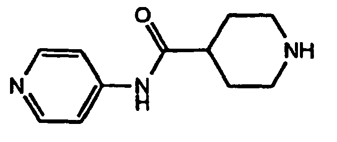

### Step A:

### 1-N-(tert-Butoxycarbonyl-4-piperidine carboxylic acid or 1-N-(tert -butoxycarbonyl)isonipecotic acid

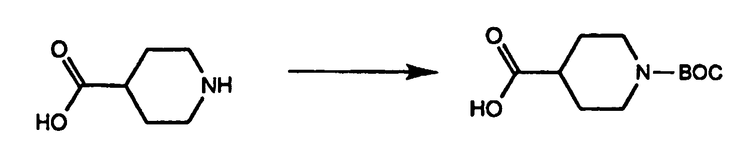

Isonipecotic acid (5g; 1 equivalent) is dissolved in water (50 ml) and a solution of di-*tert* -butyldicarbonate (8.62g; 1.02 equivalents) in THF (70 ml) is added with stirring. The mixture is stirred at 80°C for 2 h and then evaporated to dryness. The residue is partitioned between dichloromethane and brine and the dichloromethane layer is dried over magnesium sulfate, filtered and evaporated to dryness. The product is chromatographed on a silica gel column (30 X 5cm) using 15% (10% concentrated ammonium hydroxide in methanol)-dichloromethane as the eluant to give the title compound (4.3109g; 49% yield), CIMS: m/z 230 (MH⁺).

### Step B:

### 1-N-(tert -Butoxycarbonyl)-4-piperidinyl-N-(4-pyridinyl)carboxamide

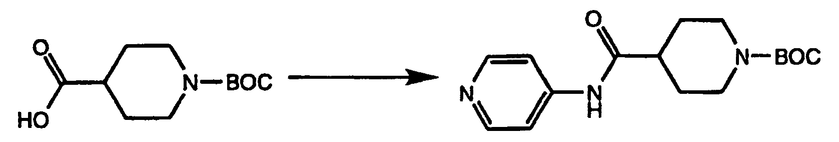

1-N-(*tert*-Butoxycarbonyl)-4-piperldinecarboxylic acid (1.218; 1 equivalent) (prepared as described in Step A above), DEC (1.0184g; 1 equivalent), HOBT (0.7179g; 1 equivalent) and N-methylmorpholine (0.5841ml; 1 equivalent) are dissolved in anhydrous DMF (30 ml) and the mixture is stirred under argon at 25°C for 19 h. The solution is evaporated to dryness. The residue is taken up in dichloromethane and washed with water. The dichloromethane layer is dried over magnesium sulfate, filtered and evaporated to dryness. The product is chromatographed on a silica gel column (60 X 2.5 cm) using 5% (10% concentrated ammonium hydroxide in methanol)-dichloromethane as the eluant to give the title compound (0.8142g; 50% yield), CIMS: m/z 306 (MH⁺).

### Step C:

### 4-Piperidinyl-N-(4-pyridinyl)carboxamide

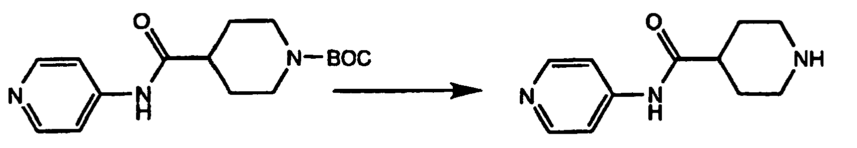

1-N-(*tert*-Butoxycarbonyl)-4-piperidinyl-N-(4-pyridyl)carboxamide (1g; 1 equivalent) is dissolved in 10% (v/v) concentrated sulfuric acid in dioxane (24.36ml) and the mixture is stirred at 25°C for 0.5 h. The mixture is poured into water (150 ml) and neutralized with Amberlite IRA401S(OH-) ion exchange resin (300ml). The resin is eluted with water (1500 ml) and the eluant is evaporated to give the title compound (0.4258g; 63% yield), CIMS: m/z 206 (MH⁺).

### PREPARATIVE EXAMPLE 2

### 1-[3-Bromo-8-chloro-6,11-dihydro-5H-benzo[5,6]cyclohepta[1,2-b]pyridin-11-yl]-4-piperidinecarboxylate

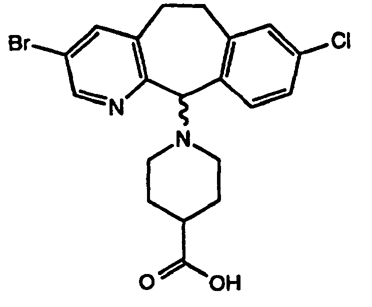

### Procedure 1, Step A:

### Ethyl 1-[3-bromo-8-chloro-6,11-dihydro-5H-benzo[5,6]cyclohepta[1,2-b]pyridin-11-yl]-4-piperidinecarboxylate

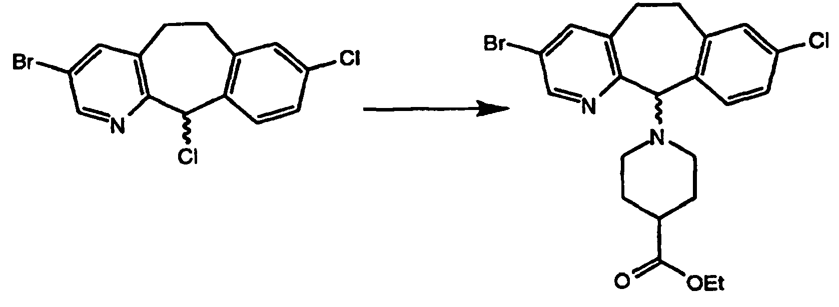

3-Bromo-8,11-dichtoro-6,11-dihydro-5H-benzo[5,6]cyctohepta[1,2-b]pyridine (1 g; 1 equivalent) and ethyl isonipecotate (2.3735ml) (5 equivalents) are dissolved in dry THF (20ml) and the mixture was stirred at 25°C under argon for 19h. The solution is evaporated to dryness and the residue is taken up in dichloromethane and washed with 1.0N sodium hydroxide. The dichloromethane layer is dried over magnesium sulfate, filtered and evaporated to dryness. The product is chromatographed on a silica gel column (30X5cm) using 0.75% (10% concentrated ammonium hydroxide in methanol)-dichloromethane as the eluant to give the title compound (1.5134g; 100% yield), CIMS: m/z 463.15 (MH⁺).

### Step B:

### 1-[3-Bromo-8-chloro-6,11-dihydro-5H-benzo[5,6]cyclohepta[1,2-b]pyridin-11-yl]-4-piperidinecarboxylate

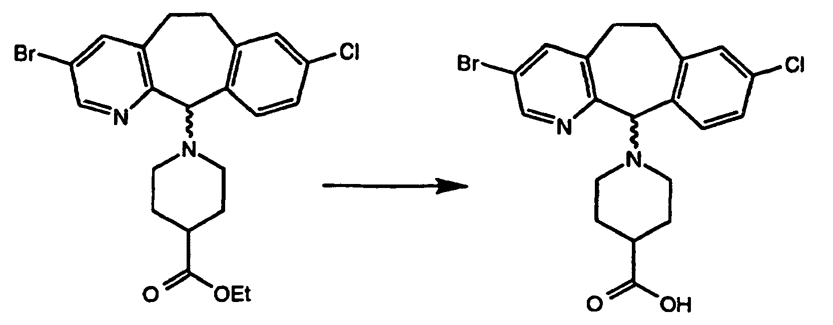

Ethyl 1-[3-bromo-8-chloro-6,11-dihydro-5H-benzo[5,6]cyclohepta [1,2-b]pyridin -11-yl]-4-piperidinecarboxylate (0.250g; 1 equivalent) (prepared as described in Step A above), is dissolved in ethanol (3ml) and dichloromethane (3ml) and 1.0M lithium hydroxide in water (1.3044m1; 2.42 equivalents) is added The mixture is stirred at 50°C for 5h. 1.0N Hydrochloric acid (1.5169ml) (2.81 equivalents) is added and the solution is evaporated to dryness after stirring for 5 min. to give the title compound which is used without further purification.

### Procedure 2:

### 1-[3-Bromo-8-chloro-6,11-dihydro-5H-benzo[5,6]cyclohepta[1,2-b]pyridin-11-YL]-4-piperidinecarboxylate

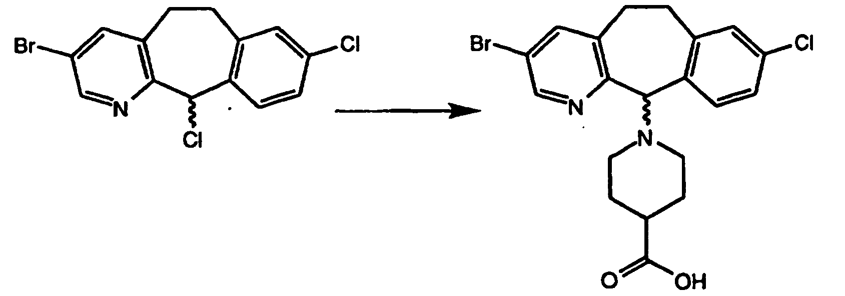

3-Bromo-8,11-Dichloro-6,11-dihydro-5H-benzo[5,6]cyclohepta[1,2-b]pyridine (prepared as described in Preparative Example 40, Step B in INO291K) (0.5g; 1 equivalent), isonipecotic acid (0.3978g; 2 equivalents) and 4-N-methylmorpholine (0.847ml; 5 equivalents) are dissolved in anhydrous DMF (9.6ml) and the mixture is heated at 80°C for 16.5h. The solution is evaporated to dryness and the product is chromatographed on a silica gel column (60X2.5cm) using 10% ethyl acetate in hexane, followed by 1% (10% concentrated ammonium hydroxide in methanol)-dichloromethane as the eluant to give the title compound (0.2426g; 36% yield), CIMS: m.z 435.1 (MH⁺).

### PREPARATIVE EXAMPLE 3

### 4-(Aminomethyl)-1-N-(tert-butoxycarbonyl)piperidine

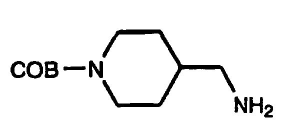

Ref.: J. D. Prugh, L. A. Birchenough and M. S. Egbertson, Synthetic Communications, 22(16), 2357-2360 (1992).

### Step A:

### 4-(Benzylidineaminomethyl)piperidine

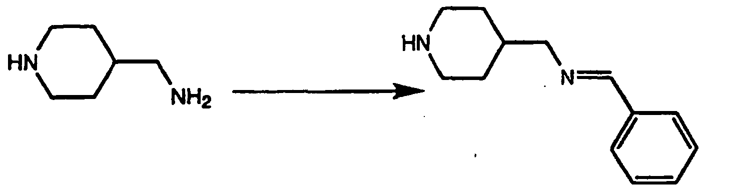

4-Aminomethylpiperidine (11.4g) (1 equivalent) is dissolved in anhydrous toluene (125ml) and benzaldehyde (10.6g) (1 equivalent) is added. The mixture is heated at 120°C under reflux for 4h, using a Dean-Stark trap to remove water. The crude solution of the title compound is used directly in Step B below.

### Step B:

### 1-N-(tert-Butoxycarbonyl)-4-(benZylidineaminomethyl)piperidine

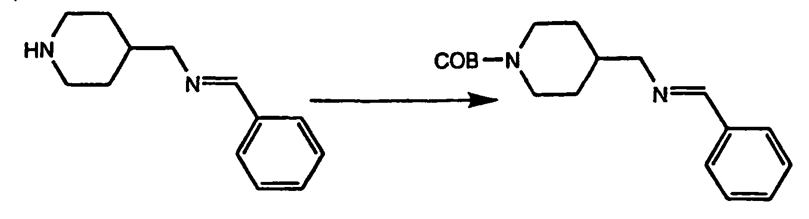

4-(Benzylideneaminomethyl)piperidine in toluene (From Step A above) is treated with di-*tert*-butyldicarbonate (24g) (1.1 equivalents) in portions over 0.5h. The mixture is stirred at 25°C for 69h. The solution is evaporated to dryness to give the title compound which is used directly in Step C below.

### Step C:

### 4-(Aminomethyl)-1-N-(tert-butoxycarbonyl)piperidine

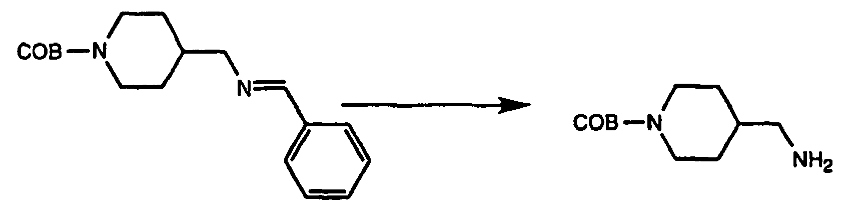

1-N-(*tert*-Butoxycarbonyl)-4-(benzylideneaminomethyl)piperidine (prepared as described in Step B above) is dissolved in 1.0N aqueous potassium hydrogen sulfate (220ml) and the mixture is stirred at 25°C for 4h. The solution is extracted with ether (3X200ml) and the ether is discarded. The aqueous layer is adjusted to pH 12.5 using 50% aqueous sodium hydroxide and the solution is then saturated with solid sodium chloride and extracted with dichloromethane. The dichloromethane layer is dried over magnesium sulfate, filtered and evaporated to dryness. The product is chromatographed on a silica gel column (60 X 5cm) using 1% increasing to 7% (10% concentrated ammonium hydroxide in methanol)-dichloromethane as the eluant to give the title compound (9.82g; 46% yield), an oil, CIMS: m/z 215 (MH⁺).

### PREPARATIVE EXAMPLE 4 1-N-Benzyl-4-(aminomethyl)piperidine

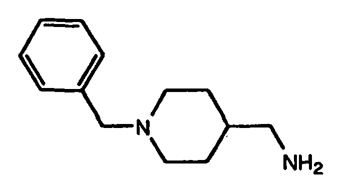

### Step A:

### 1-N-Benzyl-4-piperidinecarboxamide

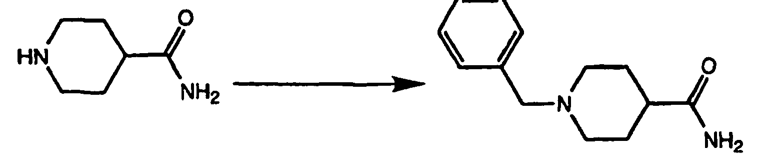

4-Piperidinecarboxamide (5g; 1 equivalent) and triethylamine (16.3ml) (3 equivalents) are dissolved in anhydrous dichloromethane (30ml) and anhydrous DMF (80ml). A solution of benzyl bromide (4.55ml) (0.98 equivalents) in anhydrous dichloromethane (10ml) is added dropwise over 10 min and the mixture is stirred at 25°C for 22h. The mixture is filtered and the filtrate is evaporated to dryness. The product is chromatographed on a silica gel column (60X5cm) using dichloromethane (1 litre) and then 5% (10% concentrated ammonium hydroxide in methanol)-dichloromethane as the eluant to give the title compound (6.15g; 72% yield), CIMS: m/z 219.05 (MH⁺).

### Step B:

### 1-N-Benzyl-4-(aminomethyl)piperidine

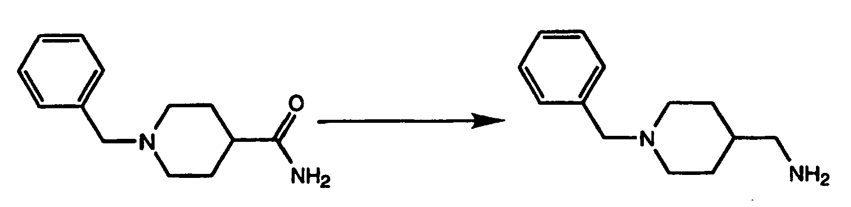

1-N-Benzyl-4-piperidinecarboxamide (1g; 1 equivalent) (prepared as described in Step A above) is dissolved in anhydrous THF (25ml). Lithium aluminum hydride (0.2173g) (1.25 equivalents) in anhydrous THF (5.726ml) is added dropwise over 0.5h and the mixture is heated under reflux under nitrogen for 20h. The mixture is cooled and diluted with dichloromethane (750ml) and washed with 1.0N sodium hydroxide. The dichloromethane layer is dried over magnesium sulfate, filtered and evaporated to dryness. The product is chromatographed on a silica gel column (60X2.5cm) using 2% (10% concentrated ammonium hydroxide in methanol)-dichloromethane as the eluant to give the title compound (0.5237g; 56% yield), FABMS: m/z 205.4 (MH⁺).

### PREPARATIVE EXAMPLE 5

### Step A:

### 4-(N-Benzyloxycarbonylaminomethyl)piperidine

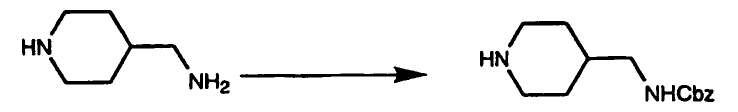

4- Aminomethylpiperidine (1 g; 1 equivalent) and DMAP (0.054g; 0.05 equivalents) are dissolved in anhydrous dichloromethane (40 ml). N-Benzyloxycarbonylimidazole (1.7709g; 1 equivalent) [prepared as described in: S. K. Sharma, M. J. Miller and S. M. Payne, J. Med. Chem., 32, 357-367 (1989)] is added and the mixture is stirred at 25°C for 23 h. The solution is diluted with dichloromethane and washed with 1.0N sodium hydroxide. The dichloromethane layer is dried over magnesium sulfate, filtered and evaporated to dryness. The product is chromatographed on a silica gel column (60X2.5cm) using 3% increasing to 7% (10% concentrated ammonium hydroxide in methanol)-dichloromethane as the eluant to give the title compound (1.0719g; 49% yield), FABMS: m/z 249.3 (MH⁺).

### Step B:

### 1-N-(Benzyloxycarbonyl)-4-[N-(tert-butoxycarbonyl)aminomethyl]piperidine

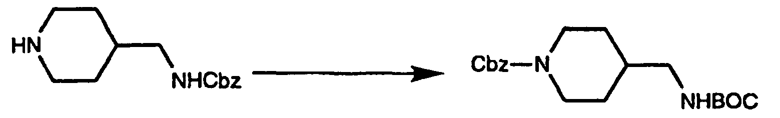

4-(N-Benzyloxycarbonylaminomethyl)piperidine (0.6814g; 1 equivalent) (prepared as described in Step A above) is dissolved in anhydrous toluene (5 ml) and di-*tert* -butyldicarbonate (0.599g; 1 equivalent) in anhydrous toluene (5 ml) is added dropwise. The mixture is stirred at 0°C for 2h and at 25°C for 20 h. The solution is evaporated to dryness and the residue is taken up in dichloromethane and washed with 1.0N sodium hydroxide. The dichloromethane layer is dried over magnesium sulfate, filtered and evaporated to dryness. The product is chromatographed on a silica gel column (60X2.5cm) using 0.5% (10% concentrated ammonium hydroxide in methanol)-dichloromethane as the eluant to give the title compound (0.9314g; 97% yield), FABMS: m/z 349.3 (MH⁺).

### Step C:

### 4-[(tert-Butoxycarbonylamino)methyl]piperidine

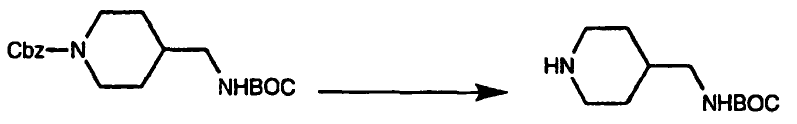

1-N-(Benzyloxycarbonyl)-4-[(*tert*-butoxycarbonylamino)methyl]-piperidine (0.4g) (1 equivalent) (prepared as described in Step B above) is dissolved in methanol (16ml) and 5% Pd-C (0.0638g) is added. The mixture is hydrogenated at 30 psi at 25°C for 17h. The catalyst is removed by filtration through Celite which is washed with methanol. The combined filtrates are evaporated to dryness. The residue is taken up in dichloromethane and washed with 1.0N sodium hydroxide. The dichloromethane layer is dried over magnesium sulfate, filtered and evaporated to dryness. The product is chromatographed on a silica gel column (45X2.5cm) using 2% increasing to 7% (10% concentrated ammonium hydroxide in methanol)-dichloromethane as the eluant to give the title compound (0.2001 g; 81% yield), FABMS: m/z 215.4 (MH⁺).

### PREPARATIVE EXAMPLE 6

### Step A

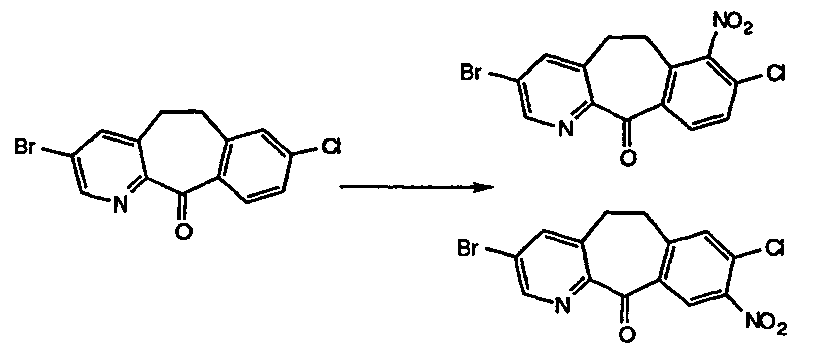

Combine 40.0 g (0.124 mole) of the starting ketone and 200 mL of H₂SO₄ and cool to 0°C. Slowly add 13.78 g (0.136 mole) of KNO₃ over a period of 1.5 hrs., then warm to room temperature and stir overnight. Work up the reaction using substantially the same procedure as described for Preparative Example 4, Step A. Chromatograph (silica gel, 20%, 30%, 40%, 50% EtOAc/hexane, then 100% EtOAc) to give 28 g of the 9-nitro product, along with a smaller quantity of the 7-nitro product and 19 g of a mixture of the 7-nitro and 9-nitro compounds.

### Step B

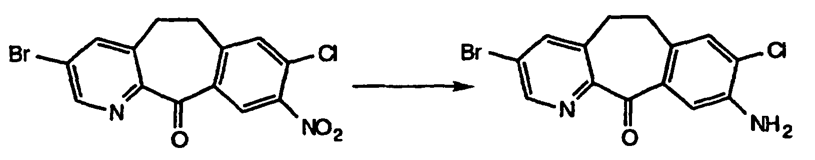

React 28 g (76.2 mmol) of the 9-nitro product of Step A, 400 mL of 85% EtOH/water, 3.8 g (34.3 mmol) of CaCl₂ and 38.28 g (0.685 mole) of Fe using substantially the same procedure as described for Preparative Example 4, Step C, to give 24 g of the product

### Step C

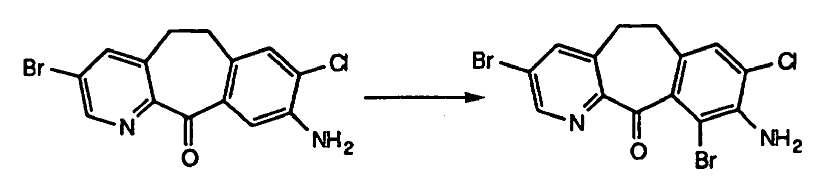

Combine 13 g (38.5 mmol) of the product of Step B, 140 mL of HOAc and slowly add a solution of 2.95 mL (57.8 mmol) of Br₂ in 10 mL of HOAc over a period of 20 min. Stir the reaction mixture at room temperature, then concentrate *in vacuo* to a residue. Add CH₂Cl₂ and water, then adjust to pH = 8-9 with 50% NaOH (aqueous). Wash the organic phase with water, then brine and dry over Na₂SO₄. Concentrate *in vacuo* to give 11.3 g of the product.

### Step D

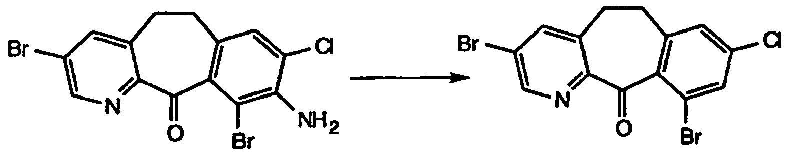

Cool 100 mL of concentrated HCl (aqueous) to 0°C, then add 5.61 g (81.4 mmol) of NaNO₂ and stir for 10 min. Slowly add (in portions) 11.3 g (27.1 mmol) of the product of Step C and stir the mixture at 0°-3°C for 2.25 hrs. Slowly add (dropwise) 180 mL of 50% H₃PO₂ (aqueous) and allow the mixture to stand at 0°C overnight. Slowly add (dropwise) 150 mL of 50% NaOH over 30 min., to adjust to pH = 9, then extract with CH₂Cl₂. Wash the extract with water, then brine and dry over Na₂SO₄. Concentrate *in vacuo* to a residue and chromatograph (silica gel, 2% EtOAc/ CH₂Cl₂) to give 8.6 g of the product.

### Step E

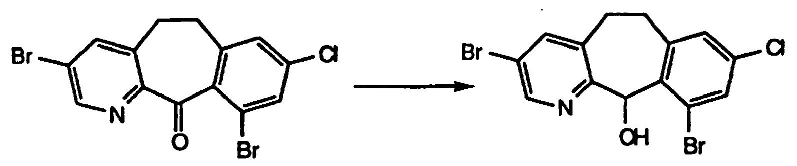

Combine 8.6 g (21.4 mmol) of the product of Step D and 300 mL of MeOH and cool to 0°-2°C. Add 1.21 g (32.1 mmol) of NaBH₄ and stir the mixture at ~0°C for 1 hr. Add another 0.121 g (3.21 mmol) of NaBH₄, stir for 2 hr. at 0°C, then let stand overnight at 0°C. Concentrate *in vacuo* to a residue then partition the residue between CH₂Cl₂ and water. Separate the organic phase and concentrate *in vacuo* (50°C) to give 8.2 g of the product.

### Step F. 3,10-dibromo-8,11-dichloro-6,11-dihydro-5H-benzo[5,6]cyclohepta[1,2-b]pyridine

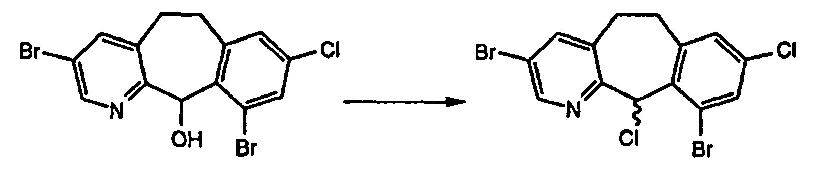

Combine 8.2 g (20.3 mmol) of the product of Step E and 160 mL of CH₂Cl₂, cool to 0°C, then slowly add (dropwise) 14.8 mL (203 mmol) of SOCl₂ over a 30 min. period. Warm the mixture to room temperature and stir for 4.5 hrs., then concentrate *in vacuo* to to give the title compound.

### PREPARATIVE EXAMPLE 7. N-(3-Pyridylmethyl)-4-piperidineacetamide

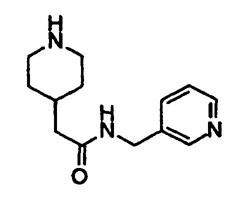

### Step A.

### 1-tert-butoxycarbonyl-N-(3-pyridylmethyl)-4-piperidineacetamide

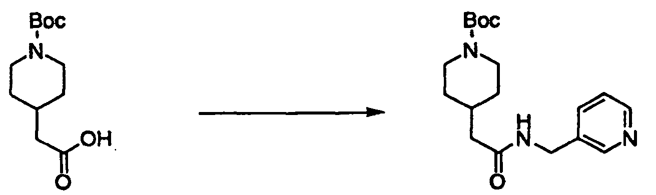

1-*tert*-Butoxycarbonyl-4-piperidineacetic acid (5g, 20.55mmoles) (prepared as described in Preparative Example 17, Step A in INO291K), 3-aminomethylpyridine (2.72g, 26.7mmoles), DEC.HCl (5.12g, 26.7mmoles), HOBT (3.61 g, 26.7mmoles) and NMM (2.94ml, 26.7mmoles) are dissolved in anhydrous DMF (100ml) and the mixture is stirred under argon at 25°C for 22h. The solution is evaporated to dryness and the residue is dissolved in dichloromethane, washed with 1N NaOH, dried over magnesium sulfate, filtered and evaporated to dryness. The residue is chromatographed on silica gel using 2% (10% conc. ammonium hydroxide in methanol)-dichloromethane as the eluant to give the title compound (6.05g, 77%), ESIMS: m/z 334.1 (MH⁺).

### Step B.

### N-(3-Pyridylmethyl)-4-piperidineacetamide

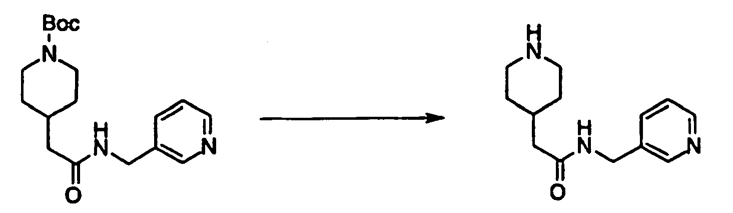

1-*tert*-Butoxycarbonyl-N-(3-pyridylmethyl)-4-piperidineacetamide (5.59g, 16.76mmoles) is dissolved in methanol (100ml) and 10% conc. sulfuric acid in dioxane (v/v) (250ml) is added. The mixture is stirred at 25°C for 2h and the neutralized with Bio Rad AG-1X8 (OH-) resin. The resin is washed with methanol and the eluate is evaporated to dryness. The residue is chromatographed on silica gel using 5%-20%-30% (10% conc. ammonium hydroxide in methanol)-dichloromethane as the eluant to give 3.64 g (93% yield) of the title compound: ESIMS: m/z 234.1 (MH⁺).

### PREPARATIVE EXAMPLE 8. N-(3-Pyridylmethyl)-4-piperidinepropanamide

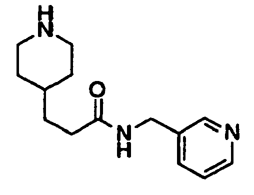

### Step A.

### 4-Piperidinepropionic acid

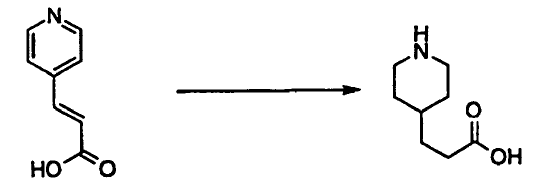

3-(4-Pyridyl)acrylic acid (2g, 13.4mmoles) is dissolved in water (70ml) and concentrated hydrochloric acid (1 ml). 10% Pd-C (1.5 spatulas) is added and the mixture is hydrogenated at 25°C at 55psi for 72h. The mixture is filtered through Celite^{®} and then passed over a bed of Bio Rad AG 1-X8 (OH⁻) resin. The resin is washed with water and the combined eluates are evaporated to dryness to give the title compound that is used in Step B without further purification.

### Step B.

### 1-tert-Butoxycarbonyl-4-piperidinepropionic acid

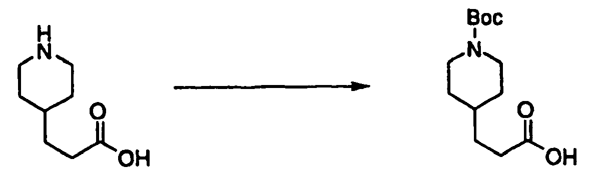

4-Piperidinepropionic acid (13.4mmoles) (prepared as described in Step A above), di-*tert*-butyldicarbonate (3.22g, 14.75mmoles) and sodium hydroxide (0.5364g, 13.4mmoles) are dissolved in THF-water (1:1) (40ml) and the mixture is stirred at 25°C for 18h. The mixture is passed over Bio Rad 50WX4 (H⁺) resin (15ml bed) and the resin is washed with THF-water. The combined eluates are evaporated to dryness and then azeotroped with THF to give the title compound (2.72g, 79%), FABMS: m/z 258.1 (MH⁺).

### Step C.

### N-(3-Pyridylmethyl)-1-tert-butoxycarbonyl-4-piperidinepropanamide

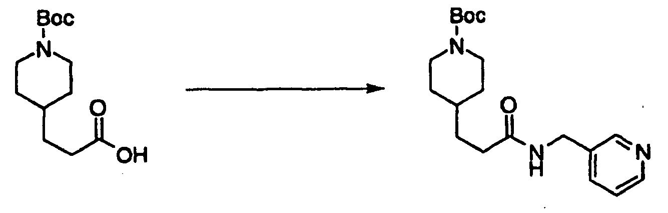

1-*tert*-Butoxycarbonyl-4-piperidinepropionic acid (2g, 7.77mmoles), 3-(aminomethyl)pyridine (1.029ml, 10.1mmoles), DEC.HCl (1.937g, 10.1 mmoles), HOBT (1.365g, 10.1 mmoles) and NMM (1.111 ml, 10.1 mmoles) are dissolved in anhydrous DMF (25ml) and the mixture is stirred under argon at 25°C for 20h. The solution is evaporated to dryness and the residue is taken up in dichloromethane, washed with 0.3N NaOH, dried over magnesium sulfate, filtered and evaporated to dryness. The residue is chromatographed on silica gel using 1.5%-2.5% (10% conc. ammonium hydroxide in methanol)-dichloromethane as the eluant to give the title compound (2.555g, 95%), ESIMS: m/z 348.1 (MH⁺).

### Step D.

### N-(3-Pyridylmethyl)-4-piperidinepropanamide

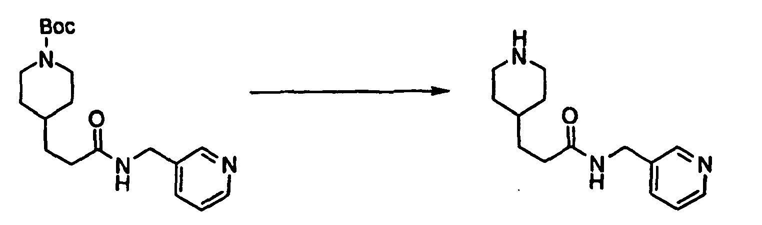

N-(3-Pyridylmethyl)-1-*tert*-butoxycarbonyl-4-piperidinepropanamide (2.222g, 6.4mmoles) is dissolved in methanol (38.15ml) and 10% conc. H₂SO₄ in dioxane (v/v) (95.38ml) is added and the mixture is stirred under argon at 25°C for 1.5h. The volume is reduced to half and the mixture is basified to pH 12 with 50% NaOH aq and extracted with dichloromethane. The latter is dried over magnesium sulfate, filtered and evaporated to dryness. The residue is chromatographed on silica gel using 10% (10% conc. ammonium hydroxide in methanol)-dichloromethane as the eluant to give the title compound (0.9586g, 61 %), CIMS: m/z 248.25 (MH⁺).

### PREPARATIVE EXAMPLE 9. Ethyl 4-piperidineacetate

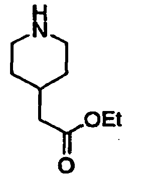

### Step A.

### Ethyl 1-tert-butoxycarbonyl-4-piperidineacetate

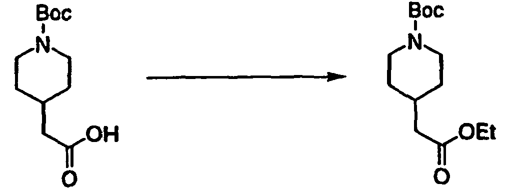

1-*tert* -Butoxycarbonyl-4-piperidineacetic acid (1 g, 4.1 mmoles) (prepared as described in Preparative Example 17, Step C in INO291K), ethanol (200 proof) (0.284g, 0.362ml, 6.2mmoles), DEC.HCl (1.18g, 6.2mmoles), HOBT (0.8331g, 6.2mmoles) and NMM (0.624g, 0.678ml, 6.2mmoles) are dissolved in anhydrous DMF (30ml) and the mixture is stirred at 25°C under argon for 24h. The solution is evaporated to dryness and the residue is dissolved in dichloromethane, washed with satd, NaHCO₃ aq, water, dried over magnesium sulfate, filtered and evaporated to dryness. The residue is chromatographed on silica gel using 0.5% (10% conc. ammonium hydroxide in methanol)-dichloromethane as the eluant to give the title compound (0.682g, 61 %), ESIMS: m/z 272.0 (MH⁺).

### Step B. Ethyl 4-piperidineacetate

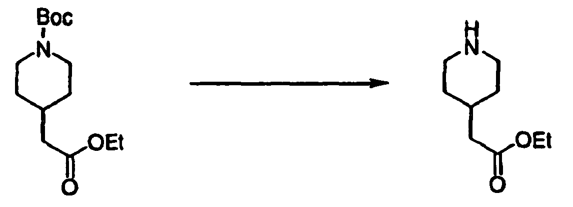

Ethyl 1-*tert*-butoxycarbonyl-4-piperidineacetate (0.6g, 2.2mmoles) is dissolved in ethanol (30ml) and 10% conc. H₂SO₄ in dioxane (v/v) (30ml) is added and the mixture is stirred at 25°C for 2h. The mixture is passed over a bed of Bio Rad AG1-X8 (OH⁻) resin and the resin is then eluted with ethanol. The combined eluates are evaporated to dryness and the residue is chromatographed on silica gel using 1% (10% conc. ammonium hydroxide in methanol)-dichloromethane as the eluant to give the title compound.

### PREPARATIVE EXAMPLE 10

### 1-(8-Chloro-3,10-dibromo-6,11-dihydro-5H-benzo[5,6]cyclohepta[1,2-b]pyridin-11-yl)-4-piperidineacetic acid

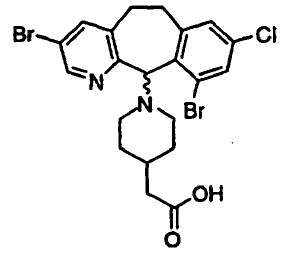

### Step A.

### Ethyl 1-(8-Chloro-3,10-dibromo-6,11-dihydro-5H-benzo[5,6]cyclohepta[1,2-b]pyridin-11-yl)-4-piperidineacetate

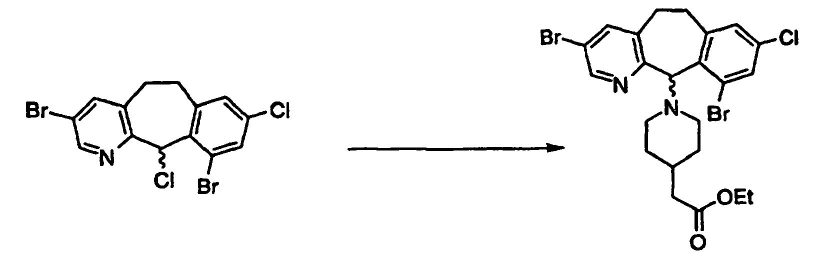

3,10-Dibromo-8,11-dichloro-6,11-dihydro-5H-benzo[5,6] cyclohepta[1,2-b]pyridine (prepared as described in Preparative Example 6, Step F) (0.486g, 1.15mmoles) is dissolved in anhydrous THF (5ml). Ethyl 4-piperidineacetate (prepared as described in Preparative Example 9, Step B) (0.6241g, 2.3mmoles) and triethylamine (0.321ml, 2.3mmoles) in anhydrous THF (5ml) are added and the mixture is stirred at 25°C for 24h. The solution is evaporated to dryness and the residue is dissolved in dichloromethane and washed with 1N NaOH , dried (MgSO₄), filtered and evaporated to dryness. The product is chromatographed on silica gel using 5% (10% conc. ammonium hydroxide in methanol)-dichloromethane as the eluant to give the title compound.

### Step B.

### 1-(8-Chloro-3,10-dibromo-6,11-dihydro-5H-benzo[5,6]cyclohepta[1,2-b]pyridin-11-yl)-4-piperidineacetic acid

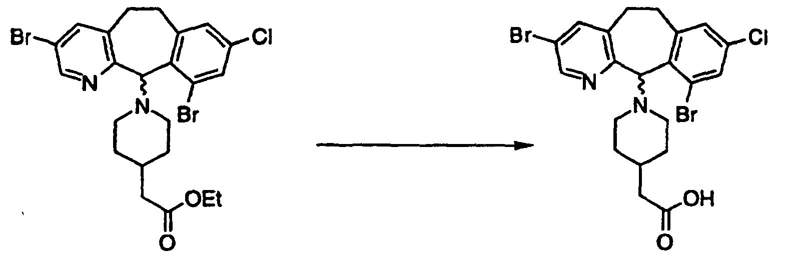

Ethyl 1-(8-Chloro-3,10-dibromo-6,11-dihydro-5H-benzo[5,6]cyclohepta[1,2-b]pyridin-11-yl)-4-piperidineacetate (0.3g, 0.5mmoles) (prepared as described in Step A above) is dissolved in ethanol (4ml) and dichloromethane (4ml) and 1 M lithium hydroxide in water (1.21 mmoles) is added. The mixture is stirred at 50°C for 5h. 1 N Hydrochloric acid (1.21 mmoles) is added and the solution is evaporated to dryness to give the title compound which is used without further purification.

### ASSAYS

1. In vitro enzyme assays: FPT IC₅₀ (inhibition of famesyl protein transferase, in vitro enzyme assay) are determined by the methods disclosed in WO/10515 or WO 95/10516. The data demonstrate that the compounds of the invention are inhibitors of Ras-CVLS farnesylation by partially purified rat brain famesyl protein transferase (FPT). The data also show that there are compounds of the invention which can be considered as potent (IC₅₀ <10 µM) inhibitors of Ras-CVLS famesylation by partially purified rat brain FPT.
   Under the test protocols employed, there were certain compounds within the scope of the present invention which did not exhibit activity. It is believed that such compounds would exhibit activity under a different test protocol.
2. Cell-based assay. COS IC₅₀ values refer to the COS cells activity inhibition of Ras processing, are determined by the methods disclosed in WO/10515 or WO 95/10516.

For preparing pharmaceutical compositions from the compounds described by this invention, inert, pharmaceutically acceptable carriers can be either solid or liquid. Solid form preparations include powders, tablets, dispersible granules, capsules, cachets and suppositories. The powders and tablets may be comprised of from about 5 to about 70 percent active ingredient. Suitable solid carriers are known in the art, e.g. magnesium carbonate, magnesium stearate, talc, sugar, lactose. Tablets, powders, cachets and capsules can be used as solid dosage forms suitable for oral administration.

For preparing suppositories, a low melting wax such as a mixture of fatty acid glycerides or cocoa butter is first melted, and the active ingredient is dispersed homogeneously therein as by stirring. The molten homogeneous mixture is then poured into convenient sized molds, allowed to cool and thereby solidify.

Liquid form preparations include solutions, suspensions and emulsions. As an example may be mentioned water or water-propylene glycol solutions for parenteral injection.

Liquid form preparations may also include solutions for intranasal administration.

Aerosol preparations suitable for inhalation may include solutions and solids in powder form, which may be in combination with a pharmaceutically acceptable carrier, such as an inert compressed gas.

Also included are solid form preparations which are intended to be converted, shortly before use, to liquid form preparations for either oral or parenteral administration. Such liquid forms include solutions, suspensions and emulsions.

The compounds of the invention may also be deliverable transdermally. The transdermal compositions can take the form of creams, lotions, aerosols and/or emulsions and can be included in a transdermal patch of the matrix or reservoir type as are conventional in the art for this purpose.

Preferably the compound is administered orally.

Preferably, the pharmaceutical preparation is in unit dosage form. In such form, the preparation is subdivided into unit doses containing appropriate quantities of the active component, e.g., an effective amount to achieve the desired purpose.

The quantity of active compound in a unit dose of preparation may be varied or adjusted from about 0.1 mg to 1000 mg, more preferably from about 1 mg. to 300 mg, according to the particular application.

The actual dosage employed may be varied depending upon the requirements of the patient and the severity of the condition being treated. Determination of the proper dosage for a particular situation is within the skill of the art. Generally, treatment is initiated with smaller dosages which are less than the optimum dose of the compound. Thereafter, the dosage is increased by small increments until the optimum effect under the circumstances is reached. For convenience, the total daily dosage may be divided and administered in portions during the day if desired.

The amount and frequency of administration of the compounds of the invention and the pharmaceutically acceptable salts thereof will be regulated according to the judgment of the attending clinician considering such factors as age, condition and size of the patient as well as severity of the symptoms being treated. A typical recommended dosage regimen is oral administration of from 10 mg to 2000 mg/day preferably 10 to 1000 mg/day, in two to four divided doses to block tumor growth. The compounds are non-toxic when administered within this dosage range.

The following are examples of pharmaceutical dosage forms which contain a compound of the invention. The scope of the invention in its pharmaceutical composition aspect is not to be limited by the examples provided.

### Pharmaceutical Dosage Form Examples

### EXAMPLE A-Tablets

| No. | Ingredients | mg/tablet | mg/tablet |
|---|---|---|---|
| 1. | Active compound | 100 | 500 |
| 2. | Lactose USP | 122 | 113 |
| 3. | Com Starch, Food Grade, as a 10% paste in Purified Water | 30 | 40 |
| 4. | Com Starch, Food Grade | 45 | 40 |
| 5. | Magnesium Stearate | 3 | 7 |
| | Total | 300 | 700 |

### Method of Manufacture

Mix Item Nos. 1 and 2 in a suitable mixer for 10-15 minutes. Granulate the mixture with Item No. 3. Mill the damp granules through a coarse screen (e.g., 1/4", 0.63 cm) if necessary. Dry the damp granules. Screen the dried granules if necessary and mix with Item No. 4 and mix for 10-15 minutes. Add Item No. 5 and mix for 1-3 minutes. Compress the mixture to appropriate size and weigh on a suitable tablet machine.

### EXAMPLE B-Capsules

| No. | Ingredient | mg/capsule | mg/capsule |
|---|---|---|---|
| 1. | Active compound | 100 | 500 |
| 2. | Lactose USP | 106 | 123 |
| 3. | Corn Starch, Food Grade | 40 | 70 |
| 4. | Magnesium Stearate NF | 7 | 7 |
| | Total | 253 | 700 |

### Method of Manufacture

Mix Item Nos. 1, 2 and 3 in a suitable blender for 10-15 minutes. Add Item No. 4 and mix for 1-3 minutes. Fill the mixture into suitable two-piece hard gelatin capsules on a suitable encapsulating machine.

## Claims

1. A compound of the formula: 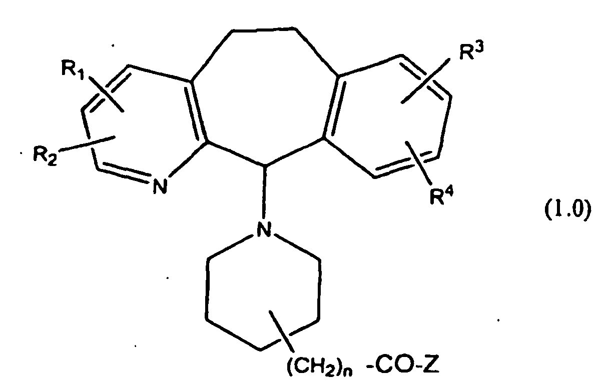 or a pharmaceutically acceptable salt thereof, wherein
n = 0, 1 or 2,
R¹ and R⁴ are H and R² and R³ are halo selected from chloro or bromo, or,
R¹ is H and R², R³ and R⁴ are halo selected from chloro or bromo,
Z is -NR⁴⁰R⁴² wherein R⁴⁰ and R⁴² independently represent H, aryl, alkyl, aralkyl, heteroaryl, heteroarylalkyl, heterocycloalkyl, heterocycloalkylalkyl, heteroalkyl, cycloalkyl or cycloalkylalkyl; or
Z is 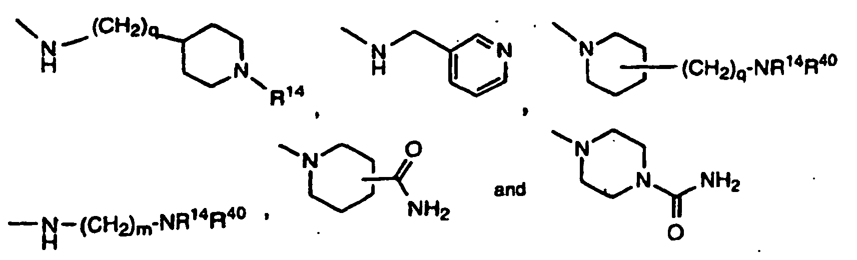 wherein R⁴⁰ is defined hereinbefore,
m is 2, 3 or 4;
q is 0 (zero), 1 or 2;
and R¹⁴ represents H, C₁₋₆ alkyl, aralkyl, heteroaryl, acyl, carboxamido, carboxamidoalkyl, cyano, alkoxycarbonyl, aralkyloxycarbonyl imido, imidamido, sulfamoyl, sulfonyl, dialkylphosphinyl, N-glycosyl or -C(NHCH₃)=CHNO₂.
and wherein
alkyl-(including the alkyl portions of alkoxy, alkylamino and dialkylamino)-represents straight and branched carbon chains and contains from one to twenty carbon atoms;
alkenyl - represents straight and branched carbon chains having at least one carbon to carbon double bond and containing from 2 to 12 carbon atoms;
alkynyl - represents straight and branched carbon chains having at least one carbon to carbon triple bond and containing from 2 to 12 carbon atoms;
aryl (including the aryl portion of aralkyl)-represents a carbocyclic group containing from 6 to 15 carbon atoms and having at least one aromatic ring;
aralkyl- represents an alkyl group, as defined above, wherein one or more hydrogen atoms of the alkyl moiety have been substituted with one or more aryl groups;
cycloalkyl - represents saturated carbocyclic rings branched or unbranched of from 3 to 20 carbon atoms;
cycloalkylalkyl - represents an alkyl group, as defined above, wherein one ore more hydrogen atoms of the alkyl moiety have been substituted with one or more cycloalkyl groups;
heteroalkyl - represents straight and branched carbon chains containing from one to twenty carbon atoms, interrupted by 1 to 3 heteroatoms selected from -O-, -S- and -N-;
heteroaryl - represents cyclic groups having at least one heteroatom selected from O, S and N, said heteroatom(s) interrupting a carbocyclic ring structure and having a sufficient number of delocalized pi electrons to provide aromatic character, with the aromatic heterocyclic groups containing from 2 to 14 carbon atoms, wherein said heteroaryl group optionally can be fused with one or more aryl, cycloalkyl, heteroaryl or heterocycloalkyl rings;
heterocycloalkyl-represents a saturated, branched or unbranched carbocyclic ring containing from 3 to 15 carbon atoms, which carbocyclic ring is Interrupted by 1 to 3 heteroatoms selected from -O-. -S- and -N-, wherein optionally, said ring may contain one or two unsaturated bonds which do not impart aromatic character to the ring;
wherein said alkyl, alkenyl, alkyaryl, cycloalkyl, cychoalkylaryl, aryl, aralkyl, heteroaryl, heteroalkyl and hetero cycloalkyl groups may be optionally and independently substituted with one, two, three or more of the following: halo, alkyl, aryl, alkoxy, amino, alkylamino, cyano, -CF₃, dialkylamino, hydroxy, oxy, phenoxy, -OCF₃, heterocycloalkyl, -SO₂H₂, -NHSO₂R¹⁰, -SO₂NHR¹⁰, -SO₂R¹⁰, -SOR¹⁰, -SR¹⁰, -NHSO₂, -NO₂, -CONR¹⁰, NCOR¹⁰ or -COOR¹⁰.

2. The compound of Claim 1 wherein the moiety -(CH₂)ₙ₋CO-Z is bonded at the 2-, 3- or 4-position on the piperdinyl ring.

3. The compound of Claim 2 wherein the moiety -(CH₂)ₙ-CO-Z is bonded at the 2- or 3- position on the piperdinyl ring.

4. The compound of Claim 1 wherein n is zero; Z is -NR⁴⁰R⁴² wherein R⁴⁰ represents H and R⁴² represents heteroarylalkyl.

5. The compound of Claim 4 wherein R⁴⁰ is H and R⁴² is 3-pyridylmethyl.

6. The compound of Claim 1 selected from any of the title compounds of Examples 1-54, a (Example numbers being given in parentheses):

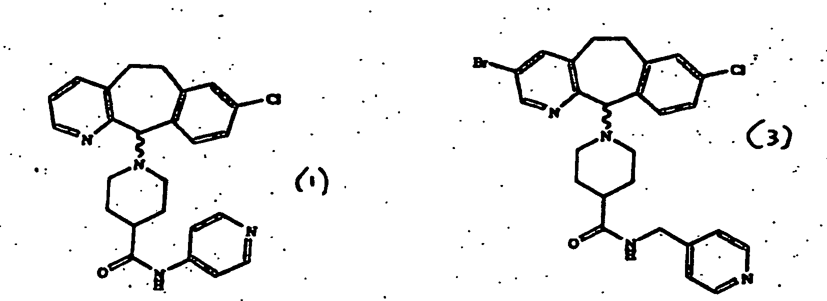

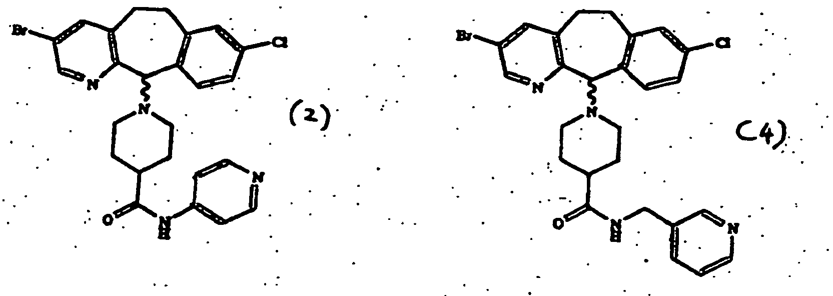

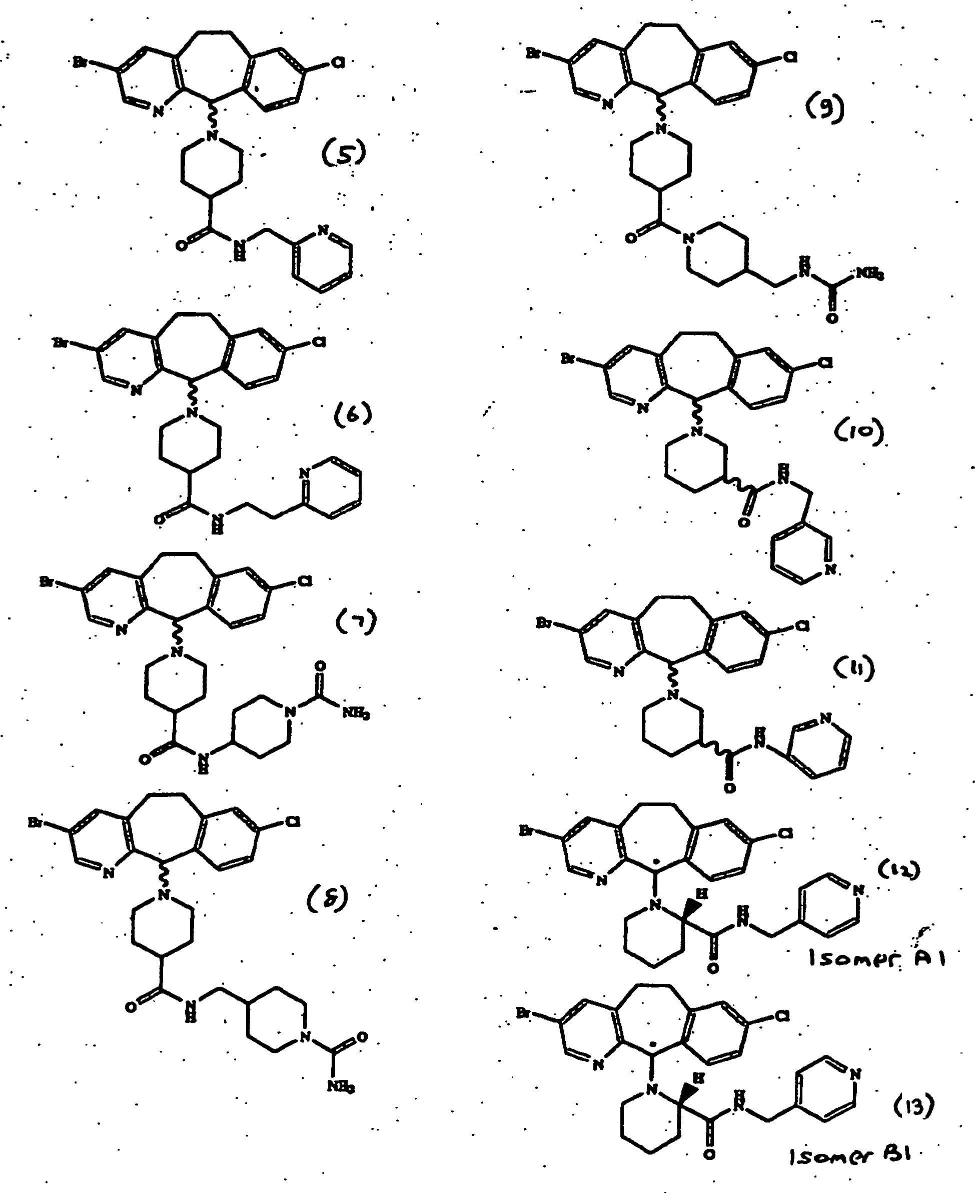

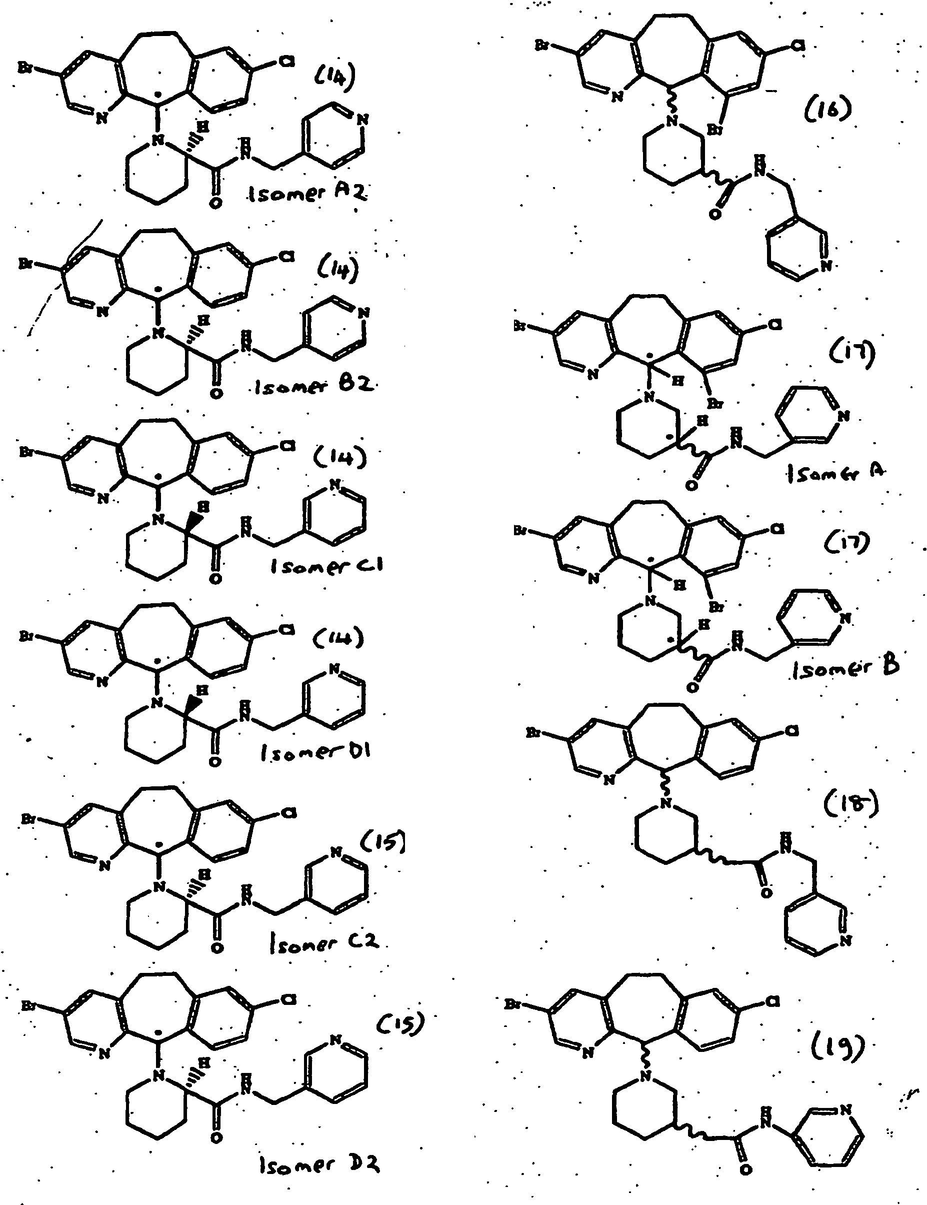

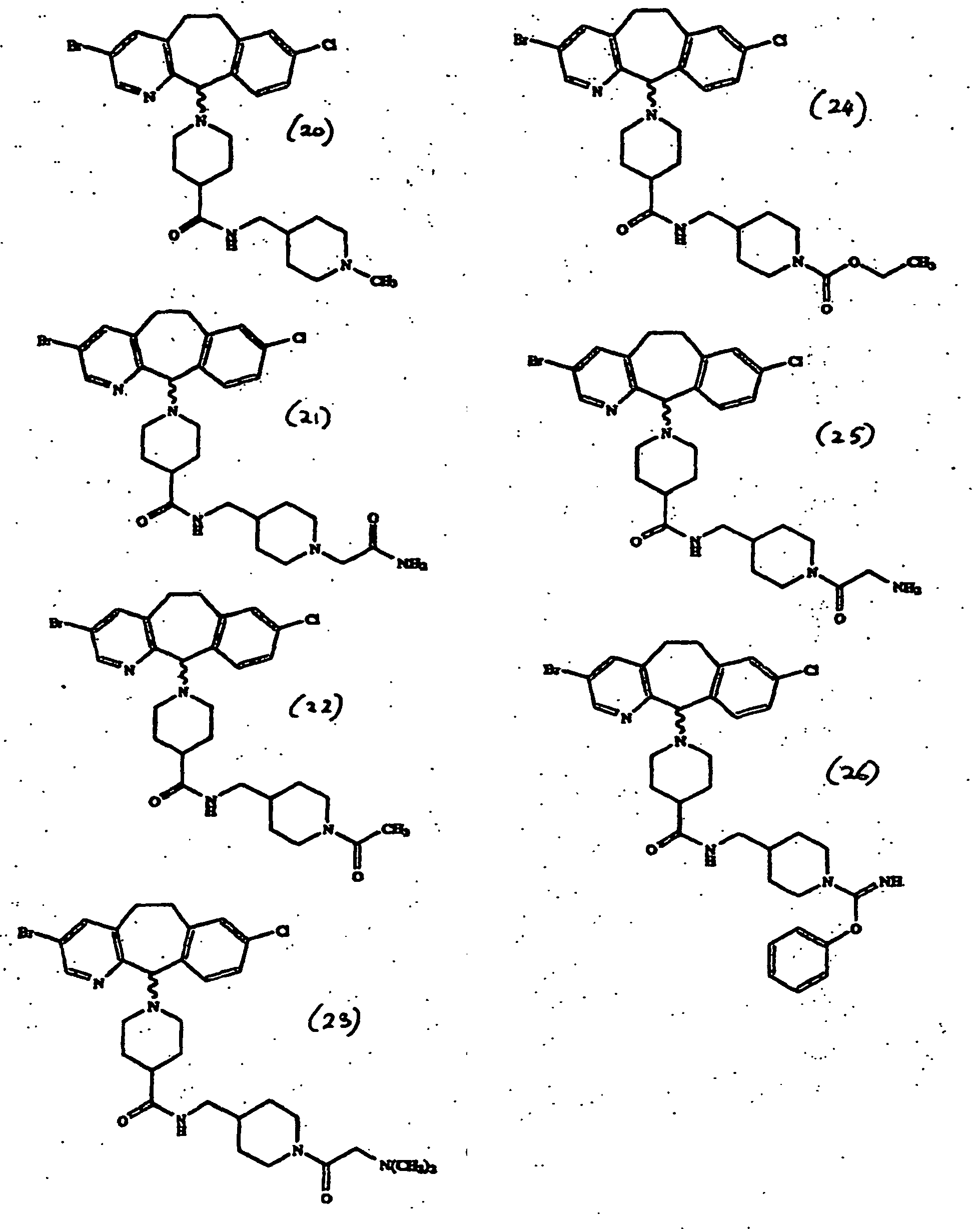

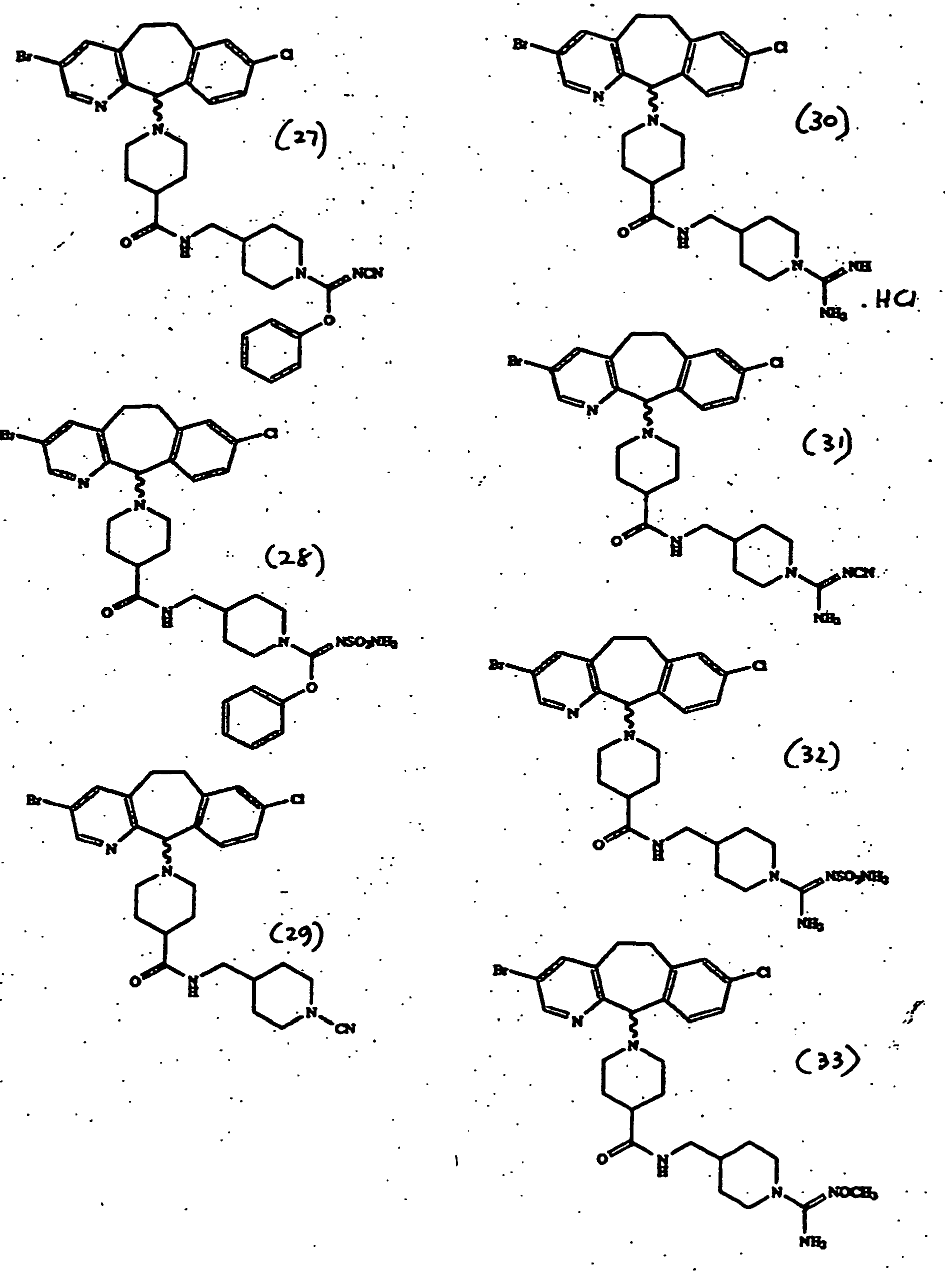

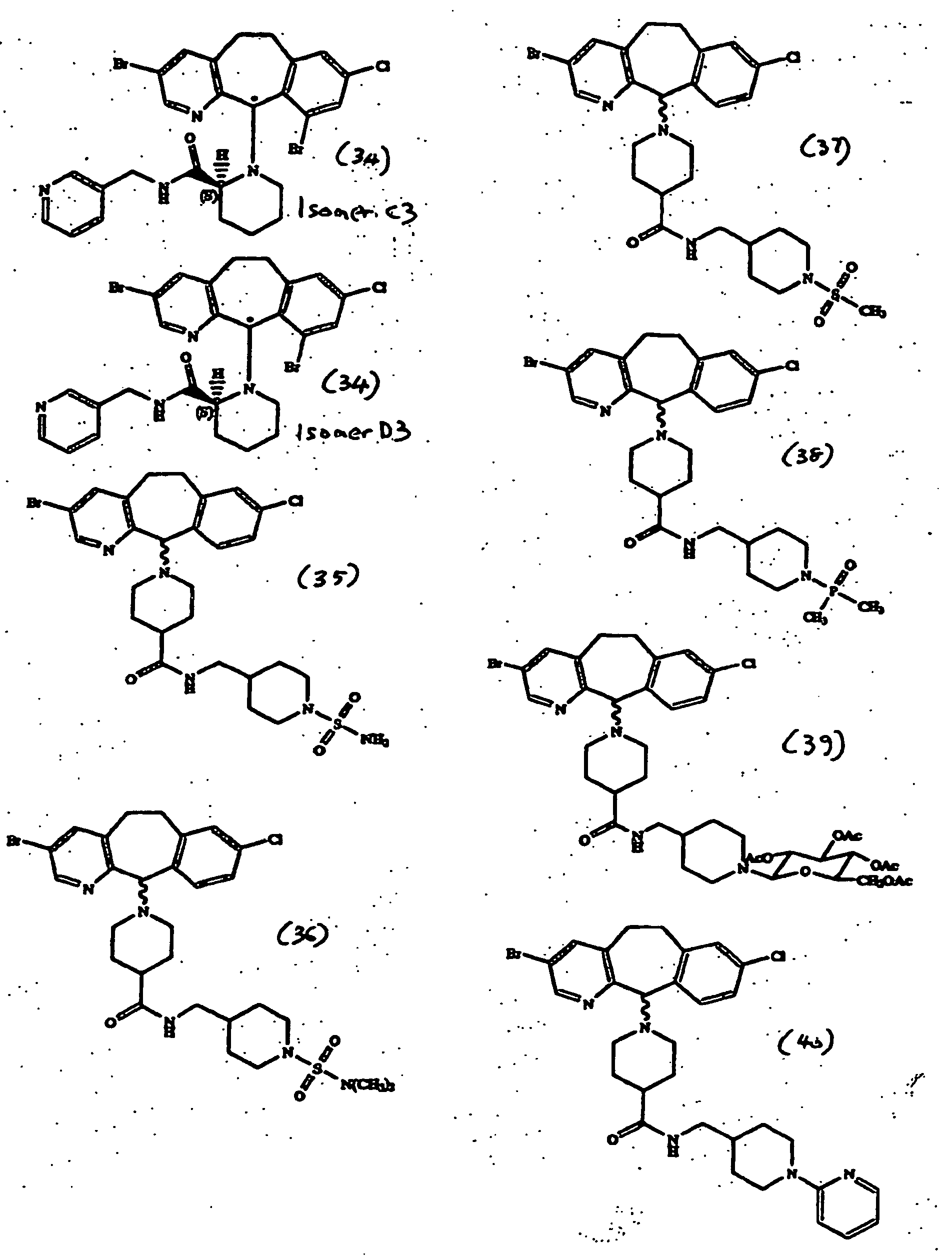

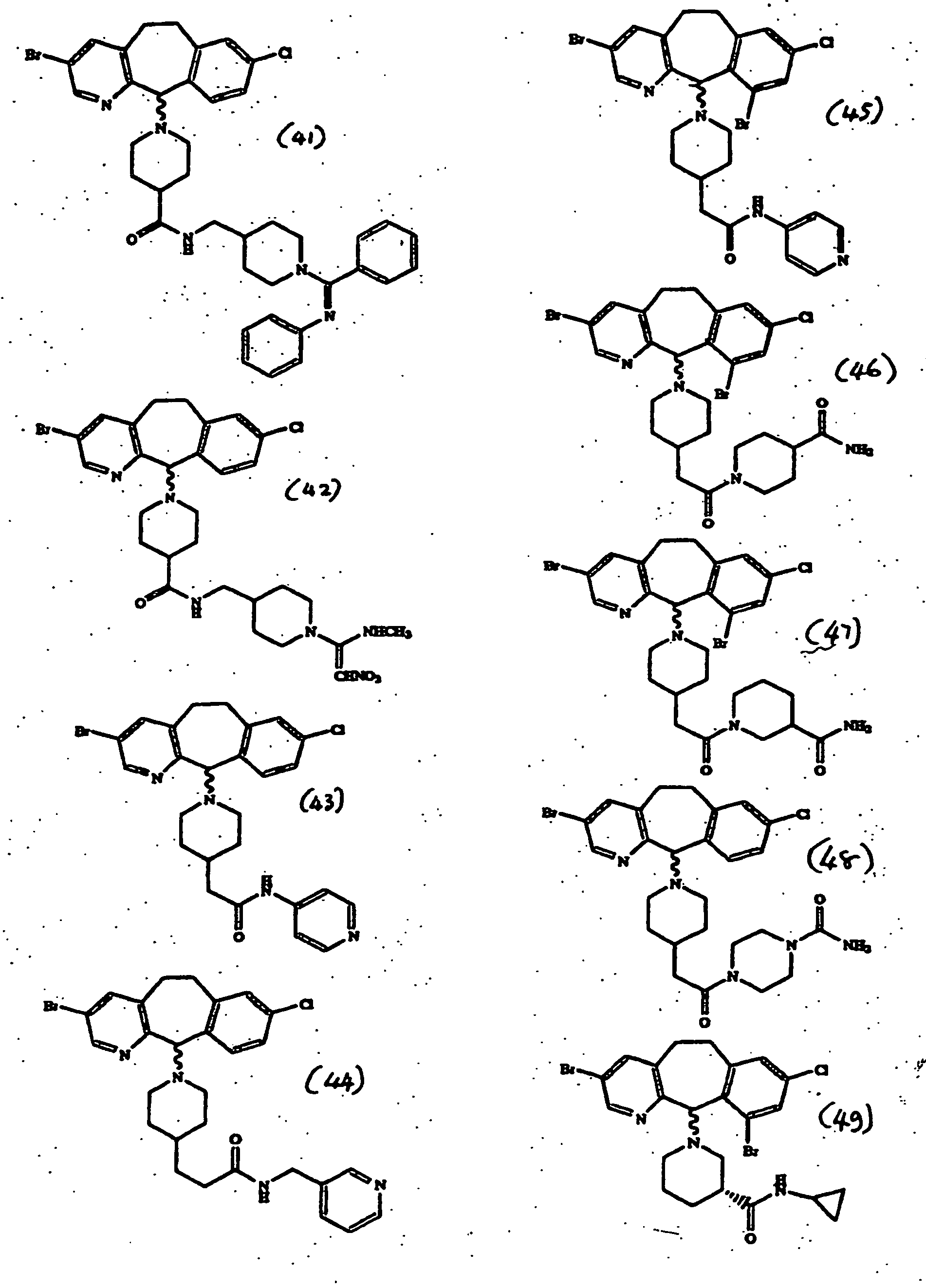

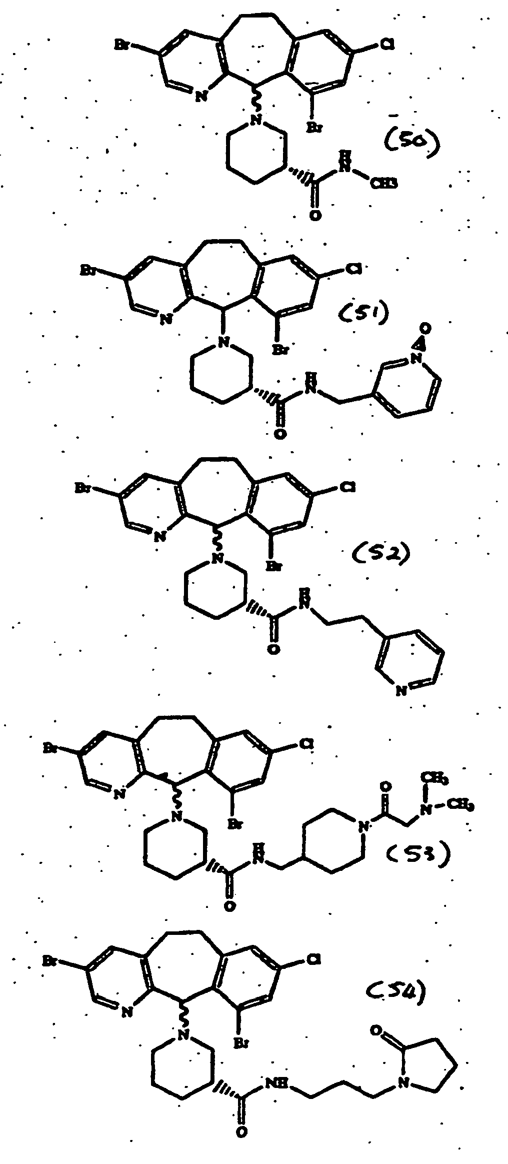

7. The compound of Claim 6 which is selected from 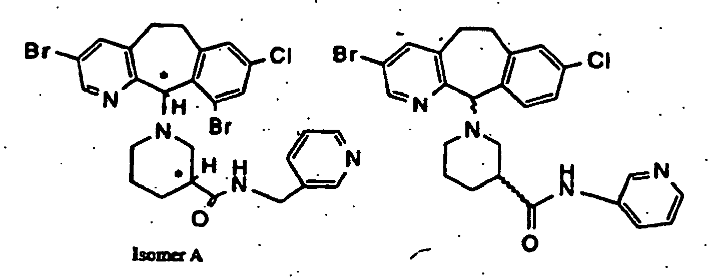 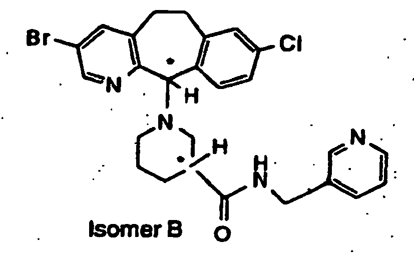 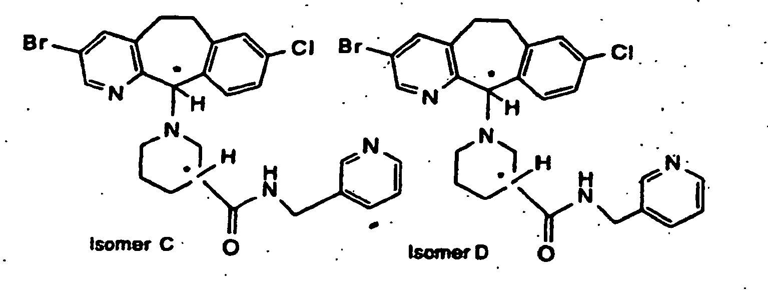 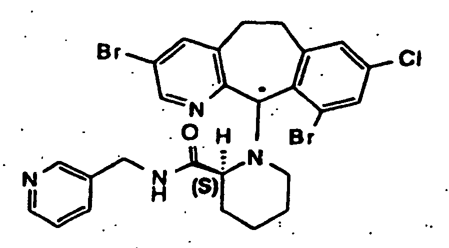 and Isomer D3 amide
or a pharmaceutically acceptable salt thereof.

8. The compound of Claim 1 which is

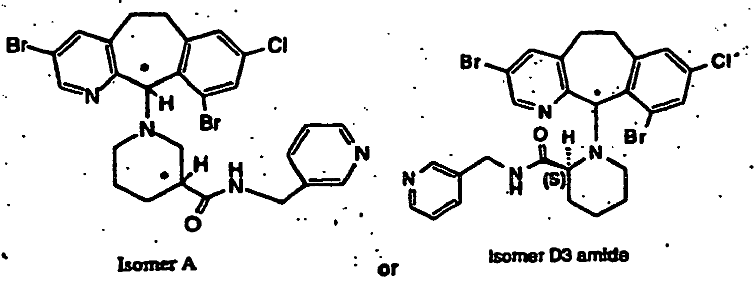

9. A composition for inhibiting the abnormal growth of cells comprising an effective amount of a compound of any preceding claim in combination with a pharmaceutically acceptable carrier.

10. A composition according to Claim 9 where in the cells inhibited are tumor cells expressing an activated ras oncogene.

11. A composition according to Claim 10 wherein the cells inhibited are pancreatic tumor cells, lung cancer cells, myeloid leukemia tumor cells, thyroid follicular tumor cells, myelodysplastic tumor cells, epidermal carcinoma tumor cells, bladder carcinoma tumor cells or colon tumors cells.

12. A composition according to Claim 9 wherein the inhibition of the abnormal growth of cells occurs by the inhibition of Ras farnesyl protein transferase.

13. A composition according to Claim 9 wherein the inhibition is of tumor cells wherein the Ras protein is activated as a result of oncogenic mutation in genes other than the Ras gene.

## Patentansprüche

1. Verbindung mit der Formel: 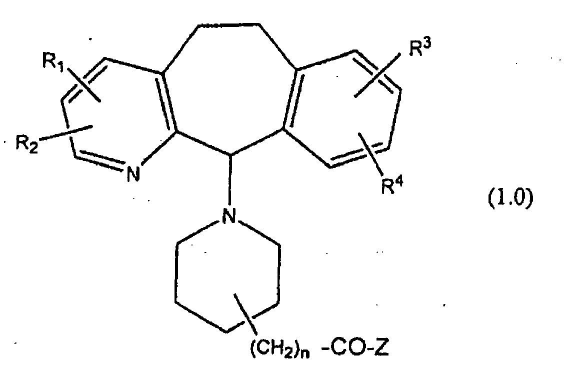 oder ein pharmazeutisch annehmbares Salz davon; worin
n 0, 1 oder 2 ist,
R¹ und R⁴ H sind, und R² und R³ Halogen ausgewählt aus Chlor oder Brom sind, oder
R¹ H ist, und R², R³ und R⁴ Halogen ausgewählt aus Chlor oder Brom sind,
Z -NR⁴⁰R⁴² ist, wobei R⁴⁰ und R⁴² unabhängig für H, Aryl, Alkyl, Aralkyl, Heteroaryl, Heteroarylalkyl, Heterocycloalkyl, Heterocycloalkylalkyl, Heteroalkyl, Cycloalkyl oder Cycloalkylalkyl stehen;
oder Z 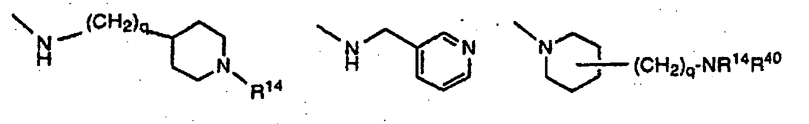 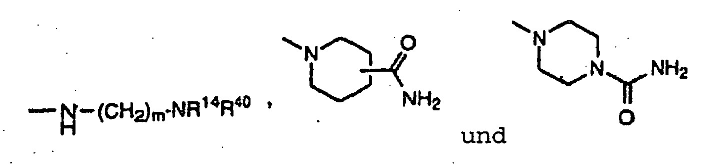 ist,
wobei R⁴⁰ wie zuvor definiert ist,
m ist 2, 3 oder 4;
q ist 0 (Null), 1 oder 2;
und R¹⁴ für H, C₁-C₆-Alkyl, Aralkyl, Heteroaryl, Acyl, Carboxamido, Carboxamidoalkyl, Cyano, Alkoxycarbonyl, Aralkyloxycarbonylimido, Imidamido, Sulfamoyl, Sulfonyl, Dialkylphosphinyl, N-Glycosyl oder
-C(NHCH₃)=CHNO₂, steht
und worin:
Alkyl - (einschließlich der Alkylanteile von Alkoxy, Alkylamino und Dialkylamino) für geradkettige und verzweigte Kohlenstoffketten steht und ein bis zwanzig Kohlenstoffatome enthält,
Alkenyl - für geradkettige und verzweigte Kohlenstoffketten mit mindestens einer Kohlenstoff-Kohlenstoff-Doppelbindung steht und 2 bis 12 Kohlenstoffatome enthält;
Alkinyl - für geradkettige und verzweigte Kohlenstoffketten mit mindestens einer Kohlenstoff-Kohlenstoff-Dreifachbindung steht und 2 bis 12 Kohlenstoffatome enthält;
Aryl - (einschließlich des Arylanteils von Aralkyl) für eine carbocyclische Gruppe steht, die 6 bis 15 Kohlenstoffatome enthält und mindestens einen aromatischen Ring aufweist;
Aralkyl - für eine Alkylgruppe wie oben definiert steht, worin ein oder mehrere Wasserstoffatome der Alkyleinheit durch eine oder mehrere Arylgruppen substituiert worden sind;
Cycloalkyl - für gesättigte carbocyclische Ringe steht, die verzweigt oder unverzweigt sind und 3 bis 20 Kohlenstoffatome aufweisen,
Cycloalkylalkyl - für eine Alkylgruppe wie oben definiert steht, worin ein oder mehrere Wasserstoffatome der Alkyleinheit durch eine oder mehrere Cycloalkylgruppen substituiert worden sind;
Heteroalkyl - für geradkettige und verzweigte Kohlenstoffketten steht, die ein bis zwanzig Kohlenstoffatome enthalten und durch 1 bis 3 Heteroatome ausgewählt aus -O-, -S- und -N- unterbrochen sind;
Heteroaryl - für cyclische Gruppen mit mindestens einem Heteroatom ausgewählt aus O, S und N steht, wobei das Heteroatom/die Heteroatome eine carbocyclische Ringstruktur unterbricht/unterbrechen und eine ausreichende Anzahl delokalisierter n-Elektronen aufweisen, um aromatischen Charakter zu liefern, wobei die aromatischen heterocyclischen Gruppen 2 bis 14 Kohlenstoffatome enthalten, wobei die Heteroarylgruppe gegebenenfalls mit einem oder mehreren Aryl-, Cycloalkyl-, Heteroaryl- oder Heterocycloalkylringen kondensiert sein kann;
Heterocycloalkyl - für einen gesättigten, verzweigten oder nicht-verzweigten carbocyclischen Ring steht, der 3 bis 15 Kohlenstoffatome enthält, wobei der carbocyclische Ring durch 1 bis 3 Heteroatome ausgewählt aus -O-, -S- und -N- unterbrochen ist, wobei der Ring gegebenenfalls ein oder zwei ungesättigte Bindungen enthalten kann, die dem Ring keinen aromatischen Charakter verleihen;
wobei die Alkyl-, Alkenyl-, Alkylaryl-, Cycloalkyl-, Cycloalkylaryl-, Aryl-, Aralkyl-, Heteroaryl-, Heteroalkyl- und Heterocycloalkylgruppen gegebenenfalls und unabhängig mit einem, zwei, drei oder mehr der Folgenden substituiert sein können: Halogen, Alkyl, Aryl, Alkoxy, Amino, Alkylamino, Cyano, -CF₃, Dialkylamino, Hydroxy, Oxy, Phenoxy, -OCF₃, Heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ oder -COOR¹⁰.

2. Verbindung nach Anspruch 1, wobei die Einheit -(CH₂)ₙ-CO-Z an die 2-, 3- oder 4-Position an dem Piperidinylring gebunden ist.

3. Verbindung nach Anspruch 2, wobei die Einheit -(CH₂)ₙ-CO-Z an die 2- oder 3-Position an dem Piperidinylring gebunden ist.

4. Verbindung nach Anspruch 1, wobei n Null ist; Z -NR⁴⁰R⁴² ist, wobei R⁴⁰ für H steht und R⁴² für Heteroarylalkyl steht.

5. Verbindung nach Anspruch 4, wobei R⁴⁰ H ist und R⁴² 3-Pyridylmethyl ist.

6. Verbindung nach Anspruch 1 ausgewählt aus einer der Titelverbindungen der Beispiele 1 bis 54 (Beispielnummern sind in Klammern angegeben):

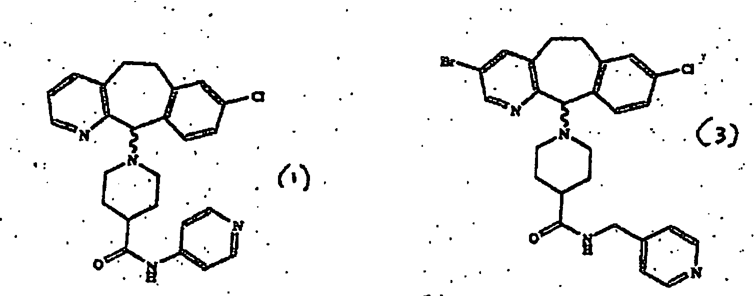

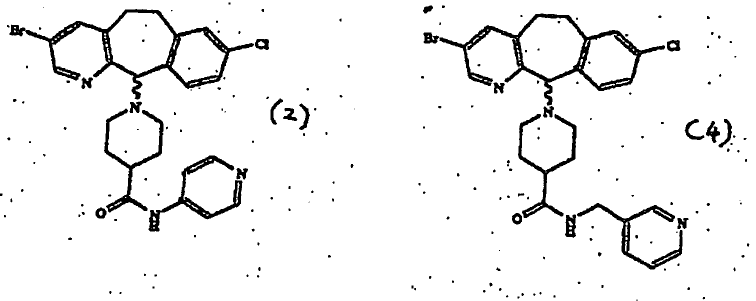

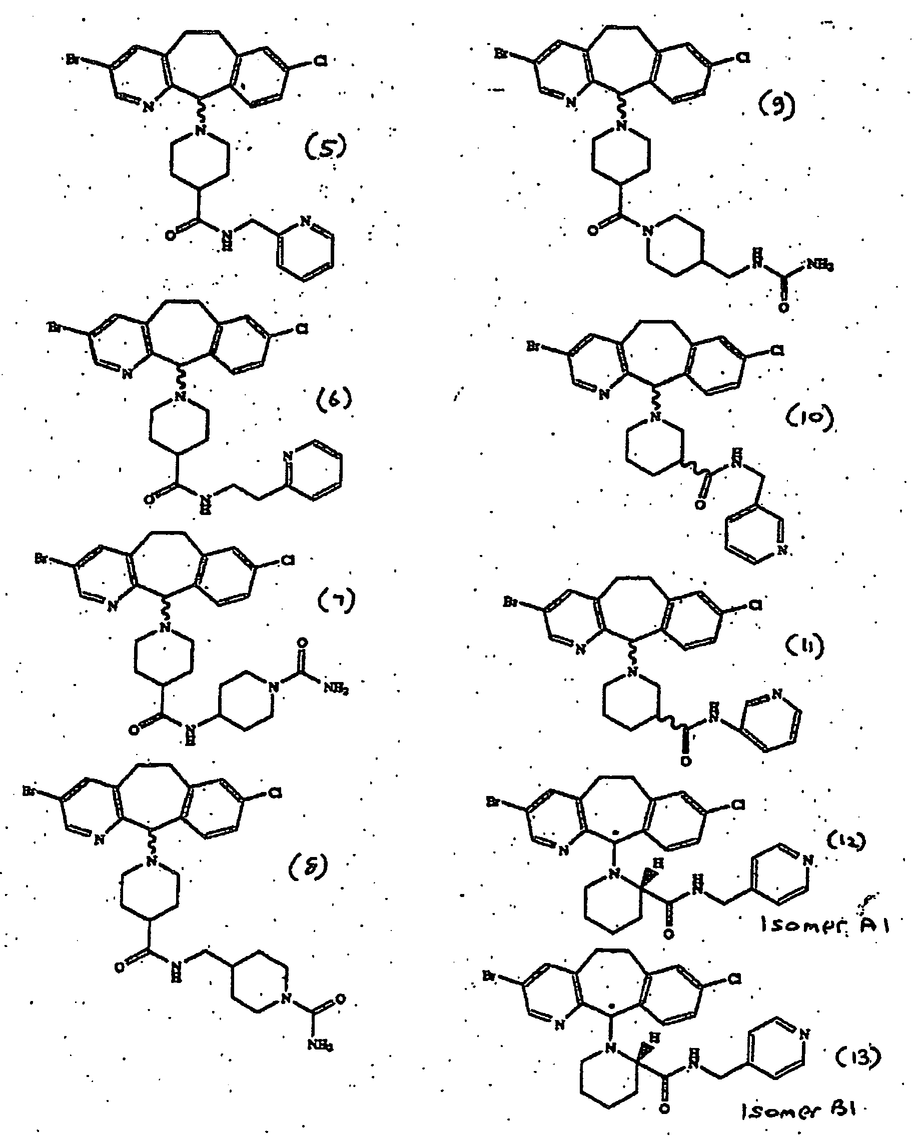

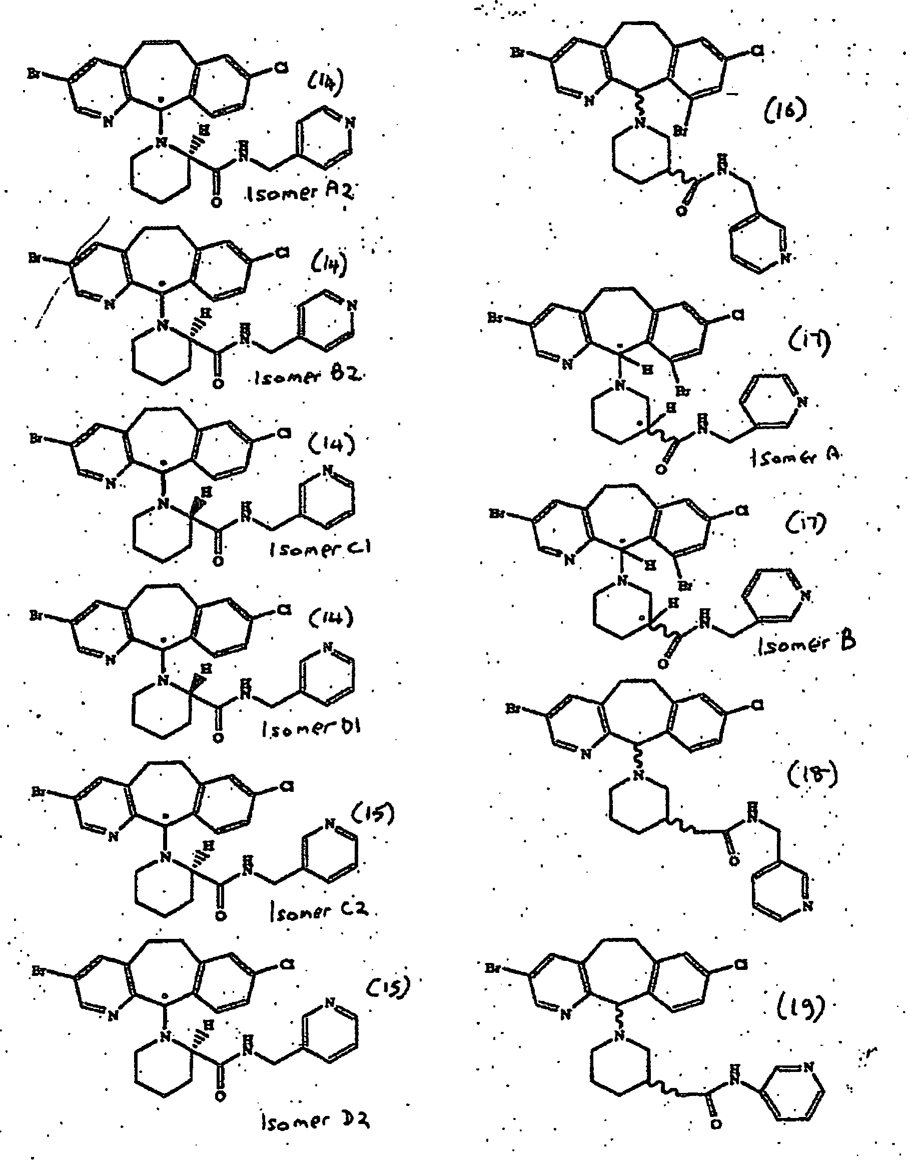

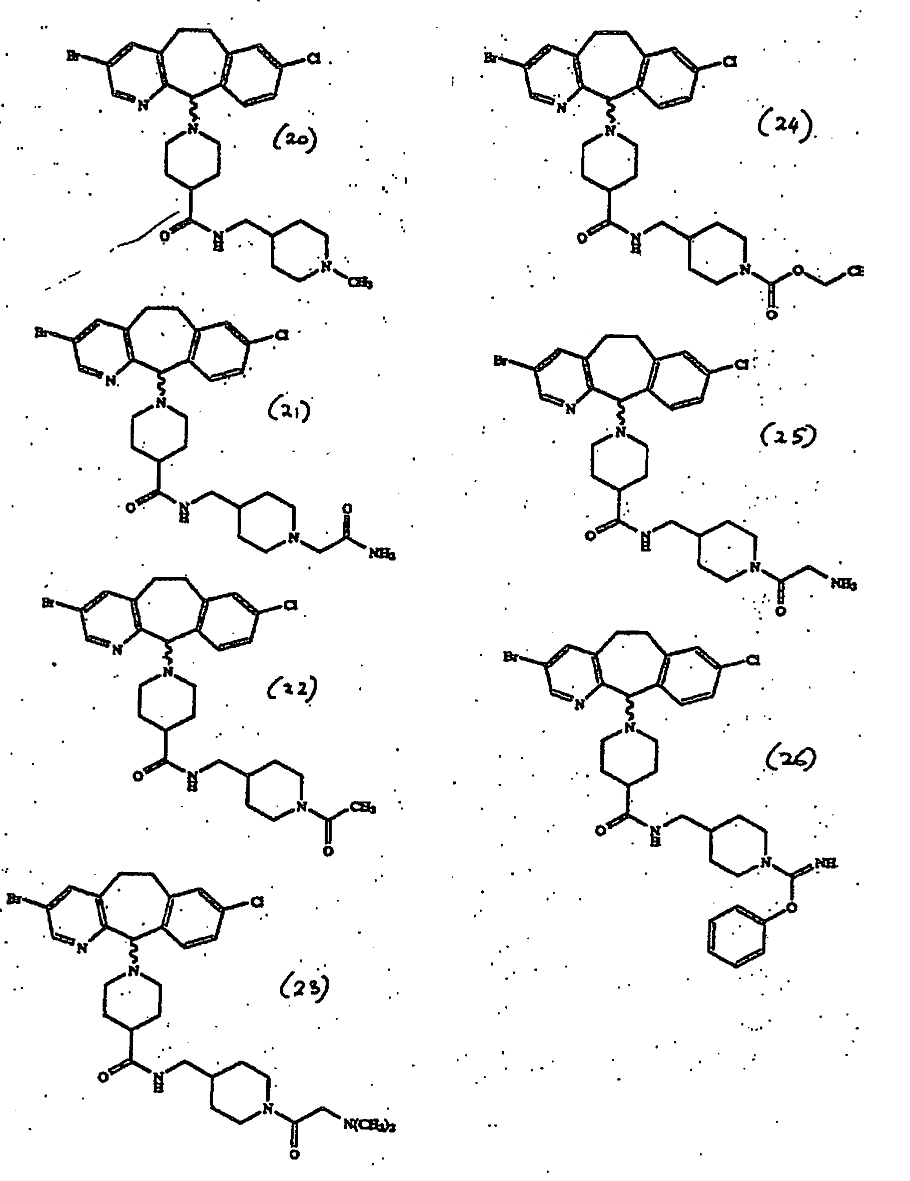

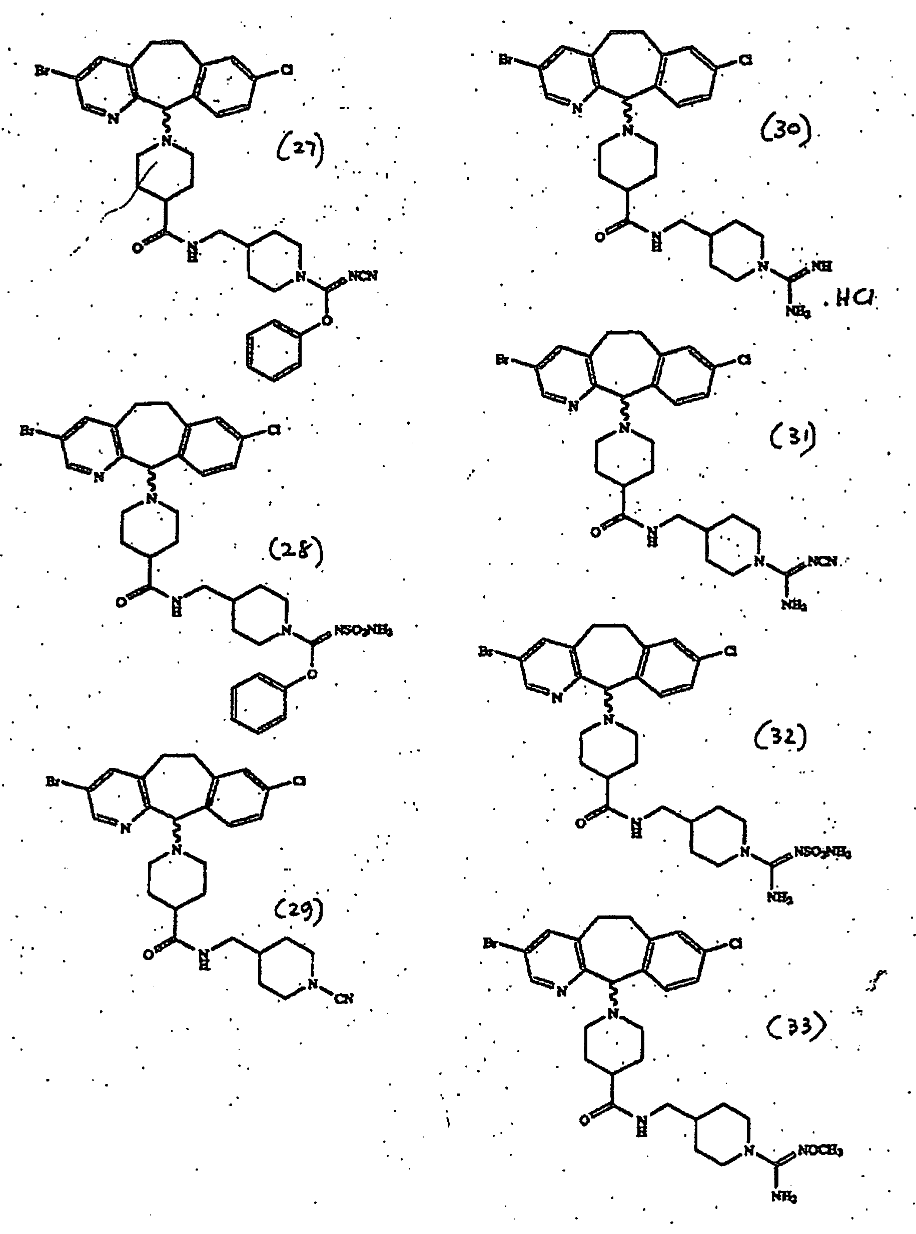

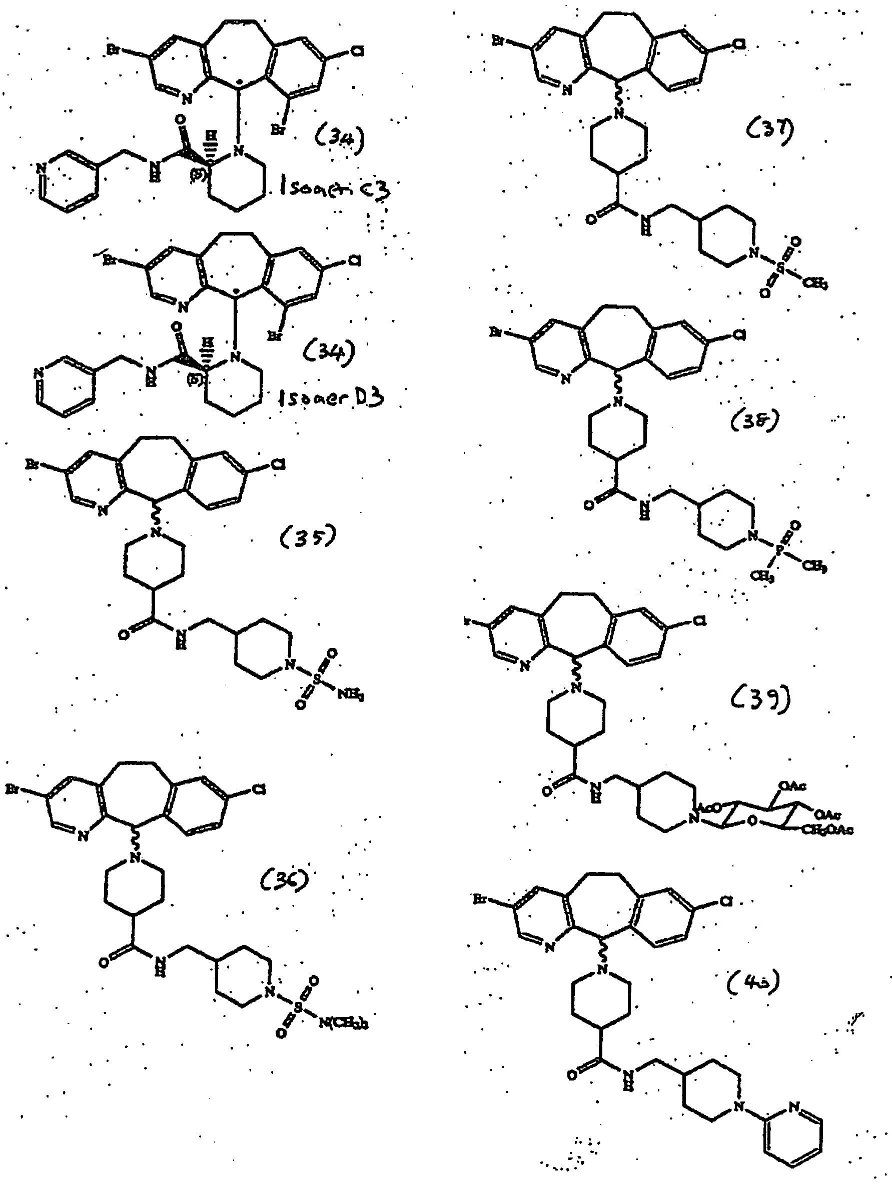

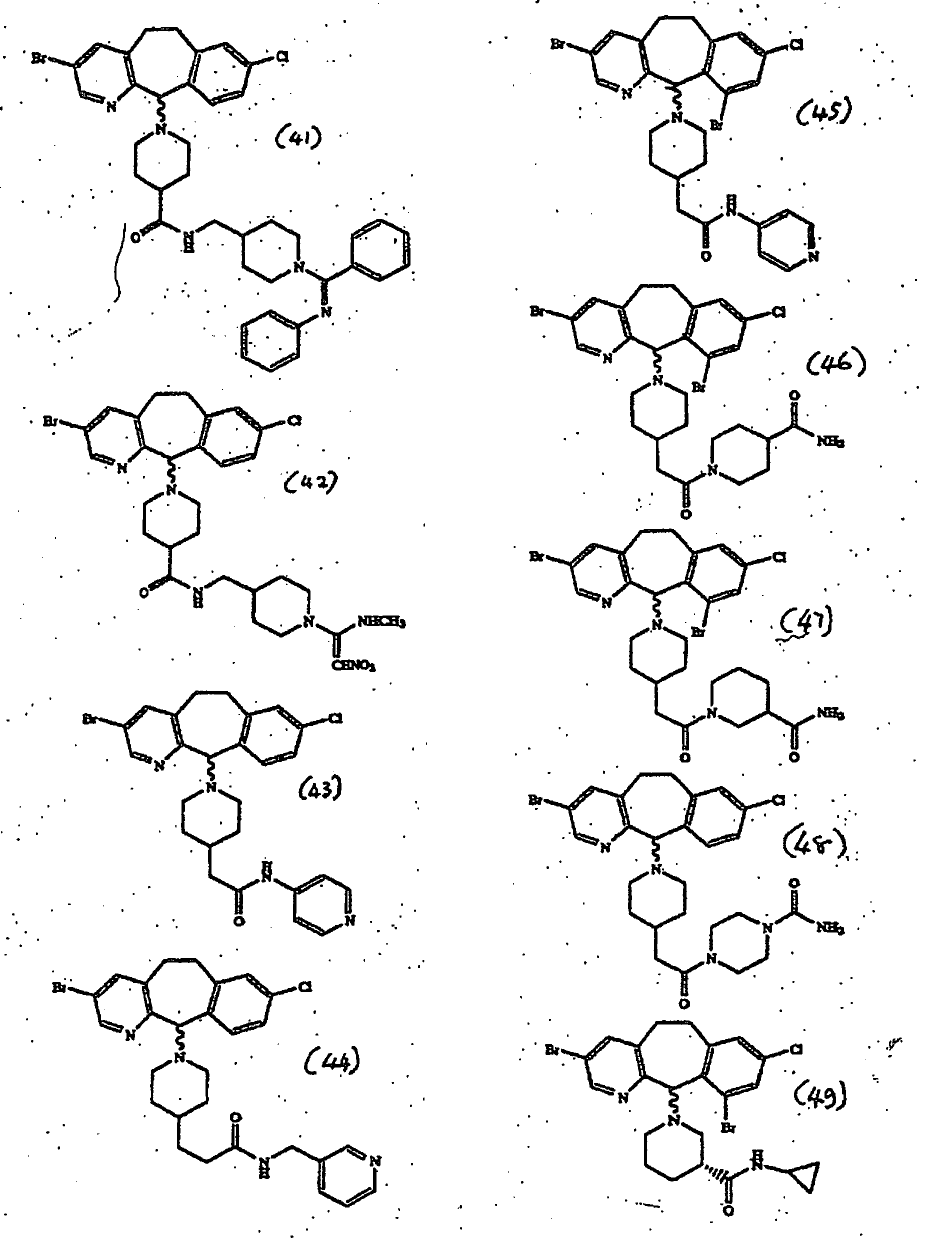

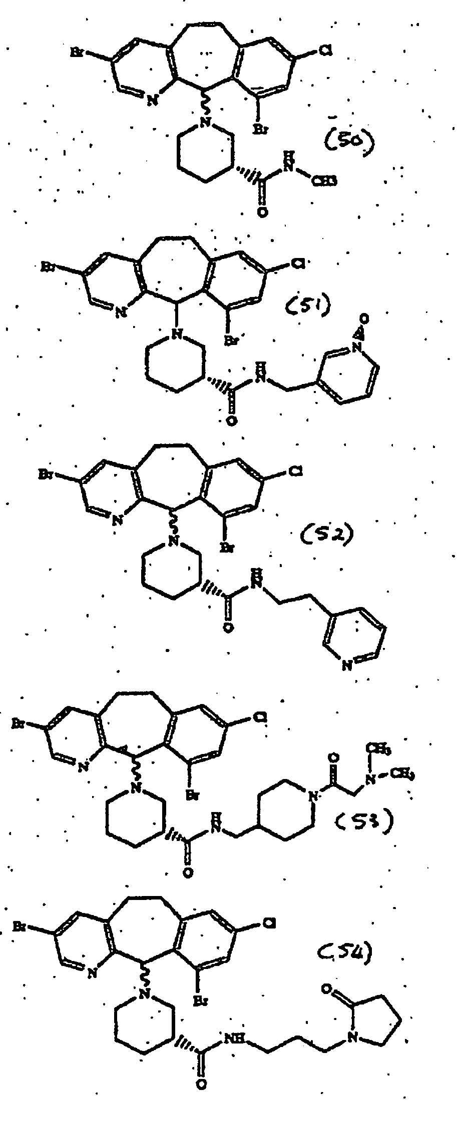

7. Verbindung nach Anspruch 6, die ausgewählt ist aus: 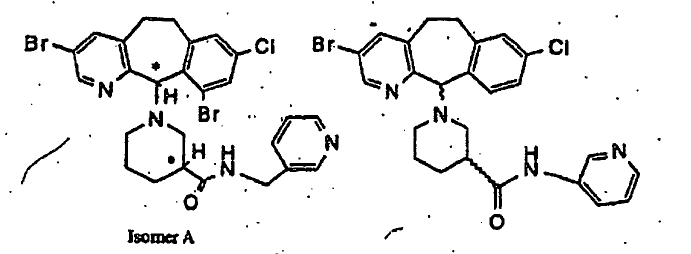 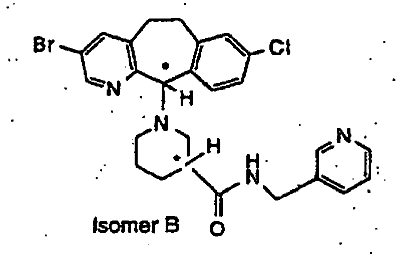 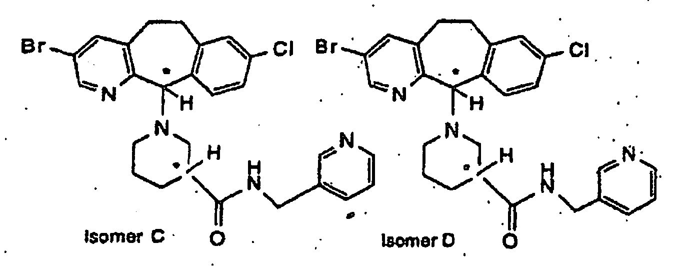 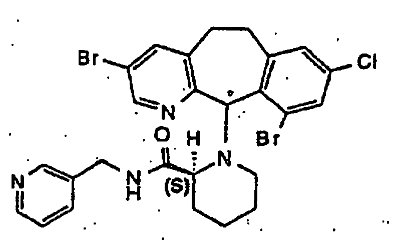 und Isomer-D3-amid
oder ein pharmazeutisch annehmbares Salz davon.

8. Verbindung nach Anspruch 1, die

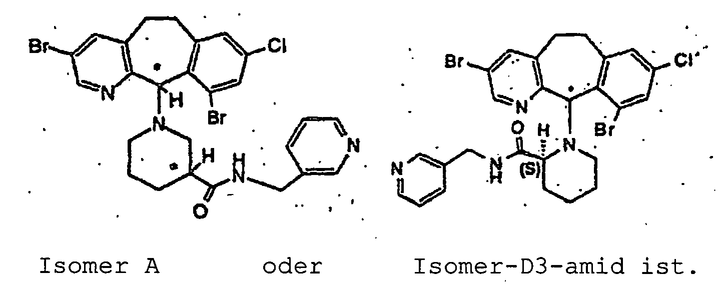

9. Zusammensetzung zum Inhibieren des abnormalen Wachstums von Zellen, die eine wirksame Menge einer Verbindung nach einem der vorhergehenden Ansprüche in Kombination mit einem pharmazeutisch annehmbaren Träger enthält.

10. Zusammensetzung nach Anspruch 9, wobei die inhibierten Zellen Tumorzellen sind, die ein aktiviertes ras-Onkogen exprimieren.

11. Zusammensetzung nach Anspruch 10, wobei die inhibierten Zellen Pankreastumorzellen, Lungenkrebszellen, myeloide Leukämietumorzellen, Schilddrüsenfollikeltumorzellen, myelodysplastische Tumorzellen, epidermale Carcinomtumorzellen, Blasencarcinomtumorzellen oder Colontumorzellen sind.

12. Zusammensetzung nach Anspruch 9, wobei die Inhibierung des abnormalen Wachstums von Zellen durch Inhibierung von ras-Farnesylproteintransferase erfolgt.

13. Zusammensetzung nach Anspruch 9, wobei die Inhibierung eine von Tumorzellen ist, bei denen das Ras-Protein infolge von onkogener Mutation in anderen Genen als dem Ras-Gen aktiviert ist.

## Revendications

1. Composé de formule 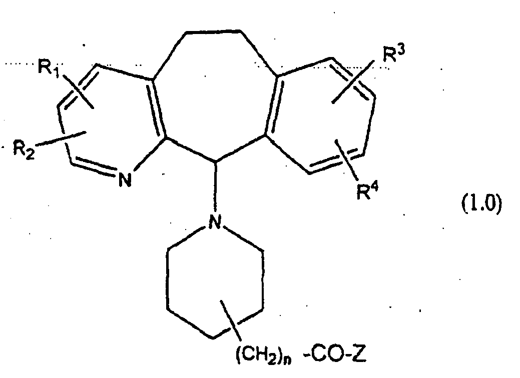 ou sel pharmacologiquement admissible d'un tel composé, dans laquelle formule :
- n vaut 0, 1 ou 2 ;
- R¹ et R⁴ représentent des atomes d'hydrogène et R² et R³ représentent des atomes d'halogène choisis parmi les atomes de chlore et de brome, ou bien R¹ représente un atome d'hydrogène et R², R³ et R⁴ représentent des atomes d'halogène choisis parmi les atomes de chlore et de brome ;
- et Z représente un groupe de formule -NR⁴⁰R⁴², où R⁴⁰ et R⁴² représentent chacun, indépendamment, un atome d'hydrogène ou un groupe aryle, alkyle, aralkyle, hétéroaryle, hétéroaryl-alkyle, hétérocycloalkyle, hétérocycloalkyl-alkyle, hétéroalkyle, cycloalkyle ou cycloalkyl-alkyle, ou bien Z représente un groupe de l'une des formules suivantes : 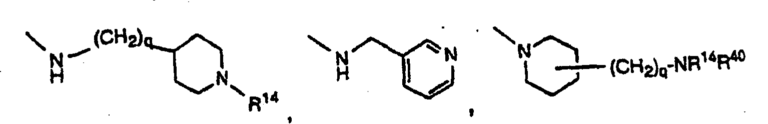 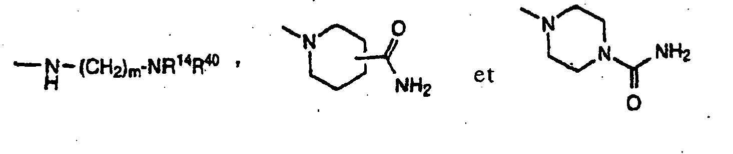 dans lesquelles
- R⁴⁰ a la signification indiquée ci-dessus,
- m vaut 2, 3 ou 4,
- q vaut 0 (zéro), 1 ou 2,
- et R¹⁴ représente un atome d'hydrogène ou un groupe alkyle en C₁₋₆, aralkyle, hétéroaryle, acyle, carboxamido, carboxamido-alkyle, cyano, alcoxycarbonyle, aralcoxycarbonyle, imido, imidamido, sulfamyle, sulfonyle, dialkylphosphinyle ou N-glycosyle, ou encore un groupe de formule -C(NHCH₃)=CHNO₂ ;
étant entendu que :
un groupe "alkyle", y compris les fragments alkyle des groupes alcoxy, alkylamino et dialkylamino, est constitué par une chaîne linéaire ou ramifiée d'atomes de carbone et comporte 1 à 20 atomes de carbone ;
un groupe "alcényle" est constitué par une chaîne linéaire ou ramifiée d'atomes de carbone contenant au moins une double liaison carbone-carbone et comporte 2 à 12 atomes de carbone ;
un groupe "alcynyle" est constitué par une chaîne linéaire ou ramifiée d'atomes de carbone contenant au moins une triple liaison carbone-carbone et comporte 2 à 12 atomes de carbone ;
un groupe "aryle", y compris le fragment aryle d'un groupe aralkyle, est un groupe carbocyclique qui comporte 6 à 15 atomes de carbone et au moins un cycle aromatique ;
un groupe "aralkyle" est un groupe alkyle, tel qu'il est défini ci-dessus, dont un ou plusieurs atomes d'hydrogène sont remplacés par un ou plusieurs groupes aryle ;
un groupe "cycloalkyle" est un groupe carbocyclique saturé, ramifié ou non ramifié, qui comporte de 3 à 20 atomes de carbone ;
un groupe "cyaloalkyl-alkyle" est un groupe alkyle, tel qu'il est défini ci-dessus, dont un ou plusieurs atomes d'hydrogène sont remplacés par un ou plusieurs groupes cycloalkyle ;
un groupe "hétéroalkyle" est constitué par une chaîne linéaire ou ramifiée d'atomes de carbone qui comporte 1 à 20 atomes de carbone et où sont intercalés 1 à 3 hétéroatomes choisis parmi les atomes d'oxygène, de soufre ou d'azote ;
un groupe "hétéroaryle" est un groupe cyclique comportant au moins un hétéroatome choisi parmi les atomes d'oxygène, de soufre ou d'azote, lequel ou lesquels hétéroatomes sont intercalés dans une structure carbocyclique et apportent à celle-ci suffisamment d'électrons π délocalisés pour qu'elle conserve le caractère aromatique, ces groupes hétérocycliques aromatiques comportant 2 à 14 atomes de carbone, et un tel groupe hétéroaryle pouvant en option être condensé avec un ou plusieurs cycles aryle, cycloalkyle, hétéroaryle ou hétérocycloalkyle ;
un groupe "hétérocycloalkyle" est constitué par un carbocycle saturé, ramifié ou non ramifié, qui comporte 3 à 15 atomes de carbone et où sont intercalés 1 à 3 hétéroatomes choisis parmi les atomes d'oxygène, de soufre ou d'azote, un tel cycle pouvant en option comporter 1 ou 2 liaisons insaturées qui ne lui confèrent pas un caractère aromatique ;
lesquels groupes alkyle, alcényle, alkylaryle, cycloalkyle, cycloalkylaryle, aryle, aralkyle, hétéroaryle, hétéroalkyle et hétérocycloalkyle peuvent, en option et indépendamment, porter un, deux ou trois substituants ou plus, choisis parmi les suivants : halogéno, alkyle, aryle, alcoxy, amino, alkylamino, cyano, -CF₃, dialkylamino, hydroxy, oxo, phénoxy, -OCF₃, hétérocycloalkyle, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ et -COOR¹⁰.

2. Composé conforme à la revendication 1, dans lequel le fragment représenté par -(CH₂)ₙ-CO-Z est attaché en position 2, 3 ou 4 du cycle pipéridinyle.

3. Composé conforme à la revendication 2, dans lequel le fragment représenté par -(CH₂)ₙ-CO-Z est attaché en position 2 ou 3 du cycle pipéridinyle.

4. Composé conforme à la revendication 1 dans lequel n vaut 0 et Z représente un groupe de formule -NR⁴⁰R⁴² où R⁴⁰ représente un atome d'hydrogène et R⁴² représente un groupe hétéroaryl-alkyle.

5. Composé conforme à la revendication 4, dans lequel R⁴⁰ représente un atome d'hydrogène et R⁴² représente un groupe 3-pyridyl-méthyle.

6. Composé conforme à la revendication 1, choisi parmi tous les composés des titres des exemples 1 à 54 (les numéros des exemples sont indiqués entre parenthèses) :

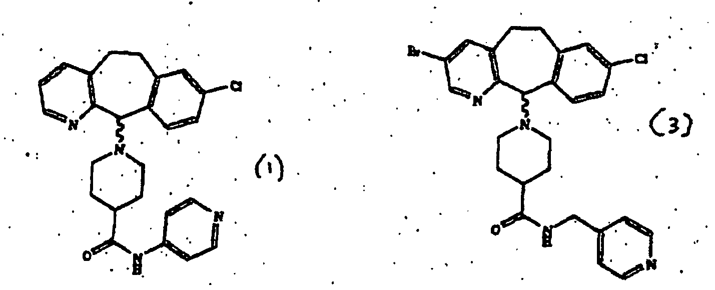

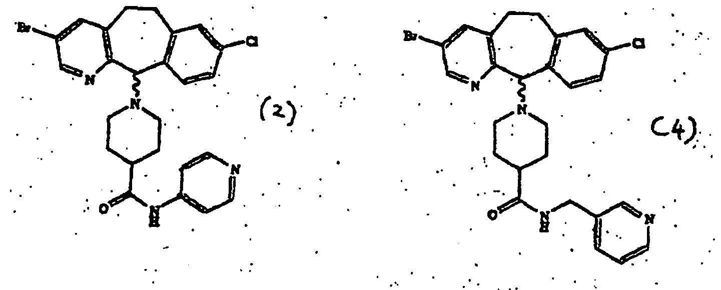

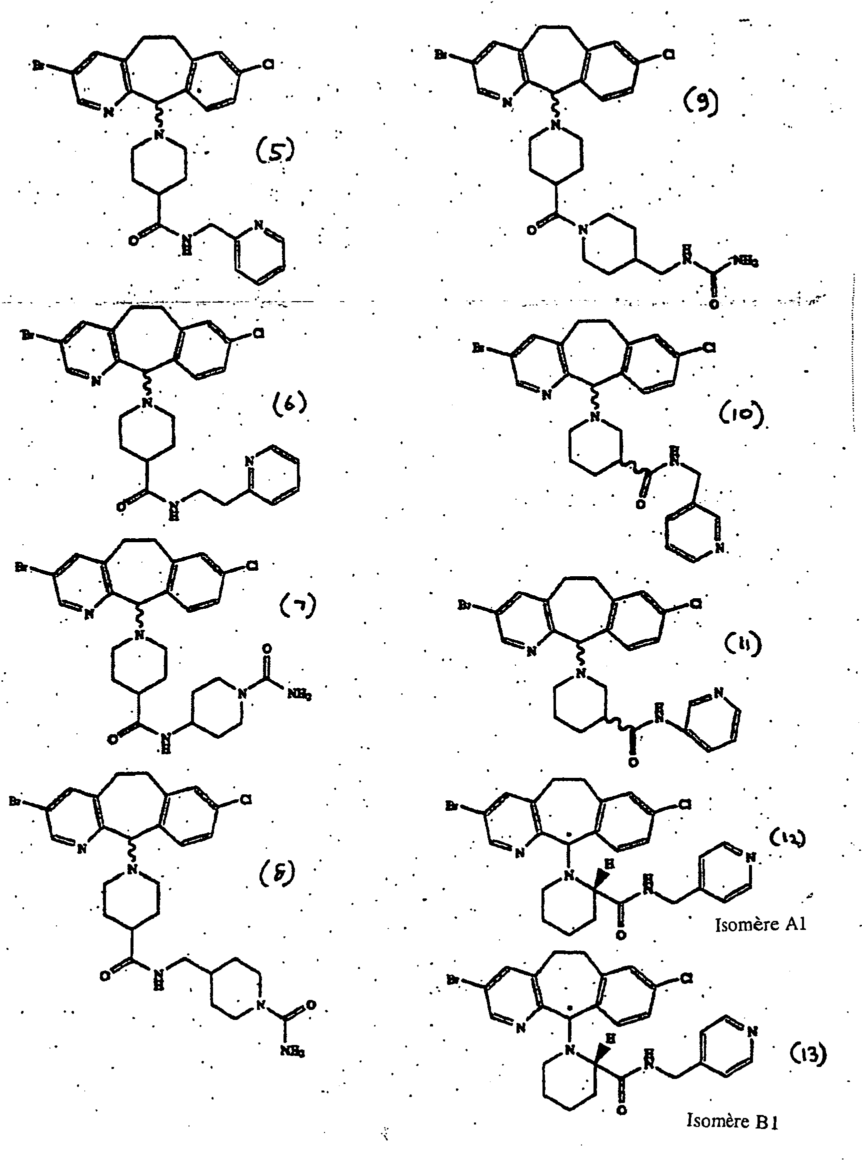

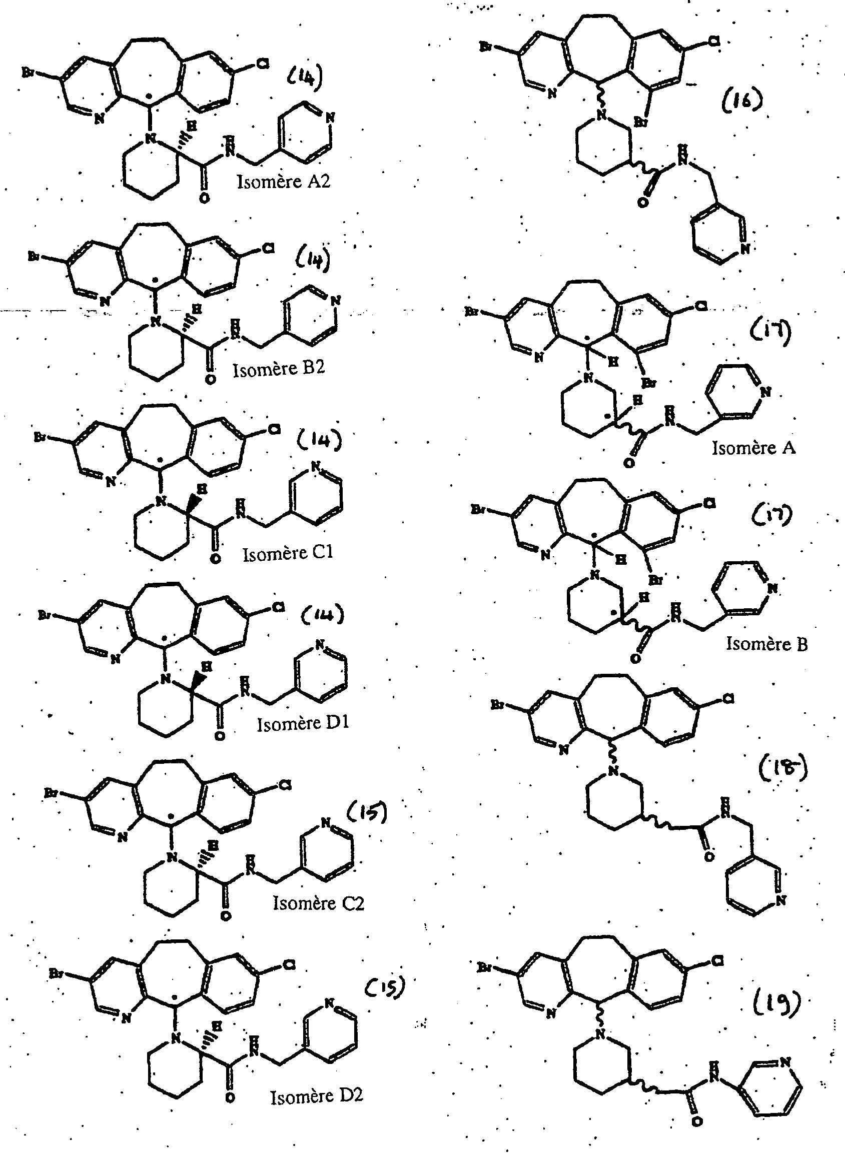

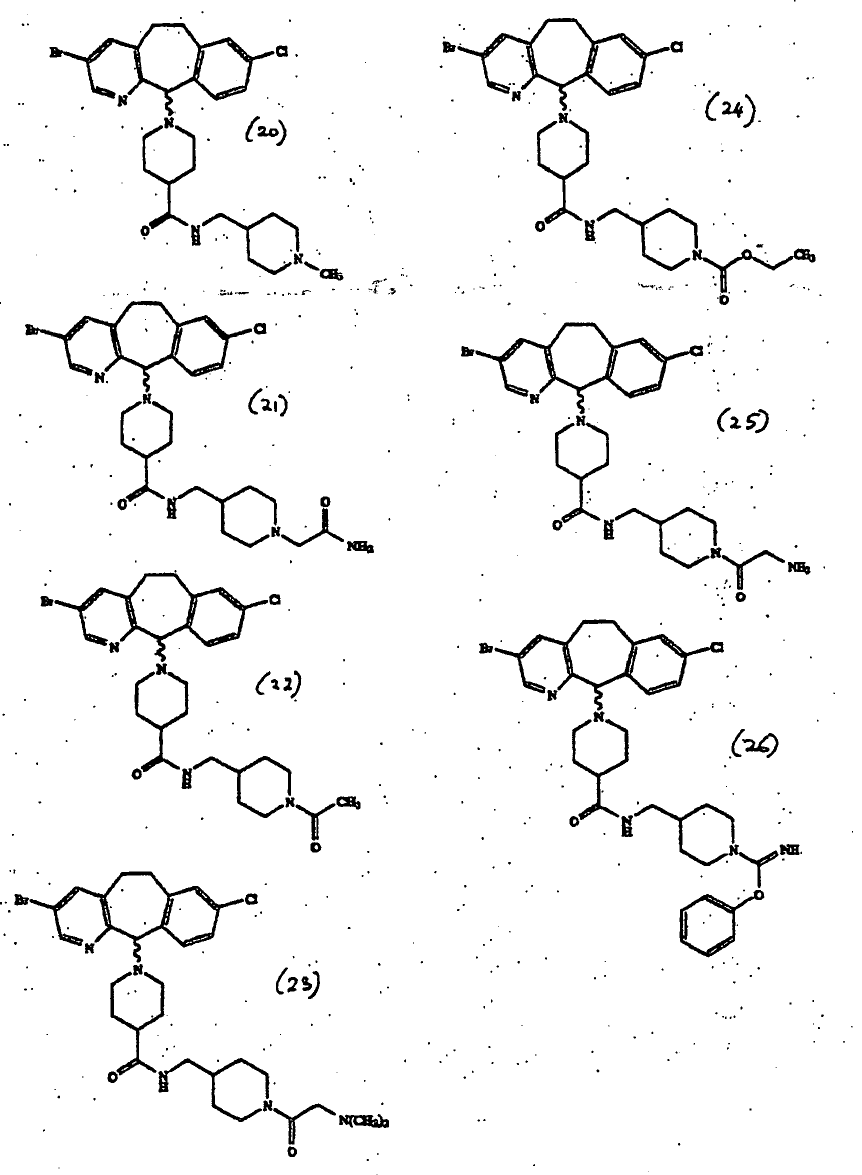

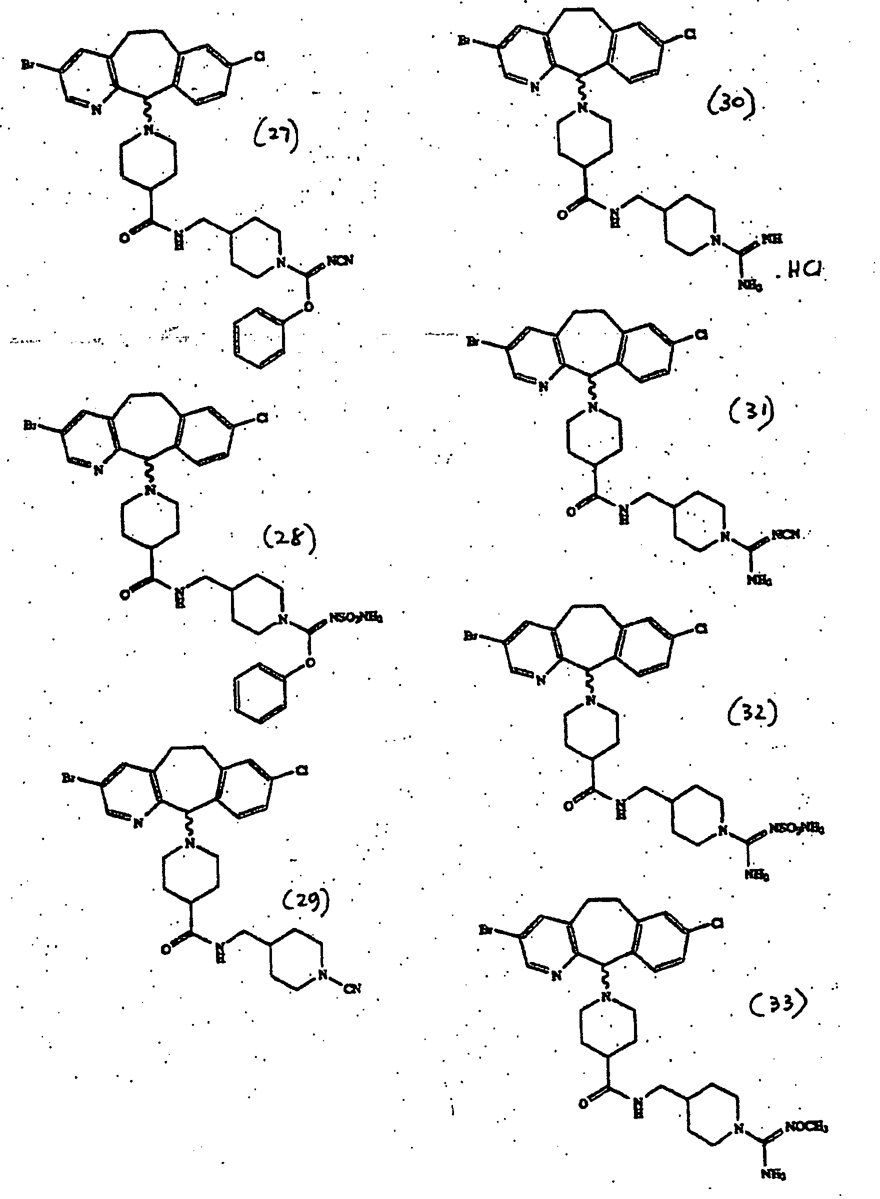

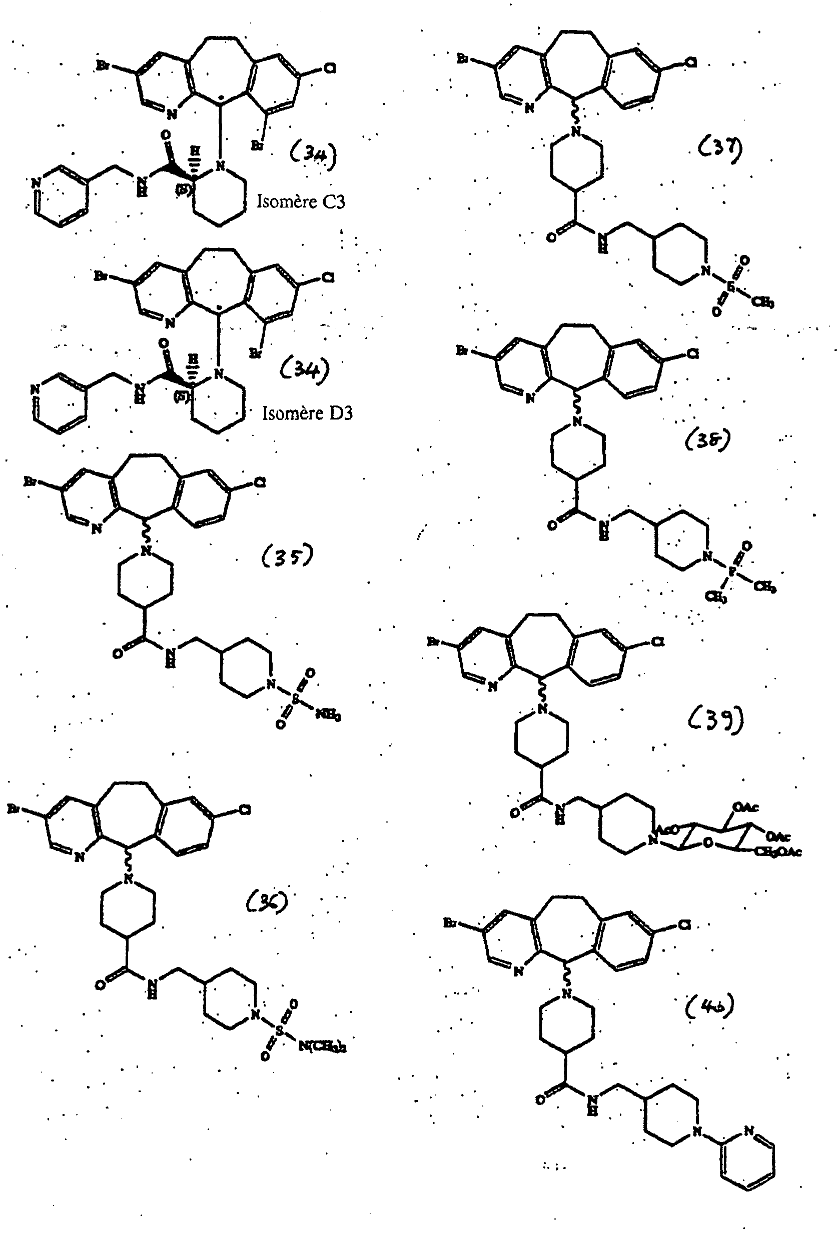

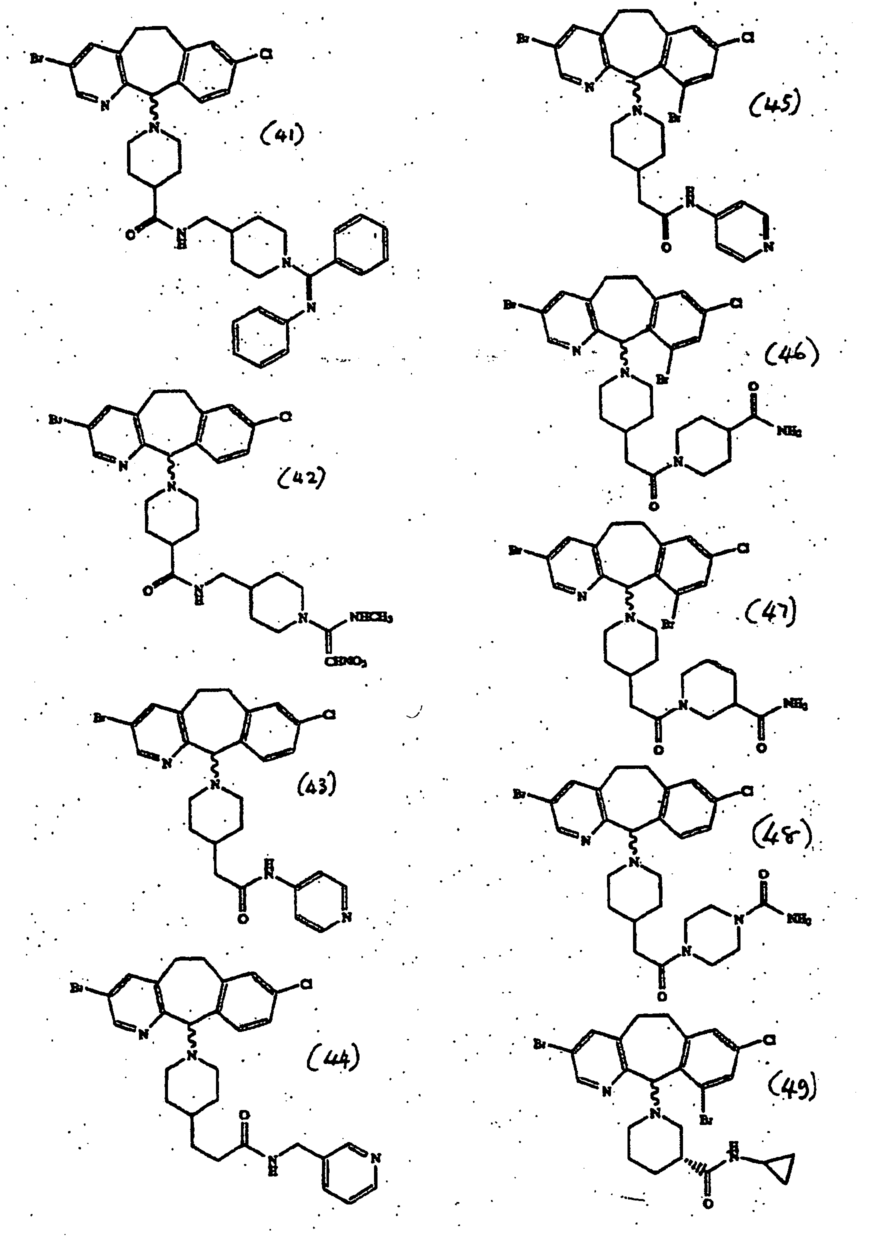

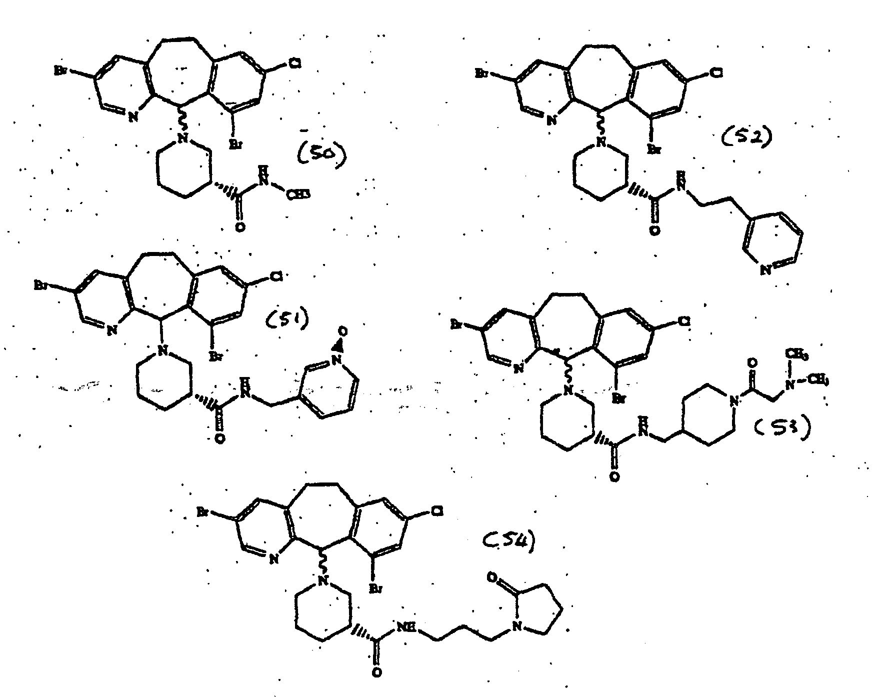

7. Composé conforme à la revendication 6, choisi parmi les suivants : 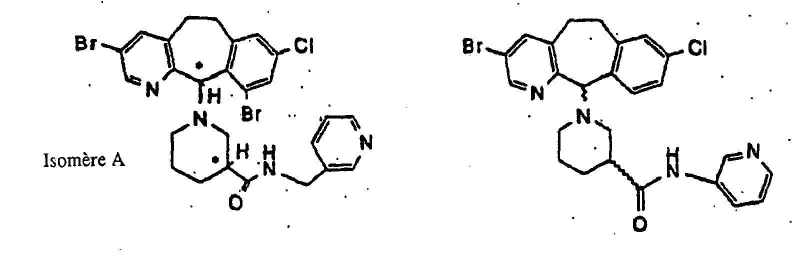 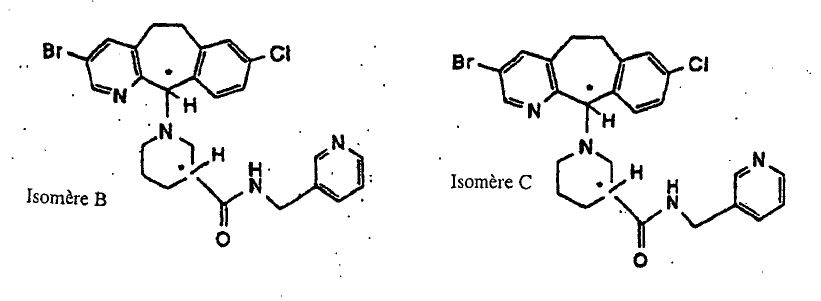 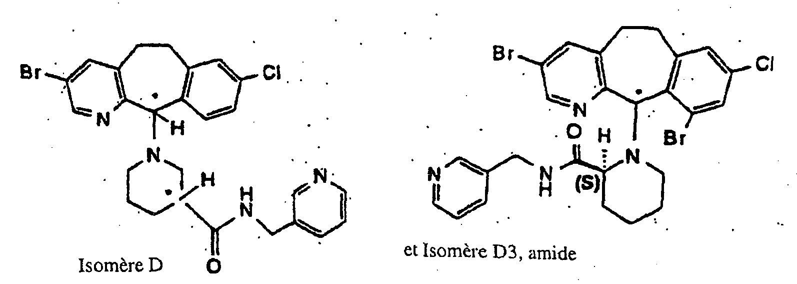 ou sel pharmacologiquement admissible d'un tel composé.

8. Composé conforme à la revendication 1, qui est

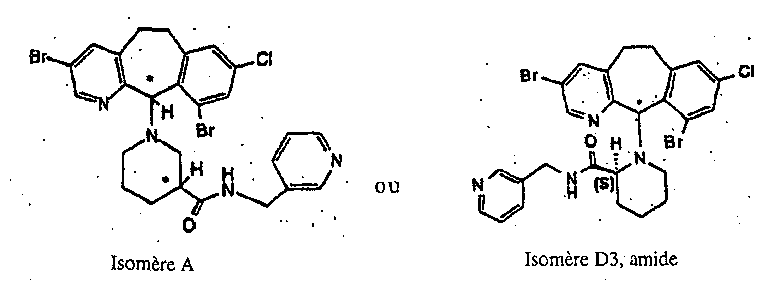

9. Composition destinée à inhiber la croissance anormale de cellules, comprenant une quantité efficace d'un composé conforme à l'une des revendications précédentes, en combinaison avec un véhicule pharmacologiquement admissible.

10. Composition conforme à la revendication 9, les cellules inhibées étant des cellules tumorales exprimant un oncogène ras activé.

11. Composition conforme à la revendication 10, les cellules inhibées étant des cellules de tumeur de pancréas, des cellules cancéreuses de poumon, des cellules tumorales de leucémie myéloïde, des cellules de tumeur de follicules thyroïdiens, des cellules tumorales de myélodysplasie, des cellules tumorales de cancer de l'épiderme, des cellules tumorales de cancer de la vessie, ou des cellules de tumeur du côlon.

12. Composition conforme à la revendication 9, l'inhibition de la croissance anormale de cellules étant due à l'inhibition de la Ras-farnésyl-transférase.

13. Composition conforme à la revendication 9, les cellules inhibées étant des cellules tumorales chez lesquelles la protéine Ras est activée par suite d'une mutation oncogénique intervenue sur des gènes autres que le gène de Ras.
